# EUROPEAN PATENT SPECIFICATION

(11) **EP 4 168 561 B1**
(45) Date of publication and mention of the grant of the patent: **02.12.2026**
(21) Application number: 21845255.5
(22) Date of filing: 20.07.2021
(51) Int. Cl.: C12N 15/87, C12N 15/82, C12N 15/00, C07H 21/04

(54) **VIROID-DERIVED POLYNUCLEOTIDES FOR MODIFICATION OF PLANTS**
VON VIROID ABGELEITETE POLYNUKLEOTIDE ZUR MODIFIZIERUNG VON PFLANZEN
POLYNUCLÉOTIDES DÉRIVÉS DE VIROÏDES POUR DES MODIFICATIONS DE PLANTES

(30) Priority: 20.07.2020 US 202063054101 P
(43) Date of publication of application: 26.04.2023
(73) Proprietor: Flagship Pioneering Innovations VII, LLC, Cambridge, MA 02142 (US)
(72) Inventor: NIU, Yajie, Cambridge, MA 02142 (US); MURALI, Swetha, Srinivasa, Cambridge, MA 02142 (US); SHARPE, Michka, Gabrielle, Cambridge, MA 02142 (US); MARTIN, Barry, Andrew, Cambridge, MA 02142 (US)
(74) Representative: Maiwald GmbH
(86) International application number: PCT/US2021/042414
(87) International publication number: WO 2022/020378

(56) References cited:
- US-A1- 2009 165 153
- US-A1- 2016 152 994
- JOSÉ-ANTONIO DARÒS ET AL: "A viroid-derived system to produce large amounts of recombinant RNA in Escherichia coli", SCIENTIFIC REPORTS, vol. 8, no. 1, 30 January 2018 (2018-01-30), XP055685866, DOI: 10.1038/s41598-018-20314-3
- DELGADO SONIA ET AL: "How sequence variants of a plastid-replicating viroid with one single nucleotide change initiate disease in its natural host", RNA BIOLOGY, vol. 16, no. 7, 16 April 2019 (2019-04-16), pages 906 - 917, XP093179705, ISSN: 1547-6286, DOI: 10.1080/15476286.2019.1600396
- ORTOLÁ BELTRÁN ET AL: "Intron-assisted, viroid-based production of insecticidal circular double-stranded RNA in Escherichia coli", RNA BIOLOGY, 20 January 2021 (2021-01-20), pages 1 - 12, XP055778565, ISSN: 1547-6286, DOI: 10.1080/15476286.2021.1872962
- DALAKOURAS ATHANASIOS, DADAMI ELENA, BASSLER ALEXANDRA, ZWIEBEL MICHELE, KRCZAL GABI, WASSENEGGER MICHAEL: "Replicating Potato spindle tuber viroid mediates de novo methylation of an intronic viroid sequence but no cleavage of the corresponding pre-mRNA", RNA BIOLOGY, vol. 12, no. 3, 4 March 2015 (2015-03-04), pages 268 - 275, XP055899546, ISSN: 1547-6286, DOI: 10.1080/15476286.2015.1017216
- DARÒS JOSÉ-ANTONIO: "Eggplant latent viroid : a friendly experimental system in the family Avsunviroidae : Eggplant latent viroid", MOLECULAR PLANT PATHOLOGY, WILEY-BLACKWELL PUBLISHING LTD., GB, vol. 17, no. 8, 1 October 2016 (2016-10-01), GB , pages 1170 - 1177, XP055899547, ISSN: 1464-6722, DOI: 10.1111/mpp.12358
- STEGER GERHARD, RIESNER DETLEV: "Viroid research and its significance for RNA technology and basic biochemistry", NUCLEIC ACIDS RESEARCH, OXFORD UNIVERSITY PRESS, GB, vol. 46, no. 20, 16 November 2018 (2018-11-16), GB , pages 10563 - 10576, XP055899548, ISSN: 0305-1048, DOI: 10.1093/nar/gky903
- WEI SHUANG, BIAN RUILING, ANDIKA IDA BAGUS, NIU ERBO, LIU QIAN, KONDO HIDEKI, YANG LIU, ZHOU HONGSHENG, PANG TIANXING, LIAN ZIQIAN: "Symptomatic plant viroid infections in phytopathogenic fungi", PROCEEDINGS OF THE NATIONAL ACADEMY OF SCIENCES, NATIONAL ACADEMY OF SCIENCES, vol. 116, no. 26, 25 June 2019 (2019-06-25), pages 13042 - 13050, XP055899549, ISSN: 0027-8424, DOI: 10.1073/pnas.1900762116

## Description

### SEQUENCE LISTING

The instant application contains a Sequence Listing which has been submitted electronically in ASCII format. The ASCII copy, created on July 19, 2021, is named 51484-002WO2_Sequence_Listing_7_19_21_ST25 and is 528,405 bytes in size.

### FIELD OF THE INVENTION

Provided herein are plant-modifying polynucleotides for use in a variety of agricultural and commercial applications.

### BACKGROUND

Plant viroids are circular, single-stranded RNAs capable of invading plants. There is need in the art for plant-modifying polynucleotides (e.g., polynucleotides derived from viroids) for use in a variety of agricultural and commercial applications.

### SUMMARY OF THE INVENTION

In one aspect, disclosed herein is a method of delivering an effector to a plant, comprising providing to the plant a composition comprising a recombinant polynucleotide comprising: (i) a single-stranded RNA (ssRNA) plant viroid sequence of a viroid from the family Avsunviroidae and (ii) a heterologous RNA sequence that is not a plant viroid sequence and that comprises or encodes an effector, wherein (ii) is inserted into (i); and wherein the recombinant polynucleotide replicates in the plant, whereby the effector comprised by or encoded by the heterologous RNA sequence is delivered to the plant.

In some embodiments, the composition is delivered to only part of a plant, to only meristematic tissue of a plant, to rootstock onto which an untreated scion is grafted, or to a scion that is grafted onto untreated rootstock.

In some embodiments, the composition provides an increase in disease resistance, drought tolerance, heat tolerance, cold tolerance, salt tolerance, metal tolerance, herbicide tolerance, herbicide resistance, chemical tolerance, environmental stress resistance, water use efficiency, nitrogen utilization, resistance or tolerance to nitrogen stress, nitrogen fixation, pest resistance, herbivore resistance, pathogen resistance, disease resistance, fungal disease resistance, virus resistance, nematode resistance, bacterial disease resistance, insect control, yield under water-limited conditions, vigor, photosynthetic capability, nutrition, flavor, starch production, protein content, carbohydrate content, oil content, fatty acid content, lipid content, digestibility, biomass, shoot length, root length, root architecture, seed set, seed weight, seed quality, germination, fruit set, rate of fruit ripening, production of biopolymers, production of fibers, production of biofuels, production of pharmaceutical peptides, production of secretable peptides, enzyme production, improved processing traits, or amount of harvestable produce relative to a reference level.

In some embodiments, the ssRNA plant viroid sequence is a genome of a plant viroid from the family Avsunviroidae or a derivative thereof.

In some embodiments, the plant viroid is eggplant latent viroid (ELVd), or
the ssRNA plant viroid sequence has at least 90% sequence identity to a sequence selected from the group consisting of SEQ ID NOs: 50, 55, 56, 57, 58, 59, 60, 61, 62, 63, 64, 67, 69, 70, 71, 72, 73, 156, 157, 158, 160, 161, 162, 163, 164, 165, 167, 171, 172, 173, 174, 176, 177, 178, 179, 180, 181, 182, 183, 184, 185, 186, 187, 188, 189, 190, 191, 192, 193, 194, 195, 197, 198, 199, 200, 201, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 237, 238, 239, 240, 241, 245, 246, 247, 254, 272, 273, 275, 391, 402, 434, and 460.

In some embodiments, the effector comprises or is encoded by an ssRNA sequence.

In some embodiments, the effector is an siRNA, ta-siRNA, miRNA, pre-miRNA, or a guide RNA.

In some embodiments, the heterologous RNA sequence comprises coding RNA, non-coding RNA, or both coding and non-coding RNA.

In some embodiments, the effector comprises coding RNA, non-coding RNA, or both coding and non-coding RNA.

In some embodiments, the effector comprises non-coding RNA comprising at least one regulatory RNA or at least one interfering RNA that regulates a target gene or its transcript in a target cell.

In some embodiments, the effector modifies a trait, phenotype, or genotype in the target cell. In some embodiments, modifying comprises reducing expression of the target gene. In some embodiments, modifying comprises increasing expression of the target gene. In some embodiments, modifying comprises (a) editing the target gene or (b) regulating the target gene.

In some embodiments, the ssRNA plant viroid sequence effects one or more results selected from the group consisting of entry into a tissue or cell of the plant; transmission through a tissue or cell or subcellular component of the plant; replication in a tissue or cell of the plant; targeting to a tissue or cell of the plant; and binding to a factor in a tissue or cell of the plant.

In some embodiments, the recombinant polynucleotide lacks free ends and/or is circular.

In some embodiments, the composition is topically delivered to a plant and the topical delivery is spraying, leaf rubbing, soaking, coating, injecting, seed coating, or delivery through root uptake, or the composition is delivered by injection into the plant's vascular system

In some embodiments, the plant is capable of passing the recombinant polynucleotide on to a progeny plant.

In another aspect, disclosed herein is a composition comprising a recombinant polynucleotide comprising: (a) a single-stranded RNA (ssRNA) plant viroid sequence that is a plant viroid genome or a derivative thereof or a viroid genome fragment or a derivative thereof, wherein the plant viroid is from the family Avsunviroidae, and (b) a heterologous RNA sequence that is not a viroid sequence and comprises or encodes an effector wherein (b) is inserted into (a), wherein the recombinant polynucleotide is capable of replicating in a plant, and wherein the effector comprises a double-stranded RNA stem comprising 50 - 100 base pairs.

In some embodiments, the plant viroid genome is a genome of an eggplant latent viroid (ELVd).

In some embodiments, the ssRNA plant viroid sequence has at least 90% sequence identity to a sequence selected from the group consisting of SEQ ID NOs: 50, 55, 56, 57, 58, 59, 60, 61, 62, 63, 64, 67, 69, 70, 71, 72, 73, 156, 157, 158, 160, 161, 162, 163, 164, 165, 167, 171, 172, 173, 174, 176, 177, 178, 179, 180, 181, 182, 183, 184, 185, 186, 187, 188, 189, 190, 191, 192, 193, 194, 195, 197, 198, 199, 200, 201, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 237, 238, 239, 240, 241, 245, 246, 247, 254, 272, 273, 275, 391, 402, 434, and 460.

In some embodiments, the composition is formulated for delivery to a plant or to the environment in which the plant grows.

In another aspect, disclosed herein is a plant cell comprising a composition of any of the above embodiments. In some embodiments, the plant cell is a dicot plant cell.

In some embodiments, the cell has been transiently transformed with the recombinant polynucleotide.

In some embodiments, the cell has been stably transformed with the recombinant polynucleotide.

### DEFINITIONS

As used herein, the term "internal ribosome entry site" or "IRES" refers to a sequence (e.g., an RNA sequence) capable of recruiting a ribosome and translation machinery to initiate translation from an RNA sequence. An IRES element is generally between 100-800 nucleotides. In embodiments, the efficiency or effectiveness of an IRES in the composition and methods described herein is tested, e.g., by introducing the IRES into a circular RNA expression vector and assaying for levels of expression of a downstream cistronic protein such as firefly luciferase using enzymatic reactions, or fluorescent readouts using reporters such as green fluorescent protein (GFP). An appropriate IRES can be obtained from plant and plant viral IRES sequences such as encephalomyocarditis virus IRES (ECMV), maize hsp101 IRES 5'UTR, crucifer infecting tobamovirus crTMV CR-CP 148 IRES, tobacco etch virus (TEV) IRES 5'UTR and hibiscus chlorotic ringspot virus (HCRSV) IRES. In addition, in embodiments, an IRES sequence is derived from non-plant eukaryotic virus sequences that include but are not limited to: acute bee paralysis virus (ABPV), classical swine fever virus (CSFV), coxsackievirus B3 virus (CVB3), encephalomyocarditis virus (ECMV), enterovirus 71 (E71), hepatitis A virus (HAV), human rhinovirus (HRV2), human rhinovirus (HRV2), human lymphotropic virus (HTLV) and polyoma virus (PV). Examples of IRES sequence useful in the compositions and methods described herein are shown in Table 4.

As used herein, the term "untreated" refers to an organism (e.g., a plant) that has not been contacted with or delivered a recombinant polynucleotide (e.g., plant viroid-derived vector of a viroid from the family Avsunviroidae) described herein, including a separate organism that has not been delivered the recombinant polynucleotide, the same organism undergoing treatment assessed at a time point prior to delivery of the recombinant polynucleotide, or the same organism undergoing treatment assessed at an untreated part of the organism (that is, at an area of the organism not contacted with the recombinant polynucleotide).

As used herein, the term "effective amount," "effective concentration," or "concentration effective to" refers to an amount of a recombinant polynucleotide (e.g., plant viroid-derived vector of a viroid from the family Avsunviroidae) or a composition thereof, sufficient to effect the recited result or to reach a target level (e.g., a predetermined or threshold level) in or on a target organism.

As used herein, the term "topical delivery to a plant", and variants thereof, refers to any method of delivering a composition (e.g. a recombinant polynucleotide described herein) to a plant that does not comprise transformation (e.g., does not comprise direct introduction of the composition to the cytoplasm of the cell, e.g., does not comprise *Agrobacterium-mediated* transformation, viral vector-mediated transformation, electroporation, or use of a gene gun (biolistics)). Methods of topical delivery include, but are not limited to spraying, leaf rubbing, soaking (e.g., soaking of leaves, roots, stems, or other plant parts), coating (e.g., soaking of leaves, roots, stems, or other plant parts, e.g., coating using micro-particulates or nano-particulates), injection (e.g., injection into leaves, roots, stems, or other plant parts), seed coating, and delivery through root uptake (e.g., delivery in a hydroponic system or delivery in another growth medium, e.g., soil).

As used herein, the phrases "modulating a state of an organism", "modulating a state of a cell", and variants thereof refer to an observable change in a state (e.g., the transcriptome, proteome, epigenome, biological effect, or health or disease state) of the organism or cell (e.g., plant or plant cell; arthropod or arthropod cell; mollusk or mollusk cell; fungus or fungus cell; or nematode or nematode cell), as measured using techniques and methods known in the art for such a measurement, e.g., methods to measure the level or expression of a protein, a transcript, or an epigenetic mark, or to measure the increase or reduction of activity of a protein or biological pathway. In some embodiments, the modulation is transient, e.g., does not persist for the lifetime of the plant . In other embodiments, the modulation persists for the lifetime of the plant , but is not inherited by a progeny of the plant. In still other embodiments, the modulation is inherited by a progeny of the plant, e.g., a progeny produced by sexual reproduction, asexual reproduction, or cell division.

In some embodiments, modulating a state of the plant comprises modifying the plant. As used herein, "modifying an organism", "modifying a cell", and variants thereof refer to changing one or more characteristics of a genome of the cell (e.g., a nuclear, mitochondrial, or plastid genome of the cell), e.g., altering the nucleotide sequence or the methylation status of one or more genetic sequences.

In some embodiments, modulating a state of the plant (e.g., modifying the plant) results in a change (e.g., an increase or decrease) of the state by at least 1% relative to a reference level (e.g., a level found in a plant that is not subjected to the treatment or contacted with the composition), e.g., a change of at least 5%, 10%, 15%, 20%, 25%, 30%, 35%, 40%, 45%, 50%, 55%, 60%, 65%, 70%, 75%, 80%, 85%, 90%, 95%, 98% or more than 98% relative to a reference level. In some embodiments, modulating the state of the plant (e.g., modifying the plant) involves increasing a parameter (e.g., the level or expression of a protein, a transcript, or activity of a biological pathway) of the plant, e.g., increasing the parameter by at least 1% relative to a reference level (e.g., at least 5%, 10%, 15%, 20%, 25%, 30%, 35%, 40%, 45%, 50%, 55%, 60%, 65%, 70%, 75%, 80%, 85%, 90%, 95% 98%, 99%, 100% or more than 100% relative to a reference level). In other embodiments, modulating the state of the plant (e.g., modifying the plant) involves decreasing a parameter (e.g., the level or expression of a protein, a transcript, or activity of a biological pathway) of the plant, e.g., decreasing the parameter by at least 1% relative to a reference level (e.g., at least 5%, 10%, 15%, 20%, 25%, 30%, 35%, 40%, 45%, 50%, 55%, 60%, 65%, 70%, 75%, 80%, 85%, 90%, 95% or at least 98% or more relative to a reference level; e.g., up to 100% relative to a reference level). In some embodiments, properties that are modulated include, but are not limited to (a) one or more genetic or epigenetic characteristics of a nuclear or organellar genome or the plantl (e.g., altering the nucleotide sequence or the methylation status of one or more genetic sequences; increasing, decreasing, or otherwise altering gene expression; transiently or stably introducing into the plant a heterologous nucleotide or polypeptide sequence); (b) one or more physiological or biochemical properties of the plant (e.g., altering amino acid, lipid, carbohydrate, vitamin or pro-vitamin, or other nutritional content; altering response to biotic or abiotic stimuli); (c) one or more phenotypic properties of the plant (e.g., flower or leaf appearance, branching or other architectural characteristics, fruit or seed number or size, appearance, or flavor of a plant or a plant part); (d) one or more agronomic or commercially important characteristics of a plant (e.g., flowering time, nutrient use efficiency, water use efficiency, intrinsic yield; resistance of tissue to bruising, oxidation, or softening; resistance to diseases or pests; seed or fruit storage characteristics, or digestibility of a plant or a plant part); or any combination of these properties. In some embodiments, the modulation results in a desirable change or improvement of the plant (e.g., a desirable change or improvement in a plant, a seed of the plant, or a product made from the plant). For example, in embodiments, the modification results in an increase in plant fitness. In other embodiments, the modification results in a decrease in the decrease in plant fitness, (e.g., plant death and/or a decrease in plant fecundity) or a decrease in fitness of a plant pest (e.g., death and/or decreased fecundity of the plant pest, e.g., arthropod, nematode, mollusk, or fungus).

As used herein, the term "effector" refers to a moiety that can be integrated into a recombinant polynucleotide (e.g., plant viroid-derived vector of a viroid from the family Avsunviroidae) and that is capable of modulating (e.g., modifying) a state of a plant or plant cell. In embodiments, the effector comprises or is encoded by an RNA sequence, e.g., a single-stranded RNA (ssRNA) sequence. In embodiments, the effector comprises a coding sequence (e.g., a protein-coding sequence). In embodiments, the effector is, e.g., a regulatory RNA (e.g., a IncRNA, circRNA, tRF, tRNA, rRNA, snRNA, snoRNA, or a piRNA), an interfering RNA, a dsRNA, a microRNA (miRNA) or a pre-miRNA, a phasiRNA, a hcsiRNA, a natsiRNA, or a guide RNA. In embodiments, the effector binds a factor in the target host cell, e.g., binds a nucleic acid, a protein, a peptide, a DNA, an RNA, or a small molecule (e.g., a metabolite or ion).

As used herein, the term "heterologous", when used to describe a first element in reference to a second element means that the first element and second element do not exist in nature disposed as described. For example, a heterologous nucleic acid molecule or sequence is a nucleic acid molecule or sequence that (a) is not native to a cell in which it is expressed, (b) is linked or fused to a nucleic acid molecule or sequence with which it is not linked to or fused to in nature, or with which it is not linked to or fused to in nature in the same way, (c) has been altered or mutated by the hand of man relative to its native state, or (d) has altered expression as compared to its native expression levels under similar conditions. For example, a heterologous RNA relative to a viroid RNA means the heterologous RNA does not exist as part of, or linked to, the viroid RNA in its naturally-occurring state. For example, a recombinant polynucleotide such as those provided by this disclosure can include genetic sequences of two or more different viroids, which genetic sequences are "heterologous" in that they would not naturally occur together. In some embodiments "heterologous" refers to a molecule; for example, a cargo or payload (e.g., a nucleic acid such as a protein-encoding RNA, an ssRNA, a regulatory RNA, an interfering RNA, or a guide RNA) or a structure (e.g., a plasmid or a gene-editing system) that is not found naturally in a plant viroid.

As used herein, "increase the fitness of a plant" refers to an increase in the fitness of the plant directly resulting from contact with a recombinant polynucleotide (e.g., plant viroid-derived vector of a viroid from the family Avsunviroidae) described herein and includes, for example, an improved yield, improved vigor of the plant, or improved quality or amount of a harvested product from the plant, an improvement in pre- or post-harvest traits deemed desirable for agriculture or horticulture (e.g., taste, appearance, shelf life), or for an improvement of traits that otherwise benefit humans (e.g., decreased allergen production). An improved yield of a plant relates to an increase in the yield of a product (e.g., as measured by plant biomass, grain, seed or fruit yield, protein content, carbohydrate or oil content or leaf area) of the plant by a measurable amount over the yield of the same product of the plant produced under the same conditions, but without the application of the instant compositions or compared with application of conventional plant-modifying agents. For example, yield can be increased by at least about 0.5%, about 1%, about 2%, about 3%, about 4%, about 5%, about 10%, about 20%, about 30%, about 40%, about 50%, about 60%, about 70%, about 80%, about 90%, about 100%, or more than 100%. Yield can be expressed in terms of an amount by weight or volume of the plant or a product of the plant on some basis. The basis can be expressed in terms of time, growing area, weight of plants produced, or amount of a raw material used. An increase in the fitness of plant can also be measured in other ways, such as by an increase or improvement of the vigor rating, increase in the stand (the number of plants per unit of area), increase in plant height, increase in stalk circumference, increase in plant canopy, improvement in appearance (such as greener leaf color as measured visually), improvement in root rating, increase in seedling emergence, protein content, increase in leaf size, increase in leaf number, fewer dead basal leaves, increase in tiller strength, decrease in nutrient or fertilizer requirements, increase in seed germination, increase in tiller productivity, increase in flowering, increase in seed or grain maturation or seed maturity, less plant lodging, increased shoot growth, or any combination of these factors, by a measurable or noticeable amount over the same factor of the plant produced under the same conditions, but without the administration of the instant compositions or with application of conventional agricultural agents.

As used herein, "decrease the fitness of a plant" refers to a decrease in the fitness of the plant directly resulting from contact with a recombinant polynucleotide described herein and includes, for example, decreased survival (e.g., death) and/or decreased growth rate, tillering, plant biomass, pollen production, fecundity (e.g., seed yield), seed germination, or fruit yield of the plant compared to a plant grown under the same conditions, but without the application of the instant compositions or compared with application of conventional plant-modifying agents. For example, fitness can be decreased by at least about 0.5%, about 1%, about 2%, about 3%, about 4%, about 5%, about 10%, about 20%, about 30%, about 40%, about 50%, about 60%, about 70%, about 80%, about 90%, or about 100%.

As used herein, the term "formulated for delivery to a plant" refers to a recombinant polynucleotide (e.g., plant viroid-derived vector of a viroid from the family Avsunviroidae) composition that includes an active agent (e.g., a recombinant polynucleotide) and an additional formulation component, e.g., an agriculturally acceptable additional formulation component. As used herein, an "agriculturally acceptable" formulation component is one that is suitable for use in agriculture, e.g., for use on plants. In certain embodiments, the additional formulation component does not have undue adverse side effects to the plants, the environment, or to humans or animals who consume the resulting agricultural products derived therefrom commensurate with a reasonable benefit/risk ratio.

As used herein, the term "plant" refers to whole plants, plant organs, plant tissues, seeds, plant cells, seeds, and progeny of the same. Plant cells include, without limitation, cells from seeds, suspension cultures, embryos, meristematic regions, callus tissue, leaves, roots, shoots, gametophytes, sporophytes, pollen, and microspores. In embodiments, the plant or plant cell is haploid, diploid, triploid, tetraploid, pentaploid, hexaploid, or octoploid. In embodiments, a haploid plant or plant cell treated with a composition such as those described in this disclosure is further subjected to a haploid doubling treatment, resulting in a doubled-haploid plant or plant cell. Plant parts include differentiated and undifferentiated tissues including, but not limited to the following: roots, stems, shoots, leaves, pollen, seeds, fruit, harvested produce, tumor tissue, sap (e.g., xylem sap and phloem sap), and various forms of cells and culture (e.g., single cells, protoplasts, embryos, and callus tissue).

As used herein the term "percent identity" refers to percent (%) sequence identity with respect to a reference polynucleotide (e.g., ribonucleotide) or polypeptide sequence following alignment by standard techniques. Alignment for purposes of determining percent nucleic acid or amino acid sequence identity can be achieved in various ways that are within the capabilities of one of skill in the art, for example, using publicly available computer software such as BLAST, BLAST-2, PSI-BLAST, or Megalign software. In some embodiments, the software is MUSCLE (Edgar, Nucleic Acids Res., 32(5): 1792-1797, 2004). Those skilled in the art can determine appropriate parameters for aligning sequences, including any algorithms needed to achieve maximal alignment over the full length of the sequences being compared. For example, in embodiments, percent sequence identity values are generated using the sequence comparison computer program BLAST. As an illustration, the percent sequence identity of a given nucleic acid or amino acid sequence, A, to, with, or against a given nucleic acid or amino acid sequence, B, (which can alternatively be phrased as a given nucleic acid or amino acid sequence, A that has a certain percent sequence identity to, with, or against a given nucleic acid or amino acid sequence, B) is calculated as follows:
100 multiplied by (the fraction X/Y)
where X is the number of nucleotides or amino acids scored as identical matches by a sequence alignment program (e.g., BLAST) in that program's alignment of A and B, and where Y is the total number of nucleotides or amino acids in B.

### BRIEF DESCRIPTION OF THE DRAWINGS

**Fig. 1** is a diagram showing the secondary structure of the RNA vector ELVd-hpRNA (SEQ ID NO: 3), which comprises an eggplant latent viroid (ELVd) sequence and a heterologous hairpin RNA sequence (hpRNA-SIPDS: indicated by box) targeting the *Solanum lycopersicum* phytoene desaturase (PDS) gene.
**Fig. 2** is a diagram showing the secondary structure of the RNA vector ELVd-gRNA (SEQ ID NO: 5), which comprises an ELVd sequence (indicated by box with dashed lines) and a heterologous guide RNA sequence (gRNA-gI2; indicated by box with solid lines) targeting the *Zea mays* glossy2 (gI2) gene.
**Fig. 3** is a diagram showing the secondary structure of the RNA vector PSTVd-sRNA (SEQ ID NO: 10), which comprises a potato spindle tuber viroid (PTSVd) sequence (no color) and a heterologous small RNA sequence (sRNA-SIPDS: indicated by box) targeting the *Solanum lycopersicum* PDS gene.
**Fig. 4A** is a diagram showing the secondary structure of the RNA vector TL-R-amiR-PDS (SEQ ID NO: 14), which comprises the PSTVd left-terminal region (TL-R; indicated by box with dashed lines) and a heterologous pre-miRNA (amiR-PDS; indicated by box with solid lines) targeting the *Solanum lycopersicum* PDS gene.
**Fig. 4B** is a diagram showing the secondary structure of the RNA vector R-amiR-PDS (SEQ ID NO: 15), which comprises the PSTVd left-terminal and central conserved region (indicated by box with solid lines) and a heterologous pre-miRNA (amiR-PDS; indicated by box with dashed lines) targeting the *Solanum lycopersicum* PDS gene.
**Fig. 5** is a diagram showing the secondary structure of the RNA vector PSTVd/TLR-circGORK (SEQ ID NO: 17), which comprises the terminal left region (TLR) ribonucleotides 331 to 347, covering loops 3 to 5, of PSTVd-RG1 (indicated by box) and a heterologous circular RNA derived from the intron segment flanking exons 2 and 3 of the Arabidopsis GATED OUTWARDLY-RECTIFYING K+ CHANNEL (GORK) gene (circGORK).
**Fig. 6A** is a diagram showing the secondary structure of the RNA vector CircRNA1 (SEQ ID NO: 20), which comprises a PSTVd right terminal domain containing transmission motifs (loop 26 and loop 27) and a heterologous intact Broccoli RNA aptamer sequence (indicated by box).
**Fig. 6B** is a diagram showing the secondary structure of the RNA vector CircRNA2 (SEQ ID NO: 23), which comprises a PSTVd right terminal domain containing transmission motifs (loop 26 and loop 27), a linker region, and a heterologous split Broccoli RNA aptamer sequence (indicated by box).
**Fig. 7** is a diagram showing the secondary structure of a linear PSTVd-spinach fusion RNA vector (SEQ ID NO: 27), which comprises a PSTVd sequence in which the pathogenicity domain has been deleted and replaced with a heterologous Spinach RNA aptamer (indicated by box).
**Fig. 8** is a diagram showing the secondary structure of the RNA vector ELVd-spinach (SEQ ID NO: 29), which comprises the ELVd complete genome, isolate 2 and a heterologous Spinach RNA aptamer (indicated by box).
**Fig. 9** is a diagram showing the secondary structure of the RNA vector PSTVd/TR-amiR-PDS (SEQ ID NO: 32), which comprises the PSTVd right terminal domain containing transmission motifs (loop 26 and loop 27; indicated by box) and a heterologous pre-miRNA targeting the *Solanum lycopersicum* PDS gene.
**Fig. 10** is a diagram showing the secondary structure of the circular RNA vector PSTVd/TL-Spinach (SEQ ID NO: 34), comprising the PSTVd left terminal domain (SEQ ID NO: 33; indicated by box); a heterologous Spinach RNA aptamer (SEQ ID NO: 26); and Loop 6 (L6) (U43/C318) (SEQ ID NO: 33) of PSTVd.
**Fig. 11A** is a pair of diagrams showing alternative predicted secondary structures of a circular RNA vector (SEQ ID NO: 35) comprising a PSTVd left terminal region (SEQ ID NO: 18; indicated by box in the left structure), a heterologous encephalomyocarditis virus internal ribosome entry site (EMCV IRES) (SEQ ID NO: 37); and nanoluciferase (SEQ ID NO: 39).
**Fig. 11B** is a diagram showing the secondary structure of a circular RNA vector (SEQ ID NO: 40) comprising a PSTVd left terminal region (SEQ ID NO: 18; indicated by box), a heterologous maize HSP101 internal ribosome entry site (MAIZE HSP101 IRES) (SEQ ID NO: 38); and nanoluciferase (SEQ ID NO: 39).
**Fig. 12** is a pair of diagrams showing alternative predicted secondary structures of circular fusion RNA 3 (CircRNA3; SEQ ID NO: 43), which comprises a PSTVd viral trafficking motif sequence (SEQ ID NO: 42), a heterologous Spinach RNA aptamer sequence (SEQ ID NO: 26; indicated by box in the top structure); and a PSTVd replication motif sequence (SEQ ID NO: 41).
**Fig. 13** is a diagram showing the secondary structure of the RNA vector PSTVd/TLR-spinach-TPMVd/TLR, which comprises the TLR (331 to 347 nts covering loops 3 to 5; indicated by lower left box) of PSTVd-RG1, the TLR (1 to 72 nts) of TPMVd isolate Mex8 (middle box), and a heterologous Spinach RNA aptamer sequence (upper box).
**Fig. 14** is a diagram showing the secondary structure of circular fusion RNA 4 (CircRNA4; SEQ ID NO: 47), which comprises a PSTVd viral trafficking motif sequence (SEQ ID NO: 18) and an intact heterologous Broccoli RNA aptamer sequence (SEQ ID NO: 19; indicated by box).
**Fig. 15** is a diagram showing the secondary structure of the RNA vector R-hpRNA-RPL7 (SEQ ID NO: 899), which comprises a replication domain of PSTVd (potato spindle tuber viroid), consisting of PSTVd TL-CCR (SEQ ID NO: 12) and heterologous hpRNA-RPL7 (SEQ ID NO: 898), a hairpin RNA targeting the Ribosomal Protein L7 gene of *Leptinotarsa decemlineata.*
**Fig. 16** is a diagram showing the secondary structure of PSTVd.
**Fig. 17** is a diagram showing the secondary structure of ELVd.

### DETAILED DESCRIPTION OF THE INVENTION

### Viroid-derived polynucleotides

Viroids are small, circular, single-stranded RNAs (ssRNAs) that lack a protein coating and are characterized by secondary structures including regions of intramolecular base pairing (stems) and non-paired loops and bulges. Viroids are capable of invading and replicating in plants, and can be virulent, mildly to moderately pathogenic, or commensal with the host plant. This disclosure provides a composition comprising a recombinant polynucleotide (e.g., recombinant ssRNA, e.g. recombinant ssRNA vector) comprising (i) one or more sequences of or derived from a plant viroid sequence of a viroid from the family Avsunviroidae and (ii) one or more heterologous effector sequences that is not a plant viroid sequence and that comprises or encodes an effector, wherein (ii) is inserted into (i); and wherein the recombinant polynucleotide replicates in the plant, and that have a biological effect on a plant (e.g., compositions for topical application to a plant); and methods for modifying plants by delivery of such recombinant polynucleotides. Further disclosed but not claimed are methods for modifying insects, mollusks, fungi, and nematodes by providing for consumption a plant comprising a recombinant polynucleotide such as those described in this disclosure.

Aspects of this disclosure are related to a composition comprising a recombinant polynucleotide comprising: (i) a single-stranded RNA (ssRNA) plant viroid sequence of a viroid from the family Avsunviroidaeand (ii) a heterologous RNA sequence that is not a plant viroid sequence and that comprises or encodes an effector, wherein (ii) is inserted into (i); and wherein the recombinant polynucleotide is capable of replicating in a plant, and wherein the effector comprises a double-stranded RNA stem comprising 50 - 100 base pairs. In some embodiments, the ssRNA viroid sequence is a viroid genome of a plant viroid from the family Avsunviroidae or a derivative thereof, e.g., a viroid genome or derivative thereof described in Section IA, Table 1, and/or Appendix 1 herein. In some embodiments, the ssRNA plant viroid sequence of a plant viroid from the family Avsunviroidae is a viroid genome fragment of a plant viroid from the family Avsunviroidae or a derivative thereof. For example, in embodiments, the ssRNA plant viroid sequence is a viroid genome of a plant viroid from the family Avsunviroidae or derivative thereof described in Section IB and Tables 2 and 3 herein, e.g., a functional domain of a plant viroid from the family Avsunviroidae.

In some embodiments, the recombinant polynucleotide comprises at least two ssRNA viroid sequences, e.g., comprises 2, 3, 4, 5, 6, 7, 8, 9, 10, or more than 10 ssRNA viroid sequences. The two or more ssRNA sequences can occur contiguously or non-contiguously in the recombinant polynucleotide. The two or more ssRNA sequences can be derived from a single viroid (e.g., the recombinant polynucleotide includes two or more fragments or functional domains from a single viroid genome), or can be derived from more than one viroid (e.g., the recombinant polynucleotide encodes two or more viroid genomes or fragments or functional domains from two or more viroid genomes). In instances in which the ssRNA sequences are derived from a single viroid, sequences can be included in the recombinant polynucleotide in an order that corresponds to the order of the sequences in a wild-type version of the viroid, or can be rearranged. In some embodiments, the recombinant polynucleotide comprises more than one copy of a viroid sequence, e.g., comprises two, three, four, five, or more than five copies of such a sequence.

In some embodiments, the ssRNA plant viroid sequence of a viroid from the family Avsunviroidae comprises a loop, an internal loop, a stem-loop, a bulge loop, or a pseudoknot. In some embodiments, the ssRNA plant viroid sequence of a viroid from the family Avsunviroidae comprises a secondary structure element, e.g., a loop, internal loop, stem-loop, bulge loop, or pseudoknot, that is present in a wild-type version of the viroid from which the ssRNA plant viroid sequence of a viroid from the family Avsunviroidae is derived.

In embodiments, the ssRNA plant viroid sequence of a viroid from the family Avsunviroidae participates in, e.g., invasion of the recombinant polynucleotide into plant cells and/or replication of the recombinant polynucleotide in plant cells, thus delivering the effector to plant cells. In some embodiments, the ssRNA plant viroid sequence of a viroid from the family Avsunviroidae effects one or more of entry into a tissue or cell of the plant (e.g., entry into a leaf, root, or stem or a cell thereof); transmission to or through a tissue or cell or subcellular component of the plant; replication in a tissue or cell of the plant; targeting to a tissue or cell of the plant; and binding to a factor in a tissue or cell of the plant.

In some embodiments, the ssRNA plant viroid sequence of a viroid from the family Avsunviroidae comprises, in secondary structure, one or more of a replication motif, a transmission motif, a targeting motif, or a binding motif. In some embodiments, the ssRNA plant viroid sequence of a viroid from the family Avsunviroidae comprises one or more of a replication domain, a transmission domain, a targeting domain, or a binding domain.

In some embodiments, the transmission domain is a tissue transmission domain, a cell-cell transmission domain, or a subcellular transition domain.

In some embodiments, the targeting domain is a tissue targeting domain, a cell targeting domain, or a subcellular targeting domain. In some embodiments, the targeting domain binds to a host cell. In some embodiments, the targeting domain is a nuclear targeting sequence or a nuclear exclusion sequence. In some embodiments, the targeting domain is a plastid targeting sequence, e.g., a chloroplast targeting sequence.

In some embodiments, the binding domain binds a molecular target in the plant. In some embodiments, the binding domain binds DICER.

In some embodiments, the ssRNA plant viroid sequence of a viroid from the family Avsunviroidae does not contain a pathogenicity domain. In some embodiments, the ssRNA plant viroid sequence of a viroid from the family Avsunviroidae is not pathogenic, e.g., is not pathogenic to a plant to which the composition is delivered or is not pathogenic to any plant.

In some embodiments, the recombinant polynucleotide does not comprise an additional polynucleotide sequence for delivery to a cell, e.g., does not comprise a plasmid or a vector (e.g., a viral vector).

In some aspects, the disclosure provides a composition comprising a recombinant polynucleotide comprising (i) a single-stranded RNA (ssRNA) plant viroid sequence of a viroid from the family Avsunviroidae and (ii) a heterologous RNA sequence that is not a plant viroid sequence and that comprises or encodes an effector, wherein (ii) is inserted into (i); and wherein the recombinant polynucleotide is capable of replicating in a plant, and wherein the effector comprises a double-stranded RNA stem comprising 50 - 100 base pairs , wherein the ssRNA plant viroid sequence does not include a chloroplast localization sequence.

### Polynucleotides comprising viroid genomes or fragments thereof

In some embodiments, the ssRNA plant viroid sequence of a viroid from the family Avsunviroidae comprises a sequence of at least 15 ribonucleotides (e.g., at least 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or 300 ribonucleotides or more than 300 ribonucleotides) which is at least 80% identical to a plant viroid genome sequence of a viroid from the family Avsunviroidae or a fragment thereof, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a viroid plant genome sequence of a viroid from the family Avsunviroidae or a fragment thereof.

In some embodiments, the plant viroid is eggplant latent viroid (ELVd), avocado sunblotch viroid, peach latent mosaic viroid or chrysanthemum chlorotic mottle viroid. In some embodiments, the plant viroid is ELVd. In other non-claimed embodiments, the plant viroid is PSTVd. In some embodiments, the plant viroid from the family Avsunviroidae is a plant viroid from the family Avsunviroidae provided in Table 1 and/or Appendix 1.

In some embodiments, the ssRNA plant viroid sequence of a viroid from the family Avsunviroidae comprises a sequence of at least 15 ribonucleotides (e.g., at least 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or 300 ribonucleotides or more than 300 ribonucleotides) which is at least 80% identical to a sequence from the family Avsunviroidae, or fragment thereof, listed in Table 1, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a sequence from the family Avsunviroidae listed in Table 1. In some embodiments, the ssRNA plant viroid sequence of a viroid from the family Avsunviroidae has at least 90% identity to a sequence from the family Avsunviroidae of Table 1. In some embodiments, the ssRNA plant viroid sequence of a viroid from the family Avsunviroidae has at least 95% identity to a sequence from the family Avsunviroidae of Table 1. In some embodiments, the ssRNA plant viroid sequence of a viroid from the family Avsunviroidae has at least 98% identity to a sequence from the family Avsunviroidae of Table 1. In some embodiments, the ssRNA plant viroid sequence of a viroid from the family Avsunviroidae has at least 99% identity to a sequence from the family Avsunviroidae of Table 1. In some embodiments, the plant ssRNA viroid sequence of a viroid from the family Avsunviroidae comprises or consists of a sequence from the family Avsunviroidae listed in Table 1.

In some embodiments, the ssRNA plant viroid sequence of a viroid from the family Avsunviroidae comprises a sequence of at least 40 ribonucleotides which is at least 80% identical to a sequence from the family Avsunviroidae, or fragment thereof, listed in Table 1, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a sequence from the family Avsunviroidae listed in Table 1.

In some embodiments, the ssRNA plant viroid sequence of a viroid from the family Avsunviroidae comprises a sequence of at least 100 ribonucleotides which is at least 80% identical to a sequence from the family Avsunviroidae, or fragment thereof, listed in Table 1, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a sequence from the family Avsunviroidae listed in Table 1.

In some embodiments, the ssRNA plant viroid sequence of a viroid from the family Avsunviroidae comprises a sequence of at least 15 ribonucleotides (e.g., at least 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or 300 ribonucleotides or more than 300 ribonucleotides) which is at least 80% identical to a sequence of an eggplant latent viroid (ELVd), e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a sequence of an ELVd. In some embodiments, the ELVd sequence is SEQ ID NO: 50 (Table 1).

In some non-claimed embodiments, the ssRNA plant viroid sequence comprises a sequence of at least 15 ribonucleotides (e.g., at least 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or 300 ribonucleotides or more than 300 ribonucleotides) which is at least 80% identical to a sequence of a potato spindle tuber viroid (PSTVd), e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a sequence of a PSTVd. In some non-claimed embodiments, the PSTVd sequence is SEQ ID NO: 51 (Table 1).

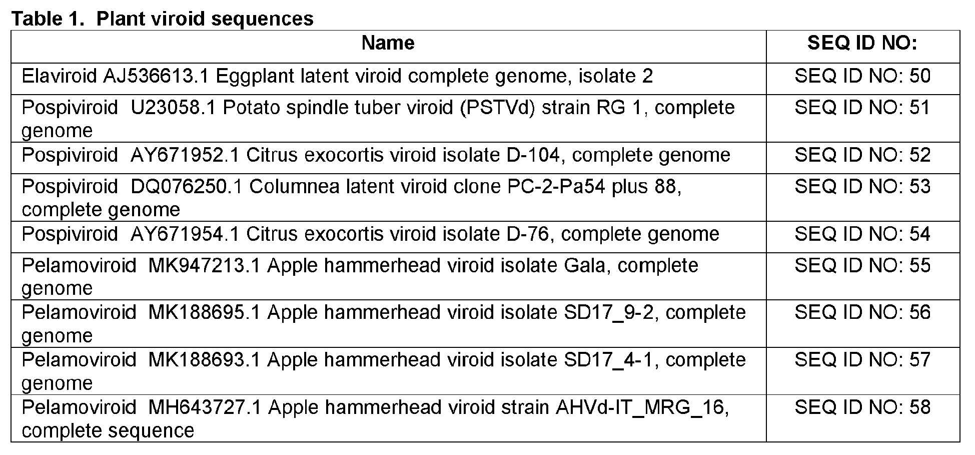

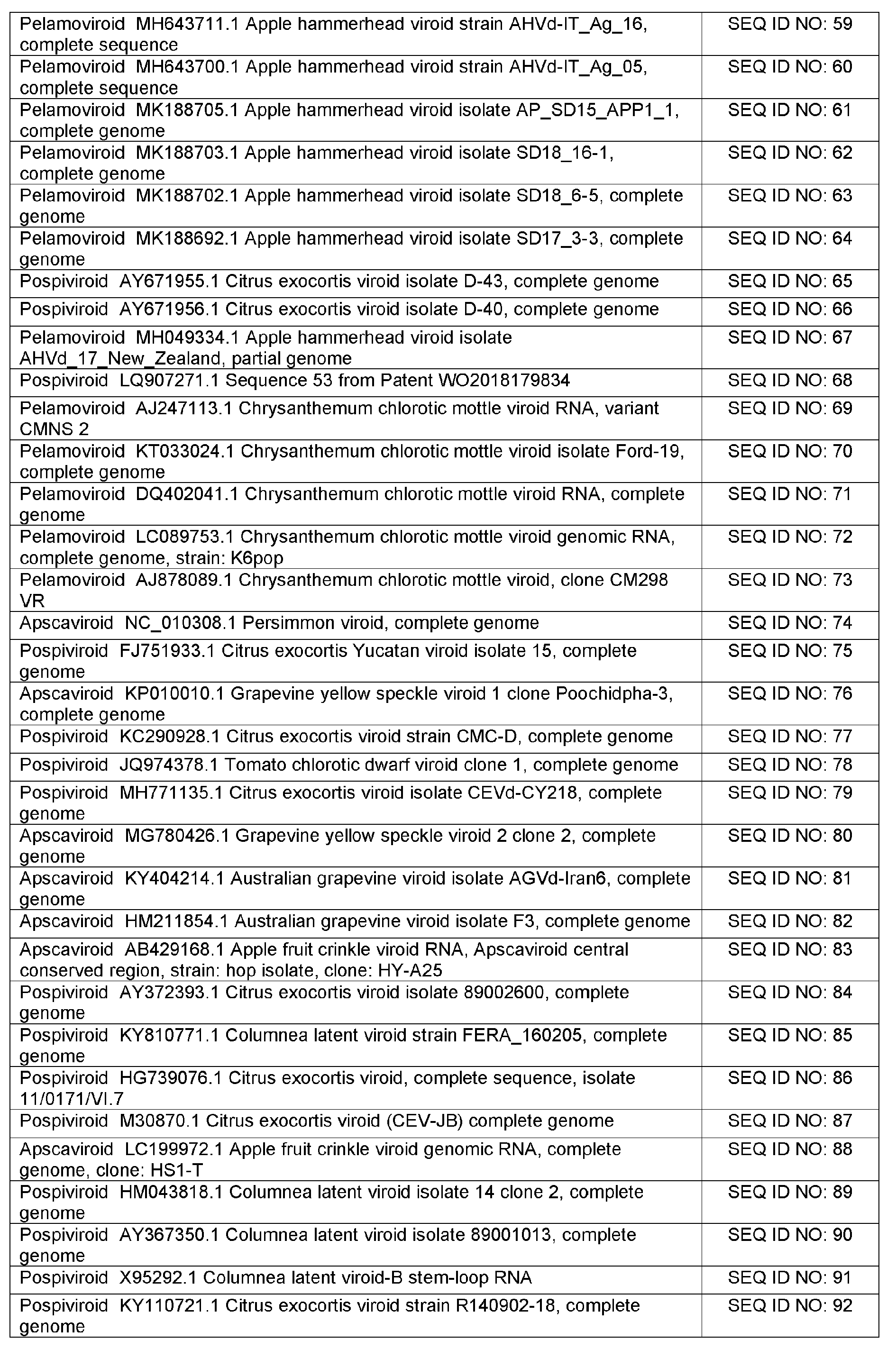

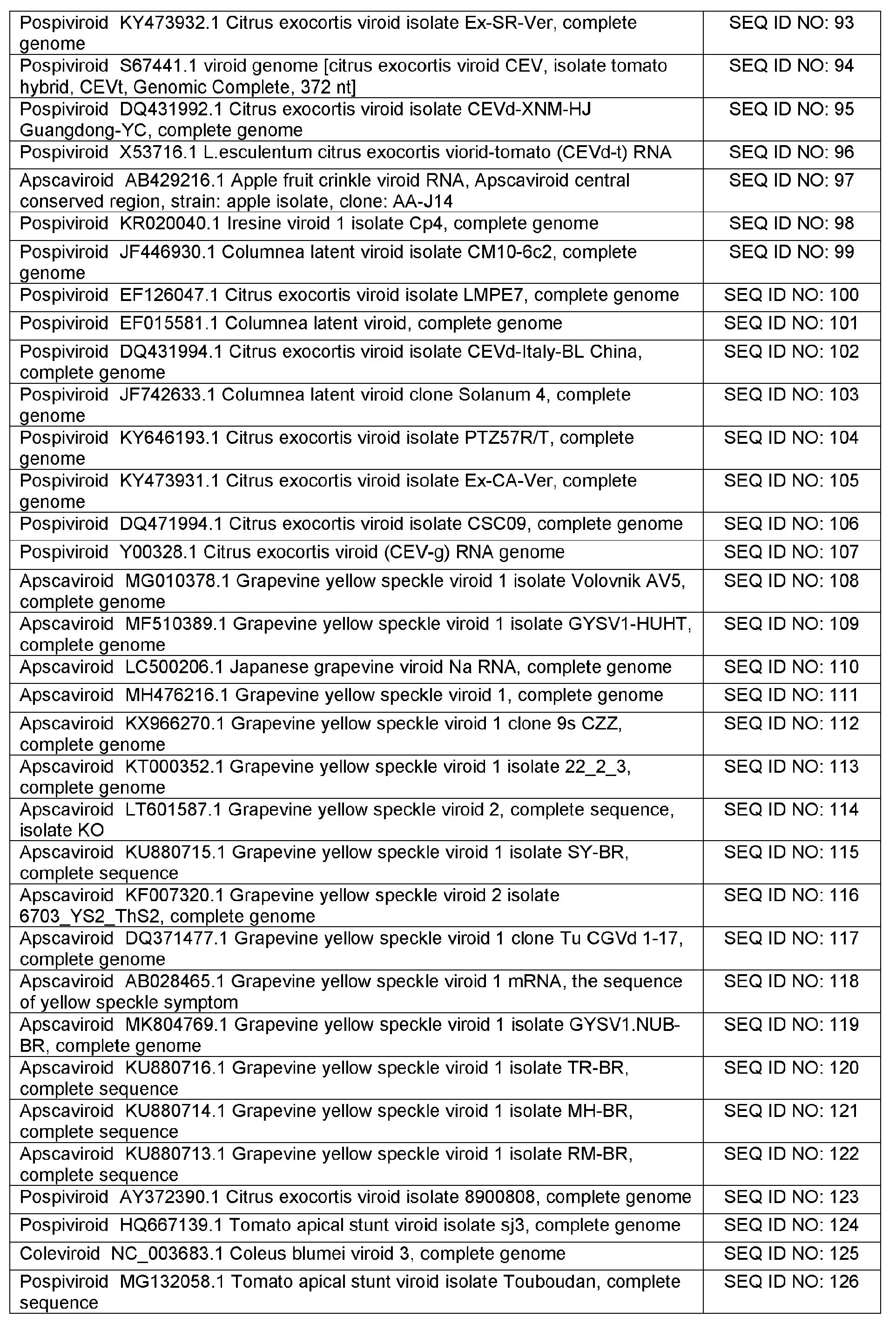

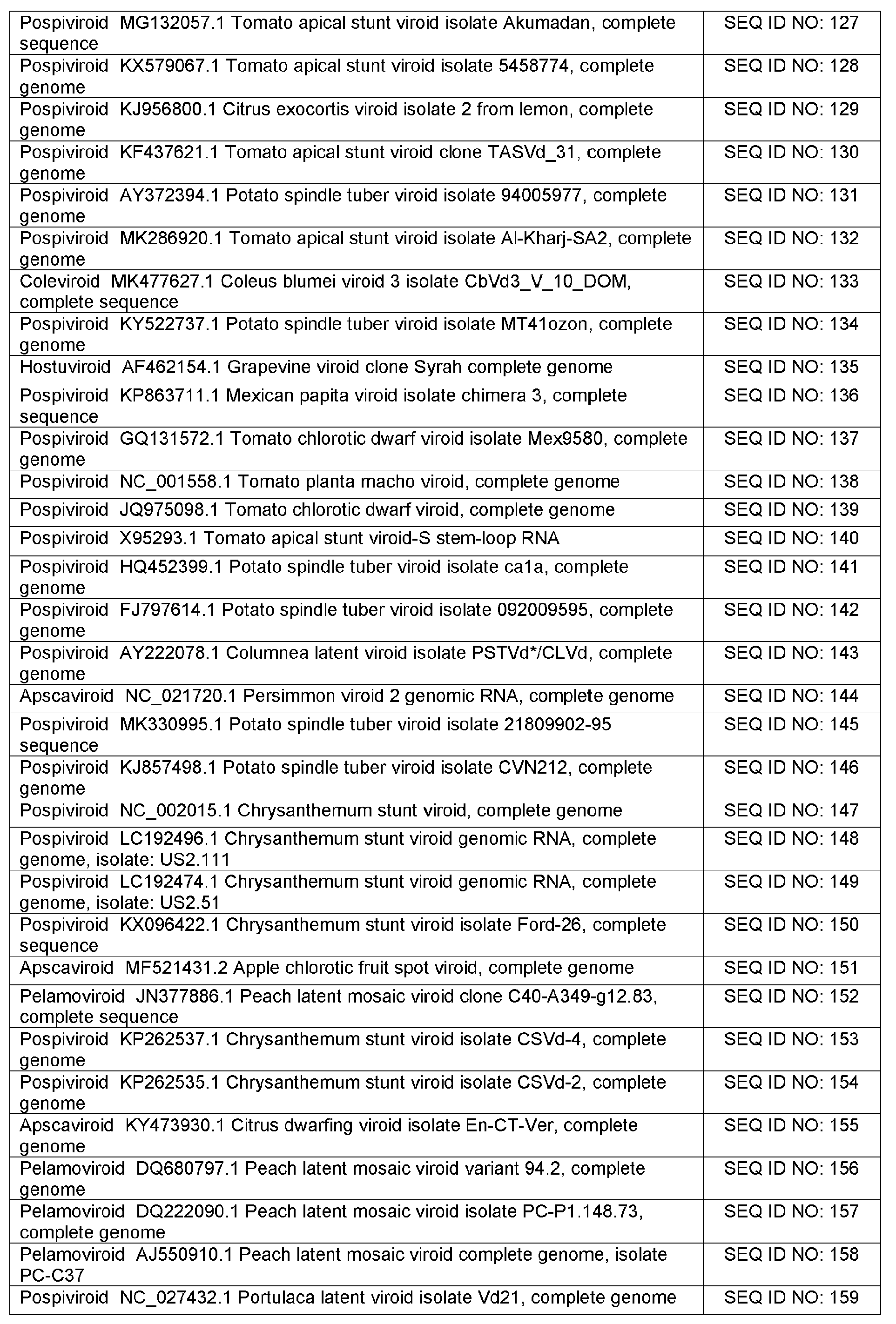

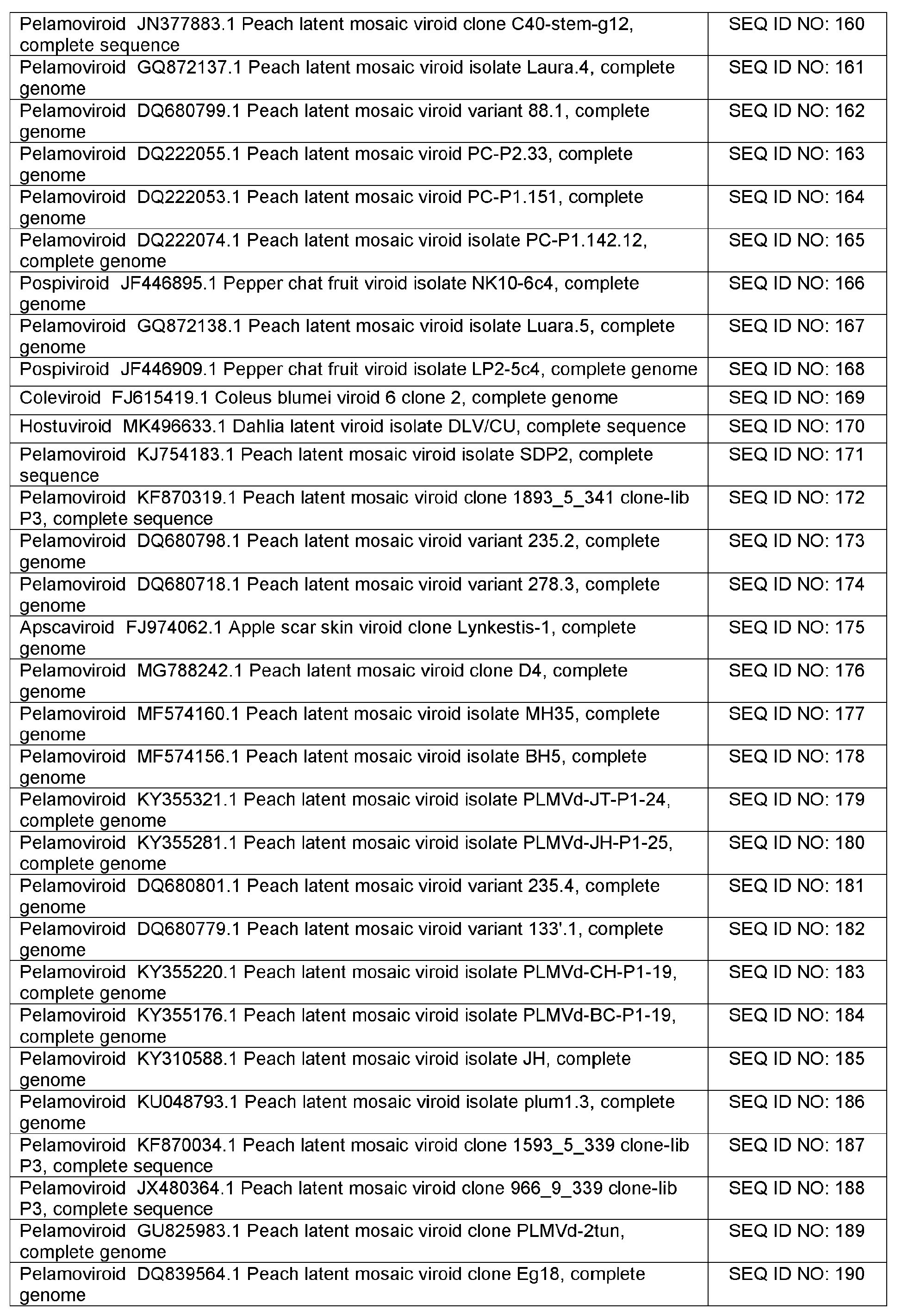

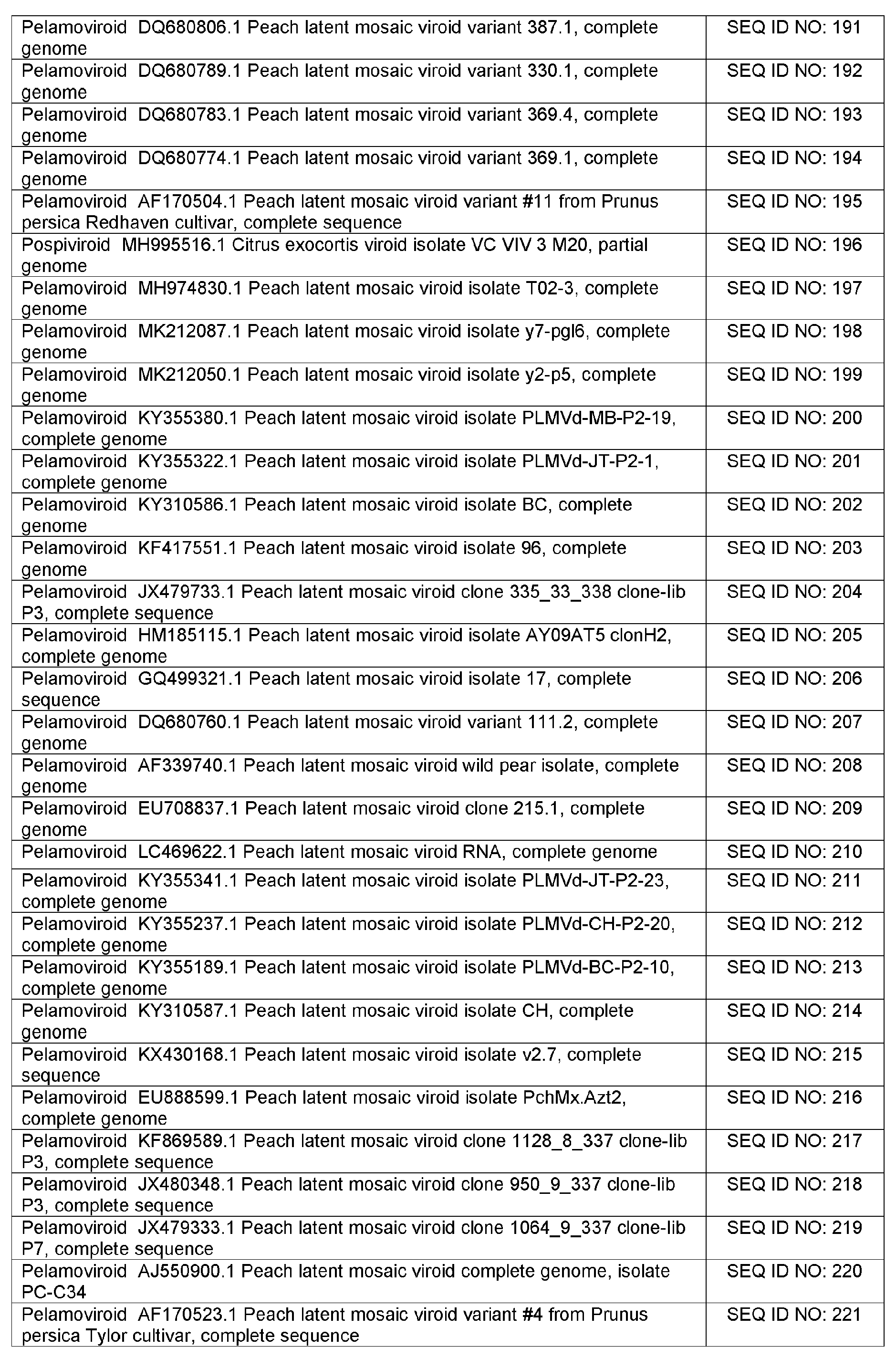

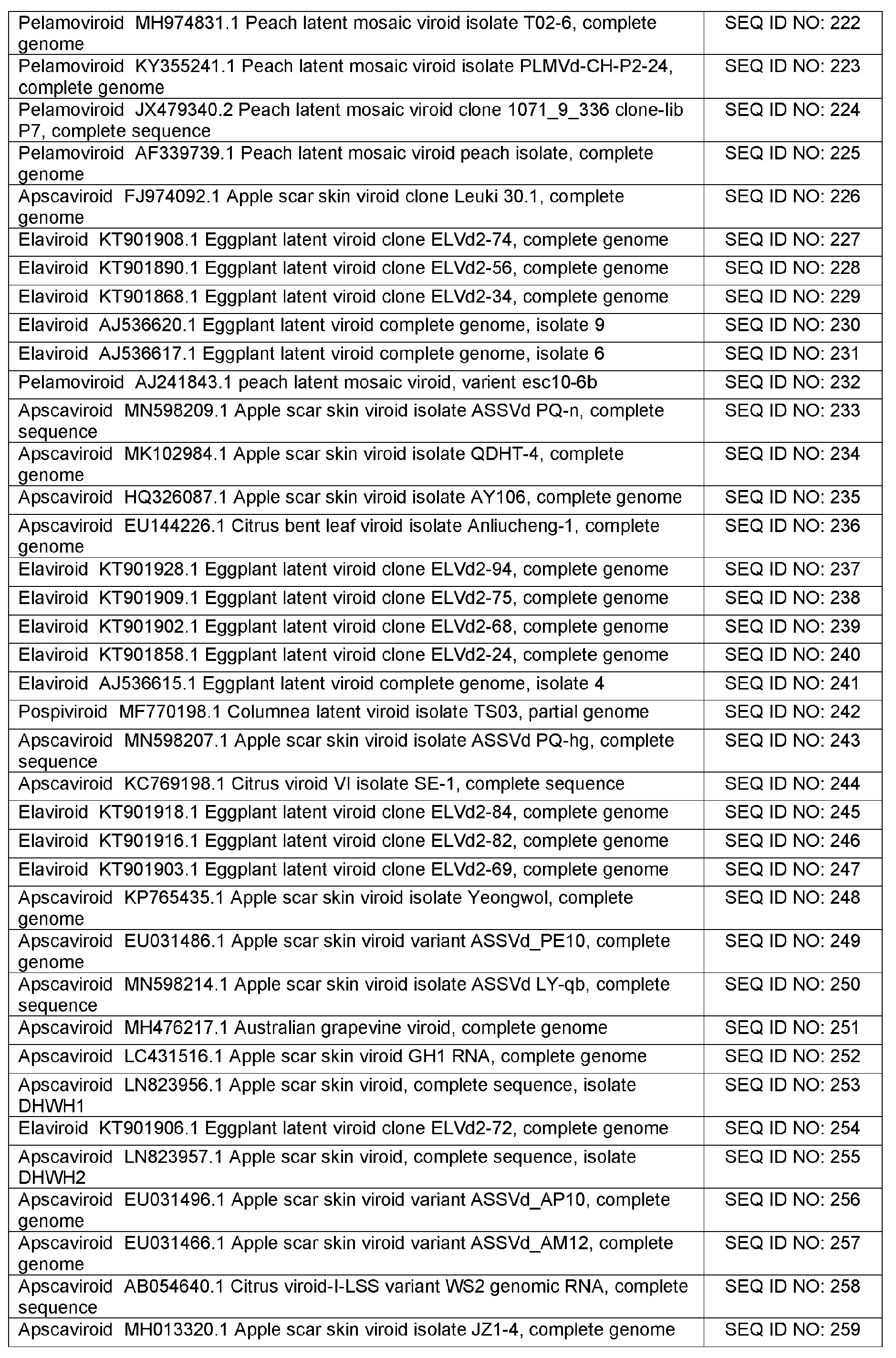

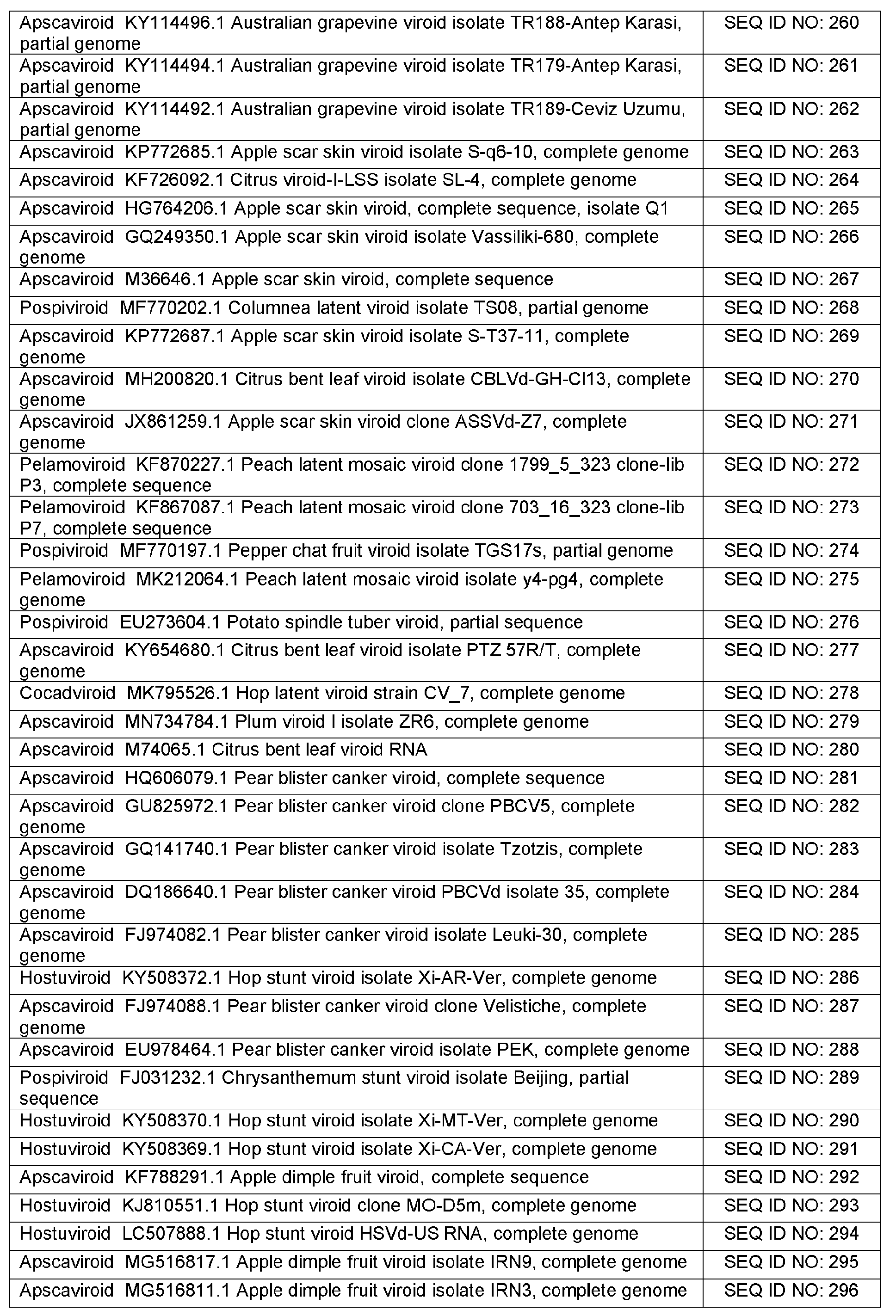

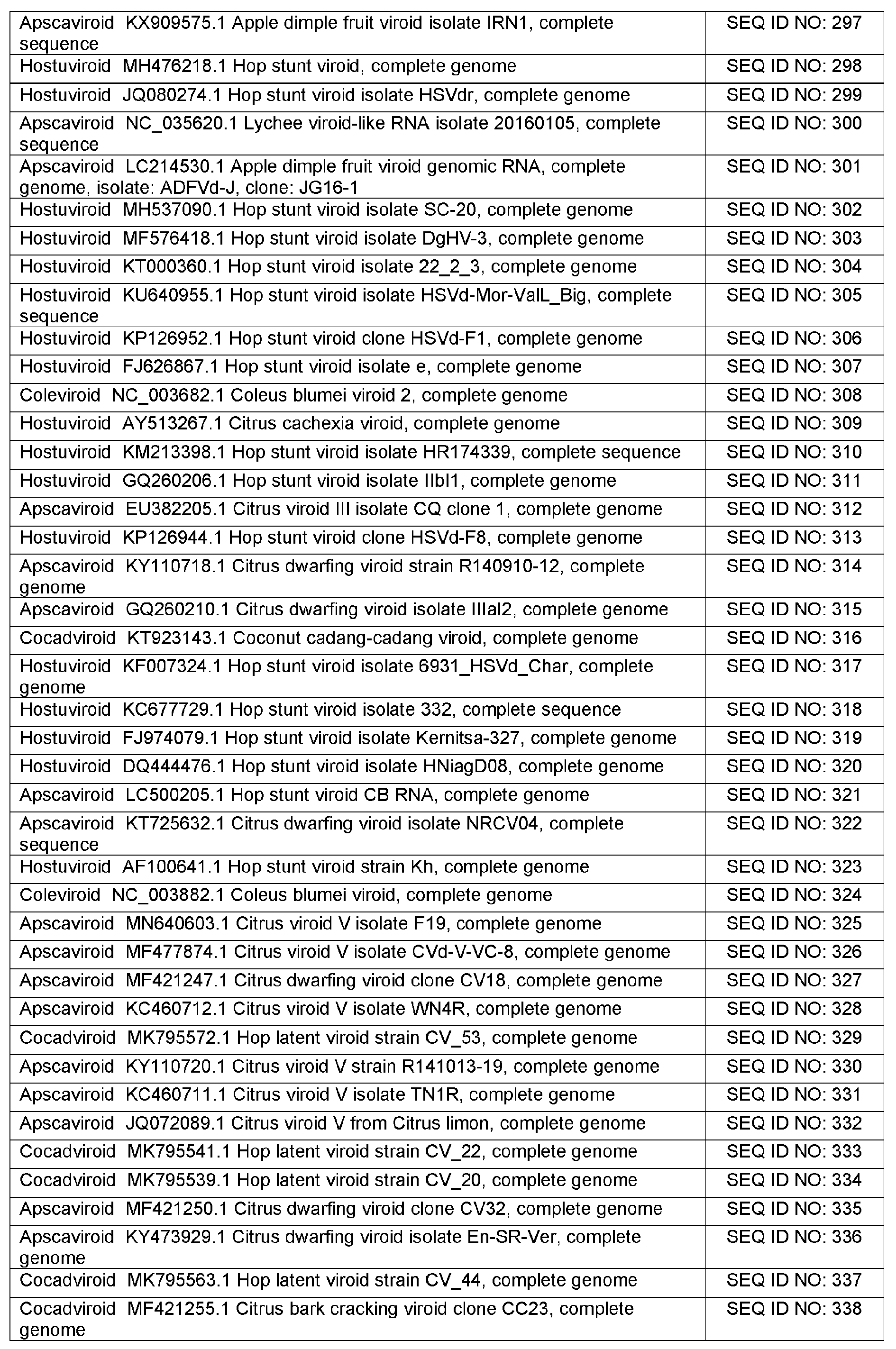

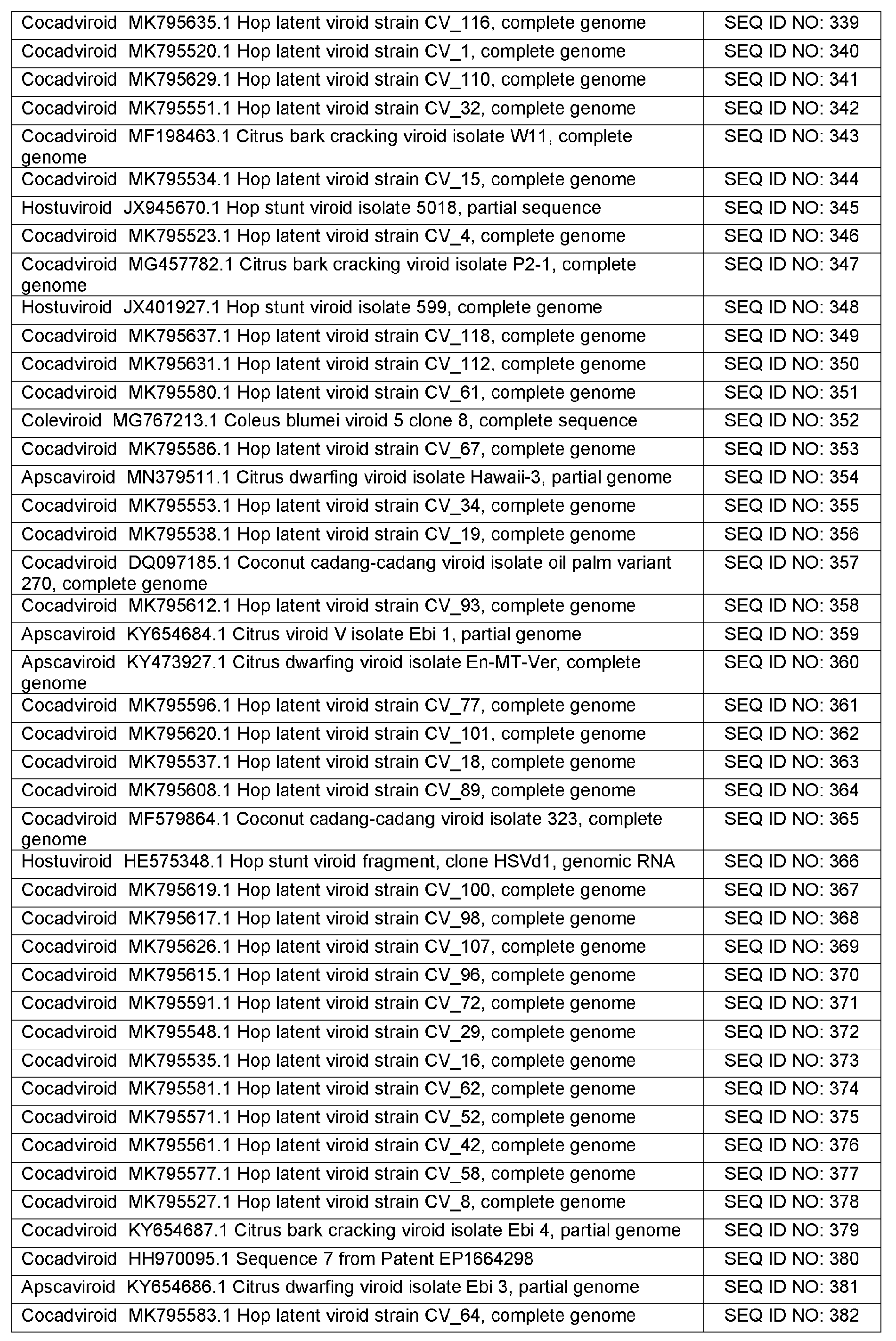

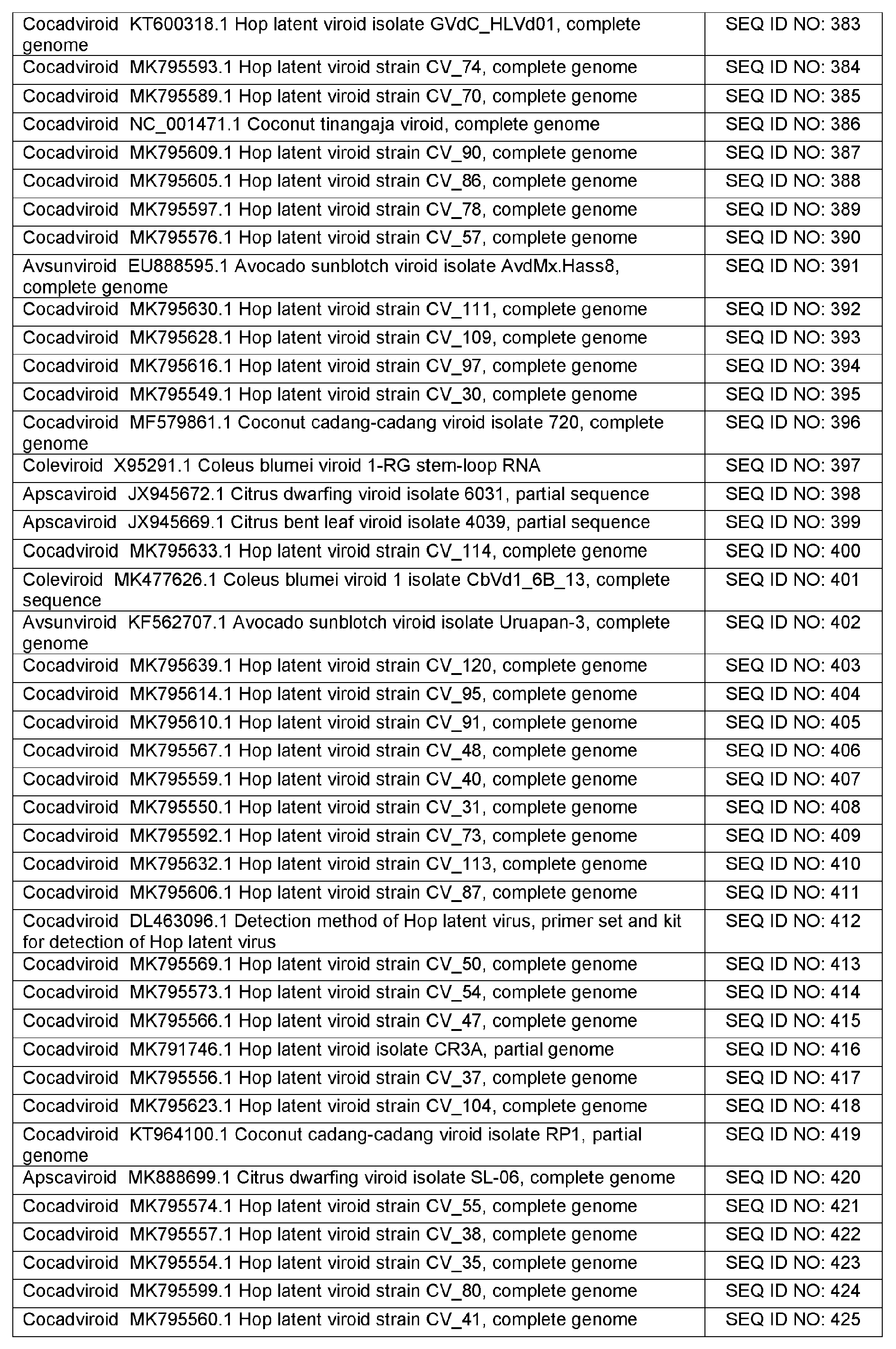

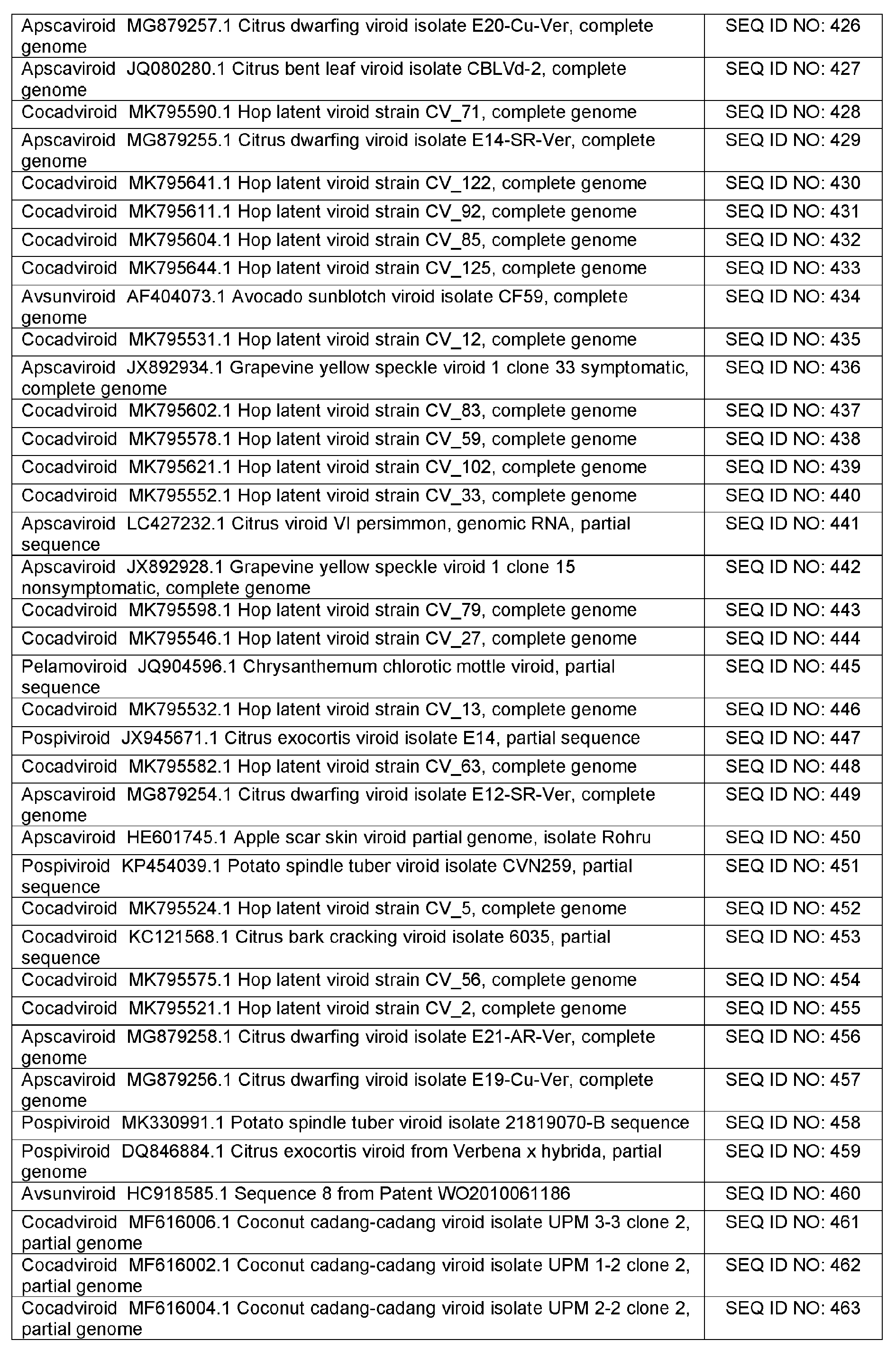

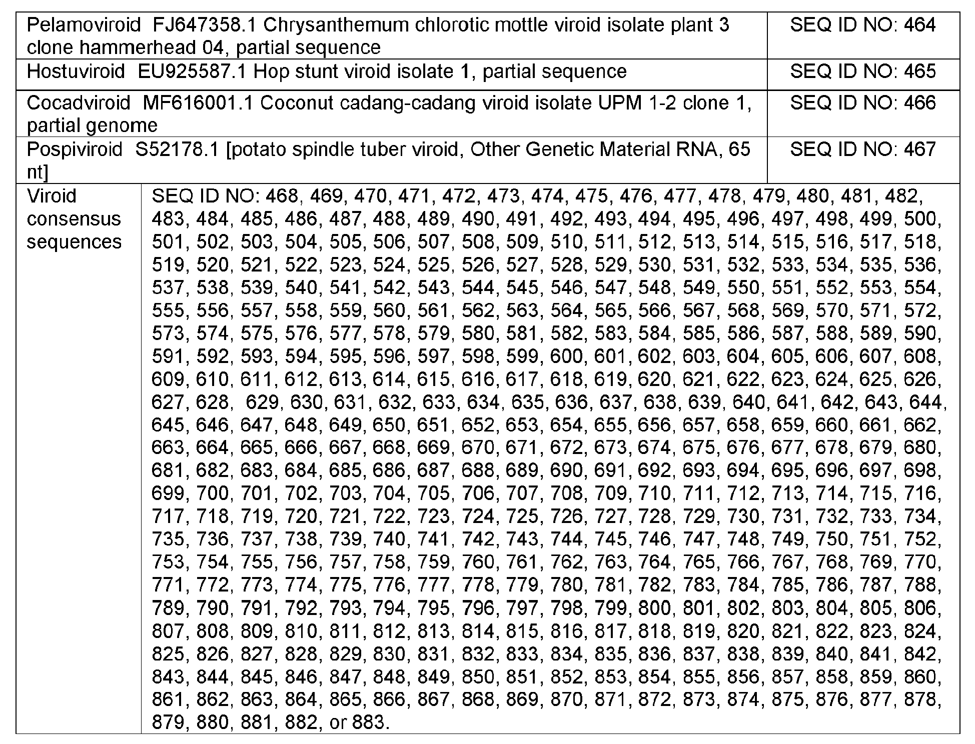

### B. Polynucleotides comprising viroid domains

In some embodiments, the ssRNA plant viroid sequence of a viroid from the family Avsunviroidae comprises a sequence that is at least 80% identical to a sequence listed in Table 3, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a sequence listed in Table 3. In some embodiments, the ssRNA plant viroid sequence of a viroid from the family Avsunviroidae has at least 90% identity to a sequence of Table 3. In some embodiments, the ssRNA plant viroid sequence of a viroid from the family Avsunviroidae has at least 95% identity to a sequence of Table 3. In some embodiments, the ssRNA plant viroid sequence of a viroid from the family Avsunviroidae has at least 98% identity to a sequence of Table 3. In some embodiments, the ssRNA plant viroid sequence of a viroid from the family Avsunviroidae has at least 99% identity to a sequence of Table 3. In some embodiments, the ssRNA plant viroid sequence of a viroid from the family Avsunviroidae comprises or consists of a sequence listed in Table 3.

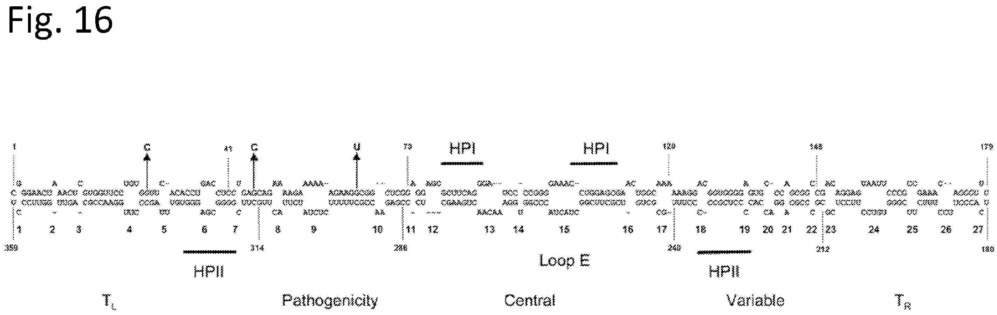

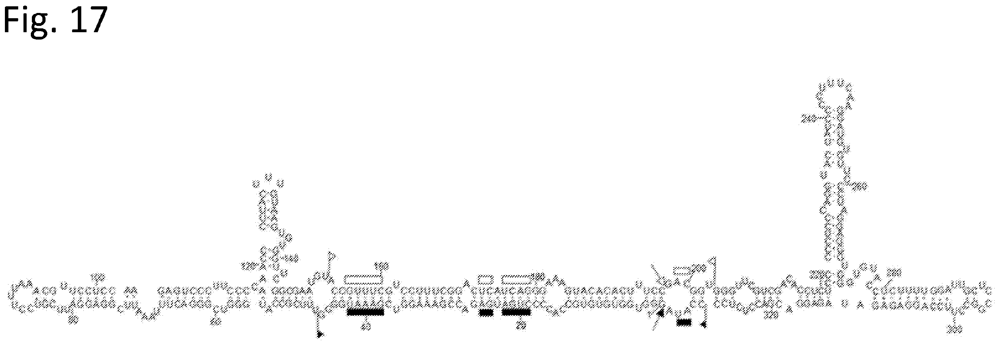

Table 2 shows the sequences of representative domains of PSTVd, and Fig. 16 shows the secondary structure of PSTVd. Table 3 shows the sequences of representative domains of ELVd, and Fig. 17 shows the secondary structure of ELVd.

In some embodiments, the recombinant polynucleotide encodes at least two ssRNA plant viroid sequences, and each of the at least two ssRNA plant viroid sequences is at least 80% identical to a sequence listed in Table 3. For example, in some embodiments, the ssRNA plant viroid sequence comprises a first sequence that is at least 80% identical to a sequence listed in Table 3, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a sequence listed in Table 3, and a second sequence that is at least 80% identical to a sequence listed in Table 2 or Table 3, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to a sequence listed in Table 2 or Table 3. In some embodiments, the recombinant polynucleotide comprises 3, 4, 5, 6, 7, 8, 9, 10, or more than 10 ssRNA plant viroid sequences that are each at least 80% identical to a sequence listed in Table 3. In some embodiments, the at least two ssRNA plant viroid sequences comprise at least one viroid sequence from each of at least two viroid genomes.

In some non-claimed embodiments, the recombinant polynucleotide encodes a sequence that is at least 80% identical to SEQ ID NO: 884 and encodes a sequence that is at least 80% identical to SEQ ID NO: 885. For example, in some non-claimed embodiments, the ssRNA viroid sequence comprises a first sequence that is at least 80% identical to SEQ ID NO: 884, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 884 and a second sequence that is at least 80% identical to SEQ ID NO: 885, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 885. In some non-claimed embodiments, the sequence that is at least 80% identical to SEQ ID NO: 884 and the sequence that is at least 80% identical to SEQ ID NO: 885 base pair with one another, e.g., base pair with one another as shown in Table 2, e.g., base pair to form one or more loops. In some non-claimed embodiments, the one or more loops have a function relating to replication; initiation of transcription (e.g., Binding to TFIIIA 7ZF); or transmission (e.g., trafficking from palisade mesophyll to spongy mesophyll cells or vascular entry). In some non-claimed embodiments, the recombinant polynucleotide comprises the left terminal domain (TL) of PSTVd.

In some non-claimed embodiments, the recombinant polynucleotide encodes a sequence that is at least 80% identical to SEQ ID NO: 886 and encodes a sequence that is at least 80% identical to SEQ ID NO: 887. For example, in some non-claimed embodiments, the ssRNA plant viroid sequence of a viroid from the family Avsunviroidae comprises a first sequence that is at least 80% identical to SEQ ID NO: 886, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 886 and a second sequence that is at least 80% identical to SEQ ID NO: 887, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 887. In some non-claimed embodiments, the sequence that is at least 80% identical to SEQ ID NO: 886 and the sequence that is at least 80% identical to SEQ ID NO: 887 base pair with one another, e.g., base pair with one another as shown in Table 2, e.g., base pair to form one or more loops. In some non-claimed embodiments, the one or more loops have a function relating to pathogenicity. In some non-claimed embodiments, the recombinant polynucleotide comprises the pathogenicity domain of PSTVd.

In some non-claimed embodiments, the recombinant polynucleotide encodes a sequence that is at least 80% identical to SEQ ID NO: 888 and encodes a sequence that is at least 80% identical to SEQ ID NO: 889. For example, in some non-claimed embodiments, the ssRNA viroid sequence comprises a first sequence that is at least 80% identical to SEQ ID NO: 888, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 888 and a second sequence that is at least 80% identical to SEQ ID NO: 889, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 889. In some non-claimed embodiments, the sequence that is at least 80% identical to SEQ ID NO: 888 and the sequence that is at least 80% identical to SEQ ID NO: 889 base pair with one another, e.g., base pair with one another as shown in Table 2, e.g., base pair to form one or more loops. In some non-claimed embodiments, the one or more loops have a function relating to replication or alternative splicing (e.g., interacts with RPL5 to regulate alternative splicing for TF IIIA). In some non-claimed embodiments, the recombinant polynucleotide comprises the central conserved domain of PSTVd.

In some non-claimed embodiments, the recombinant polynucleotide encodes a sequence that is at least 80% identical to SEQ ID NO: 890 and encodes a sequence that is at least 80% identical to SEQ ID NO: 891. For example, in some non-claimed embodiments, the ssRNA viroid sequence comprises a first sequence that is at least 80% identical to SEQ ID NO: 890, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 890 and a second sequence that is at least 80% identical to SEQ ID NO: 891, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 891. In some non-claimed embodiments, the sequence that is at least 80% identical to SEQ ID NO: 890 and the sequence that is at least 80% identical to SEQ ID NO: 891 base pair with one another, e.g., base pair with one another as shown in Table 2, e.g., base pair to form one or more loops. In some non-claimed embodiments, the one or more loops have a function relating to transmission (e.g., trafficking from palisade mesophyll to spongy mesophyll cells). In some non-claimed embodiments, the recombinant polynucleotide comprises the variable domain of PSTVd.

In some non-claimed embodiments, the recombinant polynucleotide encodes a sequence that is at least 80% identical to SEQ ID NO: 892 and encodes a sequence that is at least 80% identical to SEQ ID NO: 893. For example, in some non-claimed embodiments, the ssRNA viroid sequence comprises a first sequence that is at least 80% identical to SEQ ID NO: 892, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 892 and a second sequence that is at least 80% identical to SEQ ID NO: 893, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 893. In some non-claimed embodiments, the sequence that is at least 80% identical to SEQ ID NO: 892 and the sequence that is at least 80% identical to SEQ ID NO: 893 base pair with one another, e.g., base pair with one another as shown in Table 2, e.g., base pair to form one or more loops. In some non-claimed embodiments, the one or more loops have a function relating to replication (e.g., comprise a TF IIIA 9ZF binding site, e.g., comprise a TF IIIA 9ZF binding site involved in systemic trafficking) or epidermal exit or comprise a systemic spread signal. In some non-claimed embodiments, the recombinant polynucleotide comprises the right terminal domain (TR) of PSTVd.

In some embodiments, the recombinant polynucleotide encodes a sequence that is at least 80% identical to SEQ ID NO: 894, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 894. In some embodiments, the sequence that is at least 80% identical to SEQ ID NO: 894 base pairs with itself, e.g., base pairs with itself as shown in Table 3, e.g., base pairs to form one or more loops. In some embodiments, the one or more loops have a function relating to nuclear targeting, chloroplast targeting, or ribozyme activity (e.g., self-cleavage).

In some embodiments, the recombinant polynucleotide encodes a sequence that is at least 80% identical to SEQ ID NO: 895, e.g., is at least 80%, 81%, 82%, 83%, 84%, 85%, 86%, 87%, 88%, 89%, 90%, 91%, 92%, 93%, 94%, 95%, 96%, 97% 98%, 99%, or 100% identical to SEQ ID NO: 895. In some embodiments, the sequence that is at least 80% identical to SEQ ID NO: 895 base pairs with itself, e.g., base pairs with itself as shown in Table 3, e.g., base pairs to form one or more loops.

The dot bracket notation provided in Table 2 and Table 3 was generated using the RNA Fold software for predicting RNA secondary structure based on minimum free energy predictions of base pair probabilities. A dot '.' signifies an unpaired base and a bracket '(' or ')' represents a paired base. Dot bracket notation is further described in Mattei et al., Nucleic Acids Research, 42(10): 6146-6157, 2014; Ramlan and Zauner In International Workshop on Computing With Biomolecules, E. Csuhaj-Varju, R. Freund, M. Oswald and K. Salomaa (Eds.), 27 August 2008, Wien, Austria, pp. 75-86, From: Austrian Computer Society, 2008; and Hofacker et al., Monatshefte Fur Chemie Chem. Monthly, 125: 167-188, 1994.

**Table 2. Potato spindle tuber viroid (PSTVd) domains**

| **Domain** | **Domain sequence** | **Dot-Bracket Notation** | **Loo p** | **Sequence** | **Nt position** | **PVRD** | **Known Function** | **Host factors** |
|---|---|---|---|---|---|---|---|---|
| Left Terminal Domain (TL) | 1-41: | 5'- .(((((((......))))) ))...((((........))) )..-3' | 1 | 5'-CGG-3' | 1-3 | Replication | Initiation of transcription | RNA polymerase II |
| | 5'CGGAACUAAA CUCGUGGUUCC UGUGGUUCACA CCUGACCUC3' (SEQ ID NO: 884) | | | 3'-UCC-5' | 359-357 | | | |
| | | 5'- (((....)))((((((... ..))))))((((((..... .)))))).-3' | 2 | UAA | -9 | | | |
| | 314-359: | | | G-U | U353-352 | | | |
| | 5'GCUUCGGGG CGAGGGUGUUU AGCCCUUGGAA CCGCAGUUGGU UCCU3' (SEQ ID NO: 885) | | | | | | | |
| | | | 3 | UCG | 12-14 | Replication | Binding to TFIIIA 7ZF essential for initiation of transcription | TF IIIA 7ZF |
| | | | | A-C | 349-348 | | | |
| | | | 4 | CUGUG | 21-25 | Replication | | |
| | | | | GUUCC | 341-337 | | | |
| | | | 5 | UC-A | 28-30 | Replication | | |
| | | | | AUUU | 334-331 | | | |
| | | | TL-R | CGGAACUA AACUCGUG GUUCCUGU GGUUCACA CC (SEQ ID NO: 896) | 1-34 | Replication | | |
| | | | | GGUGUUUA GCCCUUGG AACCGCAG UUGGUUCC U (SEQ ID NO: 897) | 327-359 | | | |
| | | | 6 | UGACC | 35-39 | Transmission motif (palisade mesophyll to spongy mesophyll cells) | Decisive role in PSTVd trafficking from palisade mesophyll to spongy mesophyll cells of tobacco leaves. | |
| | | | | GAGCG | 326-322 | | | |
| | | | 7 | CUG | 42-44 | Transmission motif (vascular entry) | mediates vascular entry | |
| | | | | GCU | 319-317 | | | |
| Pathogeni city Domain | 42-73: | 5'- ((((((.............. ......))))))-3' | 8 | GAAA | 49-52 | | | |
| | CUGAGCAGAAA AGAAAAAAGAA GGCGGCUCGG (SEQ ID NO: 886) | | | 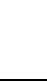 UCAU | 312-309 | | | |
| | | | 9 | AAAAA-A | 55-60 | | | |
| | | | | 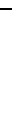 UAUCUCU | 306-300 | | | |
| | | | 10 | G- -C | 68-69 | | | |
| | 286-313: | 5'- .((((((........)))))). ..........-3' | | CAAG | 292-289 | | | |
| | CGAGAACCGCU UUUUCUCUAUC UUACUU (SEQ ID NO: 887) | | | 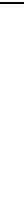 | | | | |
| Central Conserved Domain | 74-120: | 5'- .((..((((((((..(( (....)))..))))))) )..))......-3' | 11 | GAG | 73-75 | | | |
| | AGGAGCGCUUC AGGGAUCCCCG GGGAAACCUGG AGCGAACUGGC AAA (SEQ ID NO: 888) | | | 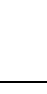 C -C | 285-284 | | | |
| | | | 12 | GAGCG | -80 | | | |
| | | | | U---C | 2-83-282 | | | |
| | | 5'- ..........((((((((((( ((....)))))).......)) )))))..-3' | 13 | G-GGAU | 86-90 | Replication | replication | |
| | | | | 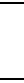 CAACAAA | 276-270 | | | |
| | | | 14 | C-C | 92-93 | Replication | Regulate replication | |
| | 240-285: | | | 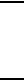 GUG | 268-266 | | | |
| | UGCGCUGUCGC UUCGGCUACUA CCCGGUGGAAA CAACUGAAGCU CC (SEQ ID NO: 889) | | 15 | GGAAAC-C | 97-103 | Replication | Interacts with RPL5 to regulate alternative splicing for TF IIIA | RPL5 |
| | | | | C G CAUCAUCG | 262-255 | | | |
| | | | 16 | AACU | 111-114 | | | |
| | | | | U--G | 247-246 | | | |
| | | | 17 | CAAAA | 117-121 | | | |
| | | | | GCG-U | 243-240 | | | |
| Variable Domain | 121-148: | 5'- ...((.((.((((.....) )))).)).))-3' | 18 | GACG | 125-128 | | | |
| | AAAGGACGGUG GGGAGUGCCCA GCGGCC (SEQ ID NO: 890) | | | GACG CC –C CC-C | 236-234 | | | |
| | | | 19 | GAG | 134-136 | Transmission motif (palisade mesophyll to spongy mesophyll cells) | Decisive role in PSTVd trafficking from palisade mesophyll to spongy mesophyll cells of tobacco leaves. | |
| | | 5'- .....(((((.......))) .))......-3' | | CCC | 228-226 | | | |
| | 212-239: | | | | | | | |
| | CGCCCGCAGGA CCACCCCUCGC CCCCUU (SEQ ID NO: 891) | | | 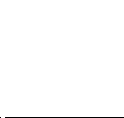 | | | | |
| | | | 20 | GC-C | 138-140 | | | |
| | | | | 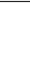 CCAG | 224-221 | | | |
| | | | 21 | CAG | 141-143 | | | |
| | | | | GAC | 220-218 | | | |
| | | | 22 | GCC | 146-148 | | | |
| | | | | [?] CCG | 215-213 | | | |
| Right Terminal Domain (TR) | 149-179: | 5'- ....(((.....))).((( ........)))..-3' | 23 | GACA | 149-152 | | | |
| | GACAGGAGUAA UUCCCGCCGAA ACAGGGUUU (SEQ ID NO: 892) | | | CGCU | 212-209 | | | |
| | | | 24 | GUAAUUC | 156-162 | | | |
| | | 5'- .((((((...........)) ))))..........-3' | | C G UCCUGU G | 205-199 | | | |
| | | | 25 | GCCG | 65-168 | Replication | TF IIIA 9ZF binding site believed to | TF IIIA 9ZF |
| | 180-211: | | | CUUC | 196-193 | | | VirP1 |
| | UCACCCUUCCU UUCUUCGGGUG UCCUUCCUCG (SEQ ID NO: 893) | | | | | | be systemic trafficking | |
| | | | 26 | A -C- A | 171-173 | Transmission motif | Systemic spread signal | |
| | | | | UCCUU | 190-186 | | | |
| | | | 27 | UUU | 177-179 | Transmission motif (epidermal exit) | PSTVd RNA across the boundary are unique and directional, and the U178G/ U179G deficiency specifically impacts epidermal exit | |
| | | | | ACU | 182-180 | | | |

**Table 3. Eggplant latent viroid (ELVd) domains**

| **Sequence** | **Dot-Bracket Notatio n** | **Function** | **EL Vd Lo op** | **Sequence** | **Nt position** | **PVRD** | **Function** |
|---|---|---|---|---|---|---|---|
| 325-333-1-204: | | | 1 | UCCU | 85-88 | Targeting motif (nucleus) | Sufficient to mediate GFP mRNA into nucleus (Gómez & Pallas (2012) *Plant Physiol.* 159:558-564); doi:10.1104/pp.112. 195214 |
| CUCCCCAUAGGG UGGUGUGUGCCA CCCCUGAUGAGA CCGAAAGGUCGA AAUGGGGUUUCG CCAUGGGUCGGG ACUUUAAAUUCG GAGGAUUCGUCC UUUAAACGUUCC UCCAAGAGUCCC UUCCCCAAACCCU UACUUUGUAAGU GUGGUUCGGCGA AUGUACCGUUUC GUCCUUUCGGAC UCAUCAGGGAAA GUACACACUUUC CGACGGUG (SEQ ID NO: 894) | 5'- ....((...((( .((((((((. ..(((((((( (.((((((((. ((((((((... ((((((..((( ..((((((((.. ....((((((.. (((.......)) ).)))))).)) ))))))..))) ..((((..(((( (...))))).)) )).)))))).. ..))))))))). ))))))))).)) ))))))))... ))))))))).) ))...))..- 3' | | | UUAAA | 89-93 | | |
| | | | 2 | AUUC | 80-83 | | |
| | | | | GUU | 95-97 | | |
| | | | 3 | UAAAUUCG | 68-75 | | |
| | | | | CAA | 102-104 | | |
| | | | 4 | GUCG | 58-61 | | |
| | | | | CUUC | 111-114 | | |
| | | | 5 | CAUG | 53-56 | | |
| | | | | CCAAACCCUUACUUUGU AAGUGUGGUUCG | 116-144 | | |
| | | Ribozyme | 6 | GGGUU | 44-48 | | |
| | | | | AUGUAC | 149-154 | | |
| | | Ribozyme | 7 | GUC | 35-37 | | |
| | | | | GUG | 161-163 | | |
| | | Ribozyme | 8 | GAC | 26-28 | | |
| | | | | GAC | 170-172 | | |
| | | Ribozyme | 9 | CCACC | 13-17 | | |
| | | | | GAAAG | 181-185 | | |
| | | Chloroplast targeting | | ACCCCUGAUGAGACCGA AAGGUCGAAAUGGGGU UUCGCCAUGGGUCGGG ACUUUAAAUUCGGAGGA UUCGUCCUUUAAACGUU CCUCCAAGAGUCCCUUC CCCAAACCCUUACUUUG UAAGUGUGGUUCGGCG AAUGUACCGUUUCGUCC UUUCGGACUCAUCAGG G (SEQ ID NO: 900) | 15-181 | Targeting motif (chloroplast) | (Gómez and Pallás, *Plant Signal Behav,* 5(11), 1517-1519, 2010) |
| | | Ribozyme | 10 | GUG | 3-5 | | |
| | | | | UUU | 193-195 | | |
| | | Ribozyme/s elf-cleavage | 11 | CGACG | 197-201 | | |
| | | | | CCCAUAG | 328-1 | | |
| | | Ribozyme | 12 | GUG | 202-204 | | |
| | | | | CUC | 325-327 | | |
| 205-324 | 5'- (((.((((.(( ((((....(((( ((.(((.(((( (((........) )))).)).)..)) ).)))))).)) ))))..((((( (((((..((... .))..))))) ))))......)) ))))).-3' | | 13 | GUU | 206-208 | | |
| GGUUCGUCGACA CCUCUCCCCCUC CCAGGUACUAUC CCCUUUCAAGGA UGUGUUCCCUAG GAGGGUGGGUGU ACCUCUUUUGGA UUGCUCCGGCCU UCCAGGAGAGAU AGAGGACGACCU (SEQ ID NO: 895) | | | | ACC | 321-323 | | |
| | | | 14 | CGACACCUCUCCCCCUC CCAGGUACUAUCCCCUU UCAAGGAUGUGUUCCC UAGGAGGGUGGGUGUA CC GAUAGAGG | 212-279 | | |
| | | | | | 310-317 | | |
| | | | 15 | AUUG | 288-291 | | |
| | | | | CCUU | 298-301 | | |
| | | | 16 | CUC | 292-294 | | |
| | | | | CGG | 295 -297 | | |

### i. Additional heterologous sequence elements

In some aspects, the composition further comprises an additional sequence element that is heterologous to the plant viroid of a viroid from the family Avsunviroidae or heterologous to the plant viroid of a viroid from the family Avsunviroidae and the effector. In some aspects, the recombinant polynucleotide comprising: (i) a single-stranded RNA (ssRNA) plant viroid sequence of a viroid from the family Avsunviroidae and (ii) a heterologous RNA sequence that is not a plant viroid sequence and that comprises or encodes an effector, wherein (ii) is inserted into (i); and wherein the recombinant polynucleotide is capable of replicating in a plant, and wherein the effector comprises a double-stranded RNA stem comprising 50 - 100 base pairs; further comprises an additional sequence element that is heterologous to the plant viroid of a viroid from the family Avsunviroidae or heterologous to the plant viroid of a viroid from the family Avsunviroidae and the effector. In embodiments, the additional heterologous sequence element is, e.g., an internal ribosome entry site (IRES; see Table 4), a 5' homology arm, a 3' homology arm, a polyadenylation sequence, a group I permuted intron-exon (PIE) sequence, an RNA cleavage site, a ribozyme (e.g., a hammerhead ribozyme, a riboswitch, or a twister/tornado), a DICER-binding sequence (e.g., one or more DICER-binding sequences flanking the effector), an mRNA fragment comprising an intron, an exon, a combination of one or more introns and exons, an untranslated region (UTR), an enhancer region, a Kozak sequence, a start codon, or a linker.

**Table 4. Internal ribosome entry sites (IRES)**

| **IRES Class** | **Name** | **Sequence** | **Size (nt)** | **Reference** |
|---|---|---|---|---|
| EMCV IRES encephalomyocarditis virus | ECMV IRES-1 | SEQ ID NO: 901 | 463 | Urwin et al., The Plant Journal, 24(5): 583-589, 2000. |
| | ECMV IRES-2 | SEQ ID NO: 902 | 552 | |
| Maize hsp101 IRES 5'UTR | ZmHSP101 IRES | SEQ ID NO: 903 | 150 | Jiménez-Gonzalez et al., PLoS ONE, 9(9): e107459, 2014. |
| crTMV CR, CP, 148 (Crucifer infecting tobamovirus) 5' UTR | Cr-TMV IRES | SEQ ID NO: 904 | 228 | Dorokhov et al., Proc Natl Acad Sci USA, 99(8): 5301-5306, 2002; Ivanov et al., Virology, 232(1): 32-43, 1997. |
| Tobacco etch virus (TEV) IRES 5'UTR | TEV IRES | SEQ ID NO: 905 | 143 | Gallie, J. Virol., 75(24): 12141-12152, 2001; Bernet and Elena, BMC Evol Biol, 15: 274, 2015. |
| Hibiscus chlorotic ringspot virus (HCRSV) | HCRSV IRES | SEQ ID NO: 906 | 100 | Koh et al., JBC, 278(23): 20565-20573, 2003; Miller et al., Biochem Soc Trans, 35(6): 1629-1633, 2007. |
| Classical swine fever virus (CSFV) | CSFV IRES | SEQ ID NO: 907 | 278 | Wesselhoeft et al., Nature Communications, 9: Article No. 2629, 2018; Friis et al., J Virol, 86(16): 8681-8692, 2012. |
| coxsackievirus B3 virus (CVB3) | CVB3 IRES | SEQ ID NO: 908 | 208 | Wesselhoeft et al., Nature Communications, 9: Article No. 2629, 2018; Bhattacharyya et al., Virology, 377(2): 435-354, 2008; Liu et al., Virology: 265(2): 206-217, 1999. |
| hepatitis A virus (HAV) | HAV IRES | SEQ ID NO: 909 | 738 | Wesselhoeft et al., Nature Communications, 9: Article No. 2629, 2018. |
| human rhinovirus human T-lymphotropic virus (HTLV) | HTLV IRES | SEQ ID NO: 910 | 614 449 | Wesselhoeft et al., Nature Communications, 9: Article No. 2629, 2018; Olivares et al., J Virol, 88(11): 5936-5955, 2014. |
| Polyoma virus (PV) | PV (SV40) IRES | SEQ ID NO: 911 | 742 | Wesselhoeft et al., Nature Communications, 9: Article No. 2629, 2018; Yu and Alwine, J Virol, 80(13): 6553-6558, 2006. |

In some aspects, the composition or the recombinant polynucleotide comprising (i) a single-stranded RNA (ssRNA) plant viroid sequence of a viroid from the family Avsunviroidae and (ii) a heterologous RNA sequence that is not a plant viroid sequence and that comprises or encodes an effector, wherein (ii) is inserted into (i); and wherein the recombinant polynucleotide is capable of replicating in a plant, and wherein the effector comprises a double-stranded RNA stem comprising 50 - 100 base pairs; further comprises 2, 3, 4, 5, 6, 7, 8, 9, 10, or more than 10 additional heterologous sequence elements, e.g., 2, 3, 4, 5, 6, 7, 8, 9, 10, or more than 10 of an internal ribosome entry site (IRES), a 5' homology arm, a 3' homology arm, a polyadenylation sequence, a group I permuted intron-exon (PIE) sequence, an RNA cleavage site, a ribozyme (e.g., a hammerhead ribozyme, a riboswitch, or a twister/tornado), a DICER-binding sequence (e.g., one or more DICER-binding sequences flanking the effector), an mRNA fragment comprising an intron, an exon, a combination of one or more introns and exons, an untranslated region (UTR), an enhancer region, a Kozak sequence, a start codon, or a linker. In some embodiments, the recombinant polynucleotide comprises more than one copy of an additional heterologous sequence element, e.g., comprises two, three, four, five, or more than five copies of such an element.

### C. Non-claimed methods of making recombinant polynucleotides

Recombinant polynucleotides comprising: (i) a single-stranded RNA (ssRNA) viroid sequence and (ii) a heterologous RNA sequence comprising or encoding an effector can be made using any method described herein or known in the art. In some non-claimed embodiments, the recombinant polynucleotide is synthesized from a DNA template, e.g., using *in vitro* transcription, thus generating a linear recombinant polynucleotide. The recombinant polynucleotide ca be used in linear format (e.g., can be linear or linearized), or can be circularized or concatemeric. Methods for circularizing polynucleotides are described below.

Aspects of this disclosure that do not form part of the claimed invention are related to a composition comprising a double-stranded recombinant polynucleotide (e.g., a DNA) that is transcribed to produce a single-stranded recombinant polynucleotide of the disclosure, e.g., a single-stranded recombinant polynucleotide (e.g., an ssRNA) comprising: (i) a single-stranded RNA (ssRNA) viroid sequence and (ii) a heterologous RNA sequence comprising or encoding an effector. The double-stranded polynucleotide can be transcribed *in vitro* or *in vivo,* e.g., manufactured in a host cell (e.g., a bacterial cell) or translated in a plant cell, an arthropod cell, a mollusk cell, a fungal cell, or a nematode cell. In some non-claimed aspects, the cell has been transiently transformed or stably transformed with the double-stranded recombinant polynucleotide (e.g., DNA).

### Circular recombinant polynucleotides

In some non-claimed aspects, the recombinant polynucleotide comprising (i) a single-stranded RNA (ssRNA) viroid sequence and (ii) a heterologous RNA sequence comprising or encoding an effector is circular, e.g., has no free ends. Circular RNA is more resistant to exonuclease degradation than linear RNA due to the lack of 5' and 3' ends. Circular recombinant polynucleotides can be produced using several methods, as described herein.

In some non-claimed embodiments, a linear recombinant polynucleotide (e.g., RNA) is circularized using splint ligation. Splint DNA is designed to anneal between about 5 and 25 nucleotides (nt) of each 5' or 3' end of the linear polynucleotide (e.g., RNA) (e.g., 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 nt), leaving 2 nt at each end of the polynucleotide (e.g., RNA) unpaired. The linear recombinant polynucleotide (e.g., RNA) is annealed with the splint DNA and incubated with a ligase (e.g., T4 RNA ligase 2).

In some non-claimed embodiments, a linear recombinant polynucleotide (e.g., RNA) is circularized using a tRNA ligase. Viroids are plant pathogens consisting of a single-stranded circular RNA that replicate in host cells and are circularized by endogenous tRNA ligases. In some non-claimed embodiments, linear recombinant polynucleotides (e.g., RNAs) are circularized in a bacterial cell, e.g., an *E. coli* cell, in which an appropriate tRNA ligase (e.g., an eggplant tRNA ligase) is present.

In some non-claimed embodiments, a linear recombinant polynucleotide (e.g., RNA) is circularized using ligation of ribozyme-cleaved ends. Ribozyme-cleaved ends of linear RNAs can be joined to synthesize circular RNA, e.g., as described in Litke and Jaffrey, Nature Biotechnology, 37: 667-675, 2019. Recently described "Twister" ribozymes undergo self-cleavage to produce 5' hydroxyl and 2',3'-cylic phosphate ends. These ends are recognized for ligation by the *E. coli* RNA ligase RtcB. To trigger RNA circularization, RNA transcripts are expressed containing an RNA of interest flanked by ribozymes that undergo spontaneous autocatalytic cleavage. The resulting RNA contains 5' and 3' ends that are then ligated by the nearly ubiquitous endogenous RNA ligase RtcB, thereby producing circular RNAs. Circularization of a polynucleotide can be detected, e.g., using denaturing polyacrylamide gel electrophoresis (PAGE). Because of their circular structure, circular polynucleotides (e.g., RNAs) migrate more slowly than linear polynucleotides (e.g., RNAs) on PAGE gels. Additionally, circularization of a polynucleotide (e.g., RNA) can be assessed using digestion with RNAse H, a nonspecific endonuclease that recognizes DNA/RNA duplexes. For a linear RNA, it is expected that after binding of the DNA oligomer and subsequent cleavage by RNAse H, two cleavage products are obtained. A concatemer is expected to produce at least three cleavage products. A circular RNA is expected to produce a single cleavage product. This is visualized as the presence of one, two or three bands on a gel.

### D. Formulations of recombinant polynucleotides

The compositions described herein can be formulated either in pure form (e.g., the composition contains only the recombinant polynucleotide) or together with one or more additional formulation components to facilitate application or delivery of the compositions. In embodiments, the additional formulation component includes, e.g., a carrier (i.e., a component that has an active role in delivering the active agent (e.g., recombinant polynucleotide); for example, a carrier can encapsulate, covalently or non-covalently modify, or otherwise associate with the active agent in a manner that improves delivery of the active agent) or an excipient (e.g., a delivery vehicle, adjuvant, diluent, surfactant, stabilizer, or tonicity agent). In some embodiments, the composition is formulated for delivery to a plant.

In some aspects, the disclosure provides a formulation comprising any of the compositions described herein. In some embodiments, the formulation is a liquid, a gel, or a powder. In some embodiments, the formulation is configured to be sprayed on plants, to be injected into plants, to be rubbed on leaves, to be soaked into plants, to be coated onto plants, or be coated on seeds, or to be delivered through root uptake (e.g., in a hydroponic system or via soil).

Depending on the intended objectives and prevailing circumstances, the composition can be formulated into emulsifiable concentrates, suspension concentrates, directly sprayable or dilutable solutions, coatable pastes, diluted emulsions, spray powders, soluble powders, dispersible powders, wettable powders, dusts, granules, encapsulations in polymeric substances, microcapsules, foams, aerosols, carbon dioxide gas preparations, tablets, resin preparations, paper preparations, nonwoven fabric preparations, or knitted or woven fabric preparations. In some instances, the composition is a liquid. In some instances, the composition is a solid. In some instances, the composition is an aerosol, such as in a pressurized aerosol can.

In some instances, the recombinant polynucleotide makes up about 0.1% to about 100% of the composition, such as any one of about 0.01% to about 100%, about 1% to about 99.9%, about 0.1% to about 10%, about 1% to about 25%, about 10% to about 50%, about 50% to about 99%, or about 0.1% to about 90% of active ingredients (e.g., recombinant polynucleotides). In some instances, the composition includes at least any of 0.1%, 0.5%, 1%, 5%, 10%, 20%, 30%, 40%, 50%, 60%, 70%, 80%, 90%, 95%, or more active ingredients (e.g., recombinant polynucleotides). In some instances, the concentrated agents are preferred as commercial products, the final user normally uses diluted agents, which have a substantially lower concentration of active ingredient.

### i. Formulation for topical delivery

In some embodiments, the composition is formulated for topical delivery to a plant. In some embodiments, the topical delivery is spraying, leaf rubbing, soaking, coating (e.g., coating using micro-particulates or nano-particulates), or delivery through root uptake (e.g., delivery in a hydroponic system). In some embodiments, the composition further comprises a carrier and/or an excipient. In other embodiments, the composition does not comprise a carrier or excipient, e.g., comprises a naked polynucleotide (e.g., a naked RNA).

In some embodiments, the recombinant polynucleotide is delivered at a concentration of at least 0.1 grams per acre, e.g., at least 0.1, 1, 2, 5, 10, 15, 20, 25, 30, 35, 40, 45, or 50 grams per 4046.86 m² (1 acre). In some embodiments, less than 120 liters per acre is delivered, e.g., less than 110, 100, 90, 80, 70, 60, 50, 40, 30, 20, 15, 10, 5, or 2 liters per acre or less than 1 liter per 4046.86 m² (1 acre).

### ii. Carriers

In some aspects, the formulation comprises a carrier. In some embodiments the formulation is an emulsion or a reverse emulsion, a liquid, or a gel. In embodiments, the formulation includes a carrier that serves as a physical support (e.g., solid or semi-solid surfaces or matrices, powders, or particles or nanoparticles). In embodiments, the active agent is encapsulated or enclosed in or attached to or complexed with a carrier including a liposome, vesicle, micelle, or other fluid compartment. In embodiments, the active agent is encapsulated or enclosed in or attached to or complexed with a carrier including a naturally occurring or synthetic, branched or linear polymer (e.g., pectin, agarose, chitin, chitosan, DEAE-dextran, polyvinylpyrrolidone ("PVP"), or polyethylenimine ('PEI")). In embodiments the carrier includes cations or a cationic charge, such as cationic liposomes or cationic polymers such as polyamines (e.g., spermine, spermidine, putrescine). In embodiments, the carrier includes a polypeptide such as an enzyme, (e.g., cellulase, pectolyase, maceroenzyme, pectinase), a cell penetrating or pore-forming peptide (e.g., poly-lysine, poly-arginine, or polyhomoarginine peptides).

Non-limiting examples of carriers include cationic liposomes and polymer nanoparticles reviewed by Zhang et al. (2007) J. Controlled Release, 123:1 - 10, and the cross-linked multilamellar liposomes described in US Patent Application Publication 2014/0356414 AI. In embodiments, the carrier includes a nanomaterial, such as carbon or silica nanoparticles, carbon nanotubes, carbon nanofibers, or carbon quantum dots. Non-limiting examples of carriers include particles or nanoparticles (e.g., particles or nanoparticles made of materials such as carbon, silicon, silicon carbide, gold, tungsten, polymers, or ceramics) in various size ranges and shapes, magnetic particles or nanoparticles (e.g., silenceMag Magnetotransfection TM agent, OZ Biosciences, San Diego, CA), abrasive or scarifying agents, needles or microneedles, matrices, and grids. In certain embodiments, particulates and nanoparticulates are useful in delivery of the polynucleotide composition or the nuclease or both. Useful particulates and nanoparticles include those made of metals (e.g., gold, silver, tungsten, iron, cerium), ceramics (e.g., aluminum oxide, silicon carbide, silicon nitride, tungsten carbide), polymers (e.g., polystyrene, polydiacetylene, and poly(3,4-ethylenedioxythiophene) hydrate), semiconductors (e.g., quantum dots), silicon (e.g., silicon carbide), carbon (e.g., graphite, graphene, graphene oxide, or carbon nanosheets, nanocomplexes, or nanotubes), and composites (e.g., polyvinylcarbazole/graphene, polystyrene/graphene, platinum/graphene, palladium/graphene nanocomposites). In certain embodiments, such particulates and nanoparticulates are further covalently or non-covalently functionalized, or further include modifiers or cross-linked materials such as polymers (e.g., linear or branched polyethylenimine, poly-lysine), polynucleotides (e.g., DNA or RNA), polysaccharides, lipids, polyglycols (e.g., polyethylene glycol, thiolated polyethylene glycol), polypeptides or proteins, and detectable labels (e.g., a fluorophore, an antigen, an antibody, or a quantum dot). In various embodiments, such particulates and nanoparticles are neutral, or carry a positive charge, or carry a negative charge. Embodiments of compositions including particulates include those formulated, e.g., as liquids, colloids, dispersions, suspensions, aerosols, gels, and solids. Embodiments include nanoparticles affixed to a surface or support, e.g., an array of carbon nanotubes vertically aligned on a silicon or copper wafer substrate. Embodiments include polynucleotide compositions including particulates (e.g., gold or tungsten or magnetic particles) delivered by a Biolistic-type technique or with magnetic force. The size of the particles used in Biolistics is generally in the "microparticle" range, for example, gold microcarriers in the 0.6, 1.0, and 1.6 micrometer size ranges (see, e.g., instruction manual for the Helios^{®} Gene Gun System, Bio-Rad, Hercules, CA; Randolph-Anderson et al. (2015) "Submicron gold particles are superior to larger particles for efficient Biolistic^{®} transformation of organelles and some cell types", Bio-Rad US/EG Bulletin 2015), but successful Biolistics delivery using larger nanoparticles has been reported in cultured animal cells; see O'Brian and Lummis (2011) BMC Biotechnol., 11 :66 - 71. Other embodiments of useful particulates are nanoparticles, which are generally in the nanometer (nm) size range or less than 1 micrometer, e.g., with a diameter of less than about 1 nm, less than about 3 nm, less than about 5 nm, less than about 10 nm, less than about 20 nm, less than about 40 nm, less than about 60 nm, less than about 80 nm, and less than about 100 nm. Specific, non-limiting embodiments ofnanoparticles commercially available (all from Sigma-Aldrich Corp., St. Louis, MO) include gold nanoparticles with diameters of 5, 10, or 15 nm; silver nanoparticles with particle sizes of 10, 20, 40, 60, or 100 nm; palladium "nanopowder" of less than 25 nm particle size; single-, double-, and multi-walled carbon nanotubes, e.g., with diameters of 0.7 - 1.1, 1.3 - 2.3, 0.7 - 0.9, or 0. 7 - 1.3 nm, or with nano tube bundle dimensions of 2 - 10 nm by 1- 5 micrometers, 6 - 9 nm by 5 micrometers, 7 - 15 nm by 0.5 - 10 micrometers, 7 - 12 nm by 0.5 - 10 micrometers, 110 - 170 nm by 5 - 9 micrometers, 6 - 13 nm by 2.5 - 20 micrometers. Embodiments include polynucleotide compositions including materials such as gold, silicon, cerium, or carbon, e.g., gold or gold-coated nanoparticles, silicon carbide whiskers, carborundum, porous silica nanoparticles, gelatin/silica nanoparticles, nanoceria or cerium oxide nanoparticles (CNPs), carbon nanotubes (CNTs) such as single-, double-, or multi-walled carbon nanotubes and their chemically functionalized versions (e.g., carbon nanotubes functionalized with amide, amino, carboxylic acid, sulfonic acid, or polyethylene glycol moeities), and graphene or graphene oxide or graphene complexes; see, for example, Wong et al. (2016) Nano Lett., 16: 1161 - 1172; Giraldo et al. (2014) Nature Materials, 13:400-409; Shen et al. (2012) Theranostics, 2:283 - 294; Kim et al. (2011) Bioconjugate Chem., 22:2558 - 2567; Wang et al. (2010) J. Am. Chem. Soc. Comm., 132:9274 - 9276; Zhao et al. (2016) Nanoscale Res. Lett., 11: 195 - 203; and Choi et al. (2016) J. Controlled Release, 235:222 - 235. See also, for example, the various types of particles and nanoparticles, their preparation, and methods for their use, e.g., in delivering polynucleotides and polypeptides to cells, disclosed in US Patent Application Publications 2010/0311168, 2012/0023619, 2012/0244569, 2013/0145488, 2013/0185823, 2014/0096284, 2015/0040268, 2015/0047074, and 2015/0208663.

### iii. Excipients

In some aspects, the composition includes an excipient, e.g., a delivery vehicle, adjuvant, diluent, surfactant, stabilizer, or tonicity agent or a combination thereof. In some embodiments, the excipient is a crop oil concentrate, a vegetable oil concentrate, a modified vegetable oil, a nitrogen source, a deposition (drift control) and/or retention agent (with or without ammonium sulfate and/or defoamer), a compatibility agent, a buffering agent and/or acidifier, a water conditioning agent, a basic blend, a spreader-sticker and/or extender, an adjuvant plus foliar fertilizer, an antifoam agent, a foam marker, a scent, or a tank cleaner and/or neutralizer. In some embodiments, the excipient is an adjuvant described in the Compendium of Herbicide Adjuvants (Young et al. (2016). Compendium of Herbicide Adjuvants (13th ed.), Purdue University).

Examples of delivery vehicles and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate, and mineral oil. Further exemplary delivery vehicles include, but are not limited to, solid or liquid excipient materials, solvents, stabilizers, slow-release excipients, colorings, and surface-active substances (surfactants). In some instances, the excipient (e.g., delivery vehicle) is a stabilizing vehicle. In embodiments, the stabilizing vehicle includes, e.g., an epoxidized vegetable oil, an antifoaming agent, e.g. silicone oil, a preservative, a viscosity regulator, a binding agent, or a tackifier. In some instances, the stabilizing vehicle is a buffer suitable for the recombinant polynucleotide. In some instances, the composition is microencapsulated in a polymer bead delivery vehicle. In some instances, the stabilizing vehicle protects the recombinant polynucleotide against UV and/or acidic conditions. In some instances, the delivery vehicle contains a pH buffer. In some instances, the composition is formulated to have a pH in the range of about 4.5 to about 9.0, including for example pH ranges of about any one of 5.0 to about 8.0, about 6.5 to about 7.5, or about 6.5 to about 7.0.

### iv. Adjuvants

In some instances, the composition provided herein includes an adjuvant. Adjuvants are agents that do not possess effector activity, but impart beneficial properties to a formulation. For example, adjuvants are either pre-mixed in the formulation or added to a spray tank to improve mixing or application or to enhance performance. They are used extensively in products designed for foliar applications. Adjuvants can be used to customize the formulation to specific needs and compensate for local conditions. Adjuvants can be designed to perform specific functions, including wetting, spreading, sticking, reducing evaporation, reducing volatilization, buffering, emulsifying, dispersing, reducing spray drift, and reducing foaming. No single adjuvant can perform all these functions, but compatible adjuvants often can be combined to perform multiple functions simultaneously.

Among nonlimiting examples of adjuvants included in the formulation are binders, dispersants and stabilizers, specifically, for example, casein, gelatin, polysaccharides (e.g., starch, gum arabic, cellulose derivatives, alginic acid, etc.), lignin derivatives, bentonite, sugars, synthetic water-soluble polymers (e.g., polyvinyl alcohol, polyvinylpyrrolidone, polyacrylic acid, etc.), PAP (acidic isopropyl phosphate), BHT (2,6-di-t-butyl-4-methylphenol), BHA (a mixture of 2-t-butyl-4-methoxyphenol and 3-t-butyl-4-methoxyphenol), vegetable oils, mineral oils, fatty acids and fatty acid esters.

### v. Liquid and Gaseous Formulations

In embodiments, the compositions provided herein are in a liquid formulation. Liquid formulations are generally mixed with water, but in some instances are used with crop oil, diesel fuel, kerosene or other light oil as an excipient. The amount of active ingredient (e.g., recombinant polynucleotides) often ranges from about 0.5 to about 80 percent by weight.

In embodiments, an emulsifiable concentrate formulation contains a liquid active ingredient, one or more petroleum-based solvents, and an agent that allows the formulation to be mixed with water to form an emulsion. Such concentrates can be used in agricultural, ornamental and turf, forestry, structural, food processing, livestock, and public health pest formulations. In embodiments, these are adaptable to application equipment from small portable sprayers to hydraulic sprayers, low-volume ground sprayers, mist blowers, and low-volume aircraft sprayers. Some active ingredients readily dissolve in a liquid excipient. When mixed with an excipient, they form a solution that does not settle out or separate, e.g., a homogenous solution. In embodiments, formulations of these types include an active ingredient, a carrier and/or an excipient, and one or more other ingredients. Solutions can be used in any type of sprayer, indoors and outdoors.

In some instances, the composition is formulated as an invert emulsion. An invert emulsion is a water-soluble active ingredient dispersed in an oil excipient. Invert emulsions require an emulsifier that allows the active ingredient to be mixed with a large volume of petroleum-based excipient, usually fuel oil. Invert emulsions aid in reducing drift. With other formulations, some spray drift results when water droplets begin to evaporate before reaching target surfaces; as a result the droplets become very small and lightweight. Because oil evaporates more slowly than water, invert emulsion droplets shrink less and more active ingredient reaches the target. Oil further helps to reduce runoff and improve rain resistance. It further serves as a sticker-spreader by improving surface coverage and absorption. Because droplets are relatively large and heavy, it is difficult to get thorough coverage on the undersides of foliage. Invert emulsions are most commonly used along rights-of-way where drift to susceptible non-target areas can be a problem.

A flowable or liquid formulation combines many of the characteristics of emulsifiable concentrates and wettable powders. Manufacturers use these formulations when the active ingredient is a solid that does not dissolve in either water or oil. The active ingredient, impregnated on a substance such as clay, is ground to a very fine powder. The powder is then suspended in a small amount of liquid. The resulting liquid product is quite thick. Flowables and liquids share many of the features of emulsifiable concentrates, and they have similar disadvantages. They require moderate agitation to keep them in suspension and leave visible residues, similar to those of wettable powders.

Flowables/liquids are easy to handle and apply. Because they are liquids, they are subject to spilling and splashing. They contain solid particles, so they contribute to abrasive wear of nozzles and pumps. Flowable and liquid suspensions settle out in their containers. Because flowable and liquid formulations tend to settle, packaging in containers of 18.93 litres (5 gallons) or less makes remixing easier. Aerosol formulations contain one or more active ingredients and a solvent. Most aerosols contain a low percentage of active ingredients. There are two types of aerosol formulations-the ready-to-use type commonly available in pressurized sealed containers and those products used in electrical or gasoline-powered aerosol generators that release the formulation as a smoke or fog.

Ready to use aerosol formulations are usually small, self-contained units that release the formulation when the nozzle valve is triggered. The formulation is driven through a fine opening by an inert gas under pressure, creating fine droplets. These products are used in greenhouses, in small areas inside buildings, or in localized outdoor areas. Commercial models, which hold five to 2.27 kg (5 pounds) of active ingredient, are usually refillable.

Smoke or fog aerosol formulations are not under pressure. They are used in machines that break the liquid formulation into a fine mist or fog (aerosol) using a rapidly whirling disk or heated surface.

In some embodiments, the composition comprises a liquid excipient. In embodiments, a liquid excipient includes, for example, aromatic or aliphatic hydrocarbons (e.g., xylene, toluene, alkylnaphthalene, phenylxylylethane, kerosene, gas oil, hexane, cyclohexane, etc.), halogenated hydrocarbons (e.g., chlorobenzene, dichloromethane, dichloroethane, trichloroethane, etc.), alcohols (e.g., methanol, ethanol, isopropyl alcohol, butanol, hexanol, benzyl alcohol, ethylene glycol, etc.), ethers (e.g., diethyl ether, ethylene glycol dimethyl ether, diethylene glycol monomethyl ether, diethylene glycol monoethyl ether, propylene glycol monomethyl ether, tetrahydrofuran, dioxane, etc.), esters (e.g., ethyl acetate, butyl acetate, etc.), ketones (e.g., acetone, methyl ethyl ketone, methyl isobutyl ketone, cyclohexanone, etc.), nitriles (e.g., acetonitrile, isobutyronitrile, etc.), sulfoxides (e.g., dimethyl sulfoxide, etc.), amides (e.g., N,N-dimethylformamide, N,N-dimethylacetamide, cyclic imides (e.g. N-methylpyrrolidone) alkylidene carbonates (e.g., propylene carbonate, etc.), vegetable oil (e.g., soybean oil, cottonseed oil, etc.), vegetable essential oils (e.g., orange oil, hyssop oil, lemon oil, etc.), or water.

In some embodiments, the composition comprises a gaseous excipient. Gaseous excipients include, for example, butane gas, flon gas, liquefied petroleum gas (LPG), dimethyl ether, and carbon dioxide gas.

### vi. Dry or Solid Formulations

Dry formulations can be divided into two types: ready-to-use and concentrates that must be mixed with water to be applied as a spray. Most dust formulations are ready to use and contain a low percentage of active ingredients (less than about 10 percent by weight), plus a very fine, dry inert excipient made from talc, chalk, clay, nut hulls, or volcanic ash. The size of individual dust particles varies. A few dust formulations are concentrates and contain a high percentage of active ingredients. Mix these with dry inert excipients before applying. Dusts are always used dry and can easily drift to non-target sites.

### vii. Granule or Pellet Formulations

In some instances, the composition is formulated as granules. Granular formulations are similar to dust formulations, except granular particles are larger and heavier. In embodiments, the coarse particles are made from materials such as clay, corncobs, or walnut shells. The active ingredient either coats the outside of the granules or is absorbed into them. In embodiments, the amount of active ingredient is relatively low, usually ranging from about 0.5 to about 15 percent by weight. Granular formulations are most often used to apply to the soil, insects or nematodes living in the soil, or absorption into plants through the roots. Granular formulations are sometimes applied by airplane or helicopter to minimize drift or to penetrate dense vegetation. Once applied, granules can release the active ingredient slowly. Some granules require soil moisture to release the active ingredient. Granular formulations also are used to control larval mosquitoes and other aquatic pests. Granules are used in agricultural, structural, ornamental, turf, aquatic, right-of-way, and public health (biting insect) pest-control operations.

In some instances, the composition is formulated as pellets. Most pellet formulations are very similar to granular formulations; the terms are used interchangeably. In a pellet formulation, however, all the particles are the same weight and shape. The uniformity of the particles allows use with precision application equipment.

### viii. Powders

In some instances, the composition is formulated as a powder. In some instances, the composition is formulated as a wettable powder. Wettable powders are dry, finely ground formulations that look like dusts. They usually must be mixed with water for application as a spray. A few products, however, can be applied either as a dust or as a wettable powder-the choice is left to the applicator. Wettable powders have about 1 to about 95 percent active ingredient by weight; in some cases more than about 50 percent. The particles do not dissolve in water. They settle out quickly unless constantly agitated to keep them suspended. They can be used for most pest problems and in most types of spray equipment where agitation is possible. Wettable powders have excellent residual activity. Because of their physical properties, most of the formulation remains on the surface of treated porous materials such as concrete, plaster, and untreated wood. In such cases, only the water penetrates the material.

In some instances, the composition is formulated as a soluble powder. Soluble powder formulations look like wettable powders. However, when mixed with water, soluble powders dissolve readily and form a true solution. After they are mixed thoroughly, no additional agitation is necessary. The amount of active ingredient in soluble powders ranges from about 15 to about 95 percent by weight; in some cases more than about 50 percent. Soluble powders have all the advantages of wettable powders and none of the disadvantages, except the inhalation hazard during mixing.

In some instances, the composition is formulated as a water-dispersible granule. Water-dispersible granules, also known as dry flowables, are like wettable powders, except instead of being dust-like, they are formulated as small, easily measured granules. Water-dispersible granules must be mixed with water to be applied. Once in water, the granules break apart into fine particles similar to wettable powders. The formulation requires constant agitation to keep it suspended in water. The percentage of active ingredient is high, often as much as 90 percent by weight. Water-dispersible granules share many of the same advantages and disadvantages of wettable powders, except they are more easily measured and mixed. Because of low dust, they cause less inhalation hazard to the applicator during handling

In some embodiments, the composition comprises a solid excipient. Solid excipients include finely-divided powder or granules of clay (e.g. kaolin clay, diatomaceous earth, bentonite, Fubasami clay, acid clay, etc.), synthetic hydrated silicon oxide, talc, ceramics, other inorganic minerals (e.g., sericite, quartz, sulfur, activated carbon, calcium carbonate, hydrated silica, etc.), a substance which can be sublimated and is in the solid form at room temperature (e.g., 2,4,6-triisopropyl-1,3,5-trioxane, naphthalene, p-dichlorobenzene, camphor, adamantan, etc.); wool; silk; cotton; hemp; pulp; synthetic resins (e.g., polyethylene resins such as low-density polyethylene, straight low-density polyethylene and high-density polyethylene; ethylene-vinyl ester copolymers such as ethylene-vinyl acetate copolymers; ethylene-methacrylic acid ester copolymers such as ethylene-methyl methacrylate copolymers and ethylene-ethyl methacrylate copolymers; ethylene-acrylic acid ester copolymers such as ethylene-methyl acrylate copolymers and ethylene-ethyl acrylate copolymers; ethylene-vinylcarboxylic acid copolymers such as ethylene-acrylic acid copolymers; ethylene-tetracyclododecene copolymers; polypropylene resins such as propylene homopolymers and propylene-ethylene copolymers; poly-4-methylpentene-1, polybutene-1, polybutadiene, polystyrene; acrylonitrile-styrene resins; styrene elastomers such as acrylonitrile-butadiene-styrene resins, styrene-conjugated diene block copolymers, and styrene-conjugated diene block copolymer hydrides; fluororesins; acrylic resins such as poly(methyl methacrylate); polyamide resins such as nylon 6 and nylon 66; polyester resins such as polyethylene terephthalate, polyethylene naphthalate, polybutylene terephthalate, and polycyclohexylenedimethylene terephthalate; polycarbonates, polyacetals, polyacrylsulfones, polyarylates, hydroxybenzoic acid polyesters, polyetherimides, polyester carbonates, polyphenylene ether resins, polyvinyl chloride, polyvinylidene chloride, polyurethane, and porous resins such as foamed polyurethane, foamed polypropylene, or foamed ethylene, etc.), glasses, metals, ceramics, fibers, cloths, knitted fabrics, sheets, papers, yarn, foam, porous substances, and multifilaments.

### ix. Nanocapsules/Microencapsulation/Liposomes

In some instances, the composition is provided in a microencapsulated formulation. Microencapsulated formulations are mixed with water and sprayed in the same manner as other sprayable formulations. After spraying, the plastic coating breaks down and slowly releases the active ingredient.

In some instances, the composition is provided in a liposome. In some instances, the composition is provided in a vesicle.

### x. Surfactants

In some instances, the composition provided herein includes a surfactant. Surfactants, also called wetting agents and spreaders, physically alter the surface tension of a spray droplet. For a formulation to perform its function properly, a spray droplet must be able to wet the foliage and spread out evenly over a leaf. Surfactants enlarge the area of formulation coverage, thereby increasing exposure to the active agent. Surfactants are particularly important when applying a formulation to waxy or hairy leaves. Without proper wetting and spreading, spray droplets often run off or fail to cover leaf surfaces adequately. Too much surfactant, however, can cause excessive runoff and reduce efficacy. Surfactants are classified by the way they ionize or split apart into electrically charged atoms or molecules called ions. A surfactant with a negative charge is anionic. One with a positive charge is cationic, and one with no electrical charge is nonionic. Formulation activity in the presence of a nonionic surfactant can be quite different from activity in the presence of a cationic or anionic surfactant. Selecting the wrong surfactant can reduce the efficacy of a product and injure the target plant. Anionic surfactants are most effective when used with contact pesticides (pesticides that control a pest by direct contact rather than being absorbed systemically). Cationic surfactants should never be used as stand-alone surfactants because they usually are phytotoxic.

Nonionic surfactants, often used with systemic pesticides, help sprays penetrate plant cuticles. Nonionic surfactants are compatible with most pesticides, and most EPA-registered pesticides that require a surfactant recommend a nonionic type. Adjuvants include, but are not limited to, stickers, extenders, plant penetrants, compatibility agents, buffers or pH modifiers, drift control additives, defoaming agents, and thickeners.

Among nonlimiting examples of surfactants included in the compositions described herein are alkyl sulfate ester salts, alkyl sulfonates, alkyl aryl sulfonates, alkyl aryl ethers and polyoxyethylenated products thereof, polyethylene glycol ethers, polyvalent alcohol esters and sugar alcohol derivatives. In some embodiments, the surfactant is a nonionic surfactant, a surfactant plus nitrogen source, an organosilicone surfactant, or a high surfactant oil concentrate.

### xi. Combinations

In formulations and in the use forms prepared from these formulations, the recombinant polynucleotide can, in embodiments, be in a mixture with other active compounds, such as pesticidal agents (e.g., insecticides, sterilants, acaricides, nematicides, molluscicides, or fungicides, attractants, growth-regulating substances, or herbicides. As used herein, the term "pesticidal agent" refers to any substance or mixture of substances intended for preventing, destroying, repelling, or mitigating any pest. A pesticide can be a chemical substance or biological agent used against pests including insects, mollusks, pathogens, weeds, nematodes, and microbes that compete with humans for food, destroy property, spread disease, or are a nuisance. The term "pesticidal agent" further encompasses other bioactive molecules such as antibiotics, antivirals pesticides, antifungals, antihelminthics, nutrients, pollen, sucrose, and/or agents that stun or slow insect movement.

In instances where the recombinant polynucleotide is applied to plants, a mixture with other known compounds, such as herbicides, fertilizers, growth regulators, safeners, semiochemicals, or else with agents for improving plant properties is also possible.

### II. Effectors

Provided herein are compositions comprising recombinant polynucleotides comprising (i) a single-stranded plant RNA (ssRNA) viroid sequence of a viroid from the family Avsunviroidaeand (ii) a heterologous RNA sequence that is not a plant viroid sequence and that comprises or encodes an effector, wherein (ii) is inserted into (i); and wherein the recombinant polynucleotide is capable of replicating in a plant, and wherein the effector comprises a double-stranded RNA stem comprising 50 - 100 base pairs . The effector can be any moiety that can be integrated into a recombinant polynucleotide comprising an ssRNA viroid sequence (e.g., a recombinant polynucleotide, e.g., a viroid-derived vector) and that has a biological effect on (e.g., is capable of modulating a state of) an organism or a cell thereof, e.g., has a biological effect on a plant or a plant cell, e.g., modulates a trait, phenotype, or genotype in a plant, plant part, or plant cell, as described in Section IIIB herein.

In some embodiments, recombinant polynucleotide comprises an RNA sequence (e.g., an ssRNA sequence) that comprises or consists of the effector. In other embodiments, the recombinant polynucleotide comprises an RNA sequence (e.g., an ssRNA sequence) that is modified to produce the effector, e.g., is processed in a cell (e.g., a target cell) to produce the effector. In still other embodiments, the recombinant polynucleotide comprises an RNA sequence (e.g., an ssRNA sequence) that encodes the effector, e.g., an RNA sequence that is translated in a cell (e.g., a target cell) to produce a protein or polypeptide effector. In some embodiments, the RNA sequence is an ssRNA sequence.

In some embodiments, the effector consists of or comprises a coding sequence. In some embodiments, the coding sequence is a protein or a polypeptide.

In some embodiments, the effector consists of or comprises a regulatory RNA, e.g., a long non-coding RNA (IncRNA), a circular RNA (circRNA), a transfer RNA-derived fragment (tRF), a transfer RNA (tRNA), a ribosomal RNA (rRNA), a small nuclear RNA (snRNA), a small nucleolar RNA (snoRNA), or a Piwi-interacting RNA (piRNA).

In some embodiments, the effector consists of or comprises an interfering RNA, e.g., a small RNA (sRNA), a double-stranded RNA (dsRNA); a hairpin RNA (hpRNA), a microRNA (miRNA); a pre-miRNA; a phased, secondary, small interfering RNA (phasiRNA); a heterochromatic small interfering RNA (hcsiRNA); or a natural antisense short interfering RNA (natsiRNA).

In some embodiments, the effector comprises or consists of a hairpin RNA (hpRNA) targeting a transcript of the plant cell.

In some embodiments, the effector comprises or consists of a small RNA (sRNA) targeting a transcript of the plant cell.

In some embodiments, the effector comprises or consists of a pre-miRNA targeting a transcript of the plant cell.

In some embodiments, the effector comprises or consists of a circRNA corresponding to a gene of the plant cell.

In some embodiments, the effector comprises or consists of an RNA sequence corresponding to a gene or gene transcript of the plant cell.

Exemplary genes that can be targeted by effectors (e.g., regulatory or interfering RNAs) include, e.g., genes encoding hormones, enzymes, and transcription factors.

In some embodiments, the effector consists of or comprises a guide RNA (e.g., a guide RNA for use in combination with a gene editing enzyme). In some embodiments, the effector comprises or consists of a guide RNA (gRNA) targeting a gene of the plant cell.

In some embodiments, the effector comprises or consists of an aptamer, e.g., a DNA aptamer, RNA aptamer, or peptide aptamer.

In some aspects, the effector (e.g., RNA, polypeptide, or protein effector) binds a target cell host factor. In embodiments, the target cell host factor is, e.g., a nucleic acid, a protein, a DNA, or an RNA.

The effector sequence is heterologous with respect to the ssRNA plant viroid sequence that is a plant viroid genome or a derivative thereof, wherein the plant viroid is from the family Avsunviroidae or sequences included in the recombinant polynucleotide. The RNA sequence comprising or encoding the effector is not a viroid sequence, e.g., is not derived from the sequence of any viroid. In embodiments, the RNA sequence is, e.g., an artificial sequence or a sequence derived from another organism.

In some embodiments, the recombinant polynucleotide comprises or encodes a single effector. In other embodiments, the recombinant polynucleotide comprises or encodes at least two effectors, e.g., 2, 3, 4, 5, 6, 7, 8, 9, 10, or more than 10 effectors.

### CRISPR guide RNAs:

In some embodiments, the effector is a CRISPR guide RNA. CRISPR-associated endonucleases such as Cas9, Cas12 and Cas13 endonucleases are used as genome editing tools in different plants; see, e.g., Wolter et al. (2019) BMC Plant Biol., 19:176-183); Aman et al. (2018) Genome Biol., 19:1-10. CRISPR/Cas9 requires a two-component crRNA:tracrRNA "guide RNA" ("gRNA") that contains a targeting sequence (the "CRISPR RNA" or "crRNA" sequence) and a Cas9 nuclease-recruiting sequence (tracrRNA). Efficient Cas9 gene editing is also achieved with the use of a chimeric "single guide RNA" ("sgRNA"), an engineered (synthetic) single RNA molecule that mimics a naturally occurring crRNA-tracrRNA complex and contains both a tracrRNA (for binding the nuclease) and at least one crRNA (to guide the nuclease to the sequence targeted for editing); see, for example, Cong et al. (2013) Science, 339:819-823; Xing et al. (2014) BMC Plant Biol., 14:327-340. Chemically modified sgRNAs have been demonstrated to be effective in genome editing; see, for example, Hendel et al. (2015) Nature Biotechnol., 985-991. Commercial manufacturers of CRISPR nucleases and guide RNAs provide algorithms for designing guide RNA sequences; see, e.g., guide design tools provided by Integrated DNA Technologies at www[dot]idtdna[dot]com/pages/products/crispr-genome-editing/alt-r-crispr-cas9-system. Some Cas nucleases, including Cas12a and Cas13, do not require a tracrRNA.

For many Cas nucleases, guide sequence designs are constrained by the requirement that the DNA target sequence (to which the crRNA is designed to be complementary) must be adjacent to a proto-spacer adjacent motif ("PAM") sequence that is recognized by the specific Cas nuclease to be employed. Cas nucleases recognize specific PAM sequences and there is a diversity of nucleases and corresponding PAM sequences; see, e.g., Smakov et al. (2017) Nature Reviews Microbial.,

doi:10.1038/nrmicro.2016.184. For example, Cas9 nucleases cleave dsDNA, require a GC-rich PAM sequence located 3' to the DNA target sequence to be targeted by the crRNA component of the guide RNA, and cleave leaving blunt ends. Cas12a nucleases cleave dsDNA, require a T-rich PAM sequence located 5' to the DNA target sequence to be targeted by the crRNA component of the guide RNA, and cleave leaving staggered ends with a 5' overhang. Cas13 nucleases cleave single-stranded RNAs and do not require a PAM sequence; instead, Cas13 nuclease are guided to their targets by a single crRNA with a direct repeat ("DR"). In practice, the crRNA component of a guide RNA is generally designed to have a length of between 17 - 24 nucleotides (frequently 19, 20, or 21 nucleotides) and exact complementarity (i. e., perfect base-pairing) to the targeted gene or nucleic acid sequence that is itself adjacent to a PAM motif (when required by the Cas nuclease). A crRNA component having less than 100% complementarity to the target sequence can be used (e. g., a crRNA with a length of 20 nucleotides and between 1 - 4 mismatches to the target sequence) but this increases the potential for off-target effects.

Non-limiting examples of effective guide design are found, e.g., in US Patent Application Publications US 2019/0032131, 2015/0082478, and 2019/0352655. For the purposes of gene editing, CRISPR "arrays" can be designed to include one or multiple guide RNA sequences corresponding to one or more desired target DNA sequence(s); see, for example, Cong et al. (2013) Science, 339:819-823; Ran et al. (2013) Nature Protocols, 8:2281-2308.

In an embodiment, an effector moiety integrated into a polynucleotide includes at least one CRISPR guide RNA; release of the guide RNA is mediated, e.g., by flanking DR sequences, ribozyme sequences, or other self-cleaving RNAs, or by cleavage by an endogenous ribonuclease. The corresponding Cas nuclease can be provided by separate or concurrent delivery, e.g., by co-delivery with the viroid-derived vector or polynucleotide, or by transient or stable expression of the corresponding Cas nuclease in the cell to which the viroid-derived vector or polynucleotide is delivered.

### siRNAs and ta-siRNAs

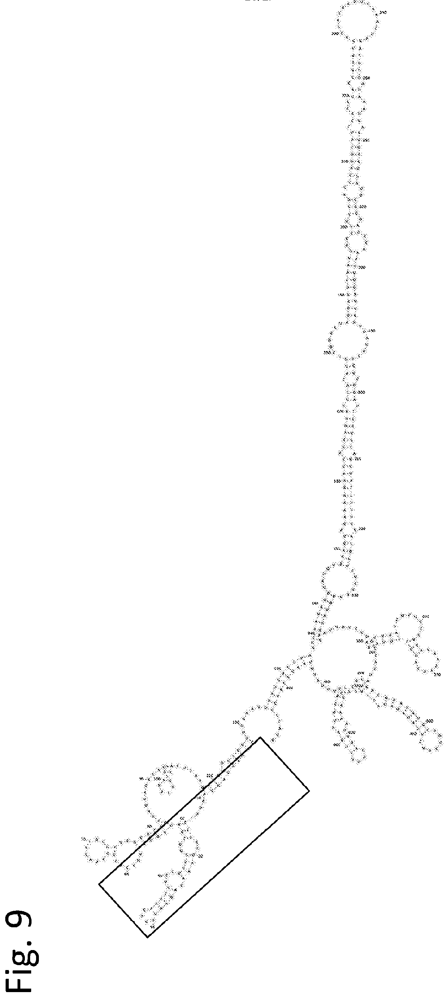

In some embodiments, the effector is a siRNA or a ta-siRNA. A double-stranded RNA (e.g., a dsRNA made of two separate hybridizing RNA strands, or a single RNA strand that forms a stem-loop structure) that includes complementary or hybridizing "sense" and "anti-sense" RNA segments that correspond to (i.e., are respectively identical to, or complementary to, a target gene) can be processed by DICER into asymmetric hybridized pairs of small interfering RNAs (siRNAs) of usually 20 - 24 base pairs, most often 21 - 23 base pairs, with 2-nucleotide 3' overhangs. A hybridized pair of siRNAs is complexed with multiple proteins to form the RNA-induced silencing complex ("RISC"); one strand is preferably bound to the protein Argonaute and acts as a "guide" for the RISC complex in binding to and directing cleavage of a target transcript. In embodiments, the resulting siRNAs silence or decrease the expression of the target gene. In some embodiments, the siRNAs silence transposable elements in heterochromatin. The target gene can be a (protein-) coding or non-coding nucleotide sequence or a combination of coding and non-coding sequence, and can be endogenous to the cell to which the siRNAs are provided, or can be exogenous (e.g., a viral sequence). Various arrangements and combinations of sense and/or anti-sense RNA segments (such as those resulting from transcription of the DNA molecules depicted in Figure 9 of US Patent No. 9,708,620) can be provided in a single contiguous polynucleotide, or in multiple, non-contiguous polynucleotide segments, or in multiple polynucleotides, which are processed into siRNAs for silencing or decreasing expression of a given target gene. Selection of sequences for siRNA production is known in the art; see, for example, Wang & Mu (2004), Bioinformatics, 20:1818-1820; Reynolds et al. (2004), Nature Biotechnol., 22:326-330; and Yuan et al. (2004), Nucleic Acids Res., 32:W130-W134. Mismatches between a given siRNA guide sequence and a target transcript can be tolerated, particularly where multiple different siRNAs generated from longer dsRNA stems provide improved overall efficacy, and therefore siRNAs are designed to have at least about 70% complementarity to a segment of equivalent length in the target transcript. However, generally it is most convenient and simplest to design an siRNA that has exact complementarity to the target gene sequence.

In an embodiment, an effector moiety integrated into a polynucleotide includes at least one double-stranded RNA stem comprising 50 - 100 base pairs designed to be processed into siRNAs for RNAi-mediated silencing or decrease of expression of a target gene. In other embodiments, the plant viroid-derived vector or polynucleotide includes at least one effector moiety that includes one or more double-stranded RNA stems comprising 50 - 100 base pairs designed to be processed into siRNAs for RNAi-mediated silencing or decrease of one or more target genes. The length of a double-stranded RNA stem is selected for efficacy and convenience. For efficacy, the double-stranded RNA stem includes 50 - 100 contiguous base pairs, and where the viroid-derived vector or polynucleotide is a circular RNA, the double-stranded RNA stem includes 50 - 100 base pairs; see, e.g., Abe et al. (2007) J. Am.Chem. Soc., 129:15108 - 15109. The double-stranded RNA stem can be longer, in the order of a few hundred base pairs, e.g., 80 - 150, 100 - 200, 150 - 250, or 200 - 300 base pairs. For convenience and economy of production and in consideration of steric effects, generally the overall length of a given double-stranded RNA stem is no longer than necessary to obtain the desired level of silencing or decrease in expression of the target gene(s).

Alternatively, multiple siRNAs can be produced from a single-stranded RNA transcript designed to generate multiple "trans-acting siRNAs" (ta-siRNAs). Production of ta-siRNAs is initiated by cleavage of the ssRNA transcript at a 5'-proximal site, followed by amplification of the 3' RNA product by RNA-dependent RNA polymerase 6 (RDR6) and processing by a specific Dicer-type enzyme, DCL4, to yield phased siRNAs (the "ta-siRNAs") that are produced in 21-nucleotide register with the cleavage site and therefore can be designed to target specific genes. See, e.g., US Patent Nos. 8,030,473, 8,476,422, 8,816,061, and 9,018,002. Also see, e.g., Allen et al. (2005) Cell, 121:207 - 221 and Cuperus et al. (2010) Nature Structural Mol. Biol., 17:997 - 1003.

### miRNAs, phased small RNAs:

In an embodiment, an effector integrated into a polynucleotide includes at least one microRNA (miRNA) precursor, which can have the sequence of a naturally occurring miRNA precursor that is processed into a naturally occurring mature miRNA, or of an artificial (synthetic) miRNA precursor sequence that is processed into an artificial mature miRNA. Mature miRNAs are small RNAs, typically of 19, 20, 21, 22, 23, 24, 25, or 26 nucleotides in length; a mature miRNA binds to complementary sequences ("miRNA recognition site") in gene transcripts, leading to cleavage and silencing or decreasing expression of that gene. In nature, a mature miRNA is encoded in a miRNA precursor, a single-stranded RNA transcript that forms a fold-back structure typically including an imperfect stem-loop containing mismatches and "bulges", at least some of which occur between the nucleotides that make up the mature miRNA and the nucleotides that make up the so-called "mir*" ("mir-star") sequence located on the opposite strand of the precursor's stem. Upon normal processing within the cell, the mature miRNA is released. Numerous naturally occurring miRNAs and their corresponding miRNA precursors have been described and can be accessed on a public, searchable database, miRbase (available on line at www[dot]miRbase[dot]org); see, e.g., Kozomara et al. (2019) Nucleic Acids Res., 47:D155 - D162; Griffiths-Jones et al. (2008) Nucleic Acids Res., 36:D154 - D158; Griffiths-Jones et al. (2004) Nucleic Acids Res., 32:D109 - D111. Artificial mature miRNAs for "silencing" a selected target gene are designed to have a sequence that is complementary to the target gene's transcript, thus allowing the artificial mature miRNA to specifically bind to and cleave the RNA transcript of that target gene. In an embodiment, an engineered miRNA precursor molecule is designed using as a design scaffold or template a native miRNA precursor sequence that encodes a native mature miRNA, with nucleotides of the native mature miRNA replaced with nucleotides that are complementary to the target RNA transcript, while maintaining the position and number of mismatches in the stem portion of the miRNA precursor's fold-back structure by altering as needed the miR* nucleotides in the precursor strand, and generally leaving the remaining nucleotides of the miRNA precursor unchanged. Non-limiting examples illustrating the design of such artificial miRNAs based on naturally occurring plant or invertebrate miRNA precursors are described respectively in, e.g., US Patent Nos. 7,786,350, 8,395,023, 8,946,511, and 9,708,620, and US Patent Nos. 8,410,334 and 10,570,414.

One general, non-limiting method for selecting a nucleotide sequence for an artificial mature miRNA (therefore determining nucleotide changes in the native miRNA precursor sequence to produce the artificial miRNA precursor) includes these steps:
Selecting a unique target sequence of at least 18 nucleotides, preferably at least 19 nucleotides, that is specific to the target gene, thereby avoiding unintentional silencing of non-target sequences, e.g., by using sequence alignment tools such as BLAST (see, for example, Altschul et al. (1990) J. Mol. Biol., 215:403-410; Altschul et al. (1997) Nucleic Acids Res., 25:3389-3402), to identify from genomic sequence any target transcript orthologues and any potential matches to unrelated genes that should be avoided;
Scoring each potential 19-mer segment along the length of the target gene for GC content, Reynolds score (see Reynolds et al. (2004) Nature Biotechnol., 22:326-330), and functional asymmetry characterized by a negative difference in free energy ("ΔΔG") (see Khvorova et al. (2003) Cell, 115:209-216); preferably 19-mers are selected that have all or most of the following characteristics: (1) a Reynolds score > 4, (2) a GC content between about 40% to about 60%, (3) a negative "ΔΔG", (4) a terminal adenosine, (5) lack of a consecutive run of 4 or more of the same nucleotide; (6) a location near the 3' terminus of the target gene; (7) minimal differences from the miRNA precursor transcript;
Determining the reverse complement of the selected 19-mers to use in making a modified mature miRNA; the additional nucleotide at position 20 is preferably matched to the selected target sequence, and the nucleotide at position 21 is preferably chosen to be unpaired to prevent spreading of silencing on the target transcript;
Optionally, testing the engineered miRNA precursor, for example, in an *Agrobacterium*-mediated transient *Nicotiana benthamiana* assay, for efficacy. Multiple 19-mers can be selected for testing, in which case the most effective engineered miRNA precursor sequence(s) can be selected for further use.

Similarly, phased small RNAs such as those described in US Patent Nos. 8,404,928 and 9,309,512 can be engineered to bind and cleave one or multiple selected RNA transcripts. The phased small RNA precursor, which contains multiple ~21-mer small RNAs, forms an extended imperfect stem-loop containing mismatches and bulges. Any one or more of the contiguous 21-mers found within this precursor can be designed to bind to and cleave a target RNA. Phased small RNAs can be designed in a manner similar to that used for designing an artificial miRNA using the criteria for selecting a nucleotide sequence for an artificial mature miRNA described above. The artificial phased small RNA precursor can be tested and the most effective phased small RNAs can be selected for further use.

### III. Methods of Use

### A. Methods of delivery to plants

In some aspects, the disclosure features a method of delivering an effector to a plant, a plant tissue, or a plant cell, the method comprising providing to the plant, plant tissue, or plant cell a composition described herein (e.g., a composition comprising or consisting of a recombinant polynucleotide (e.g., a vector) comprising (i) a single-stranded RNA (ssRNA) plant viroid sequence of a viroid from the family Avsunviroidae and (ii) a heterologous RNA sequence that is not a plant viroid sequence and that comprises or encodes an effector, wherein (ii) is inserted into (i); and wherein the recombinant polynucleotide replicates in the plant, whereby the effector comprised by or encoded by the heterologous RNA sequence is delivered to the plant. In embodiments, the effector is, e.g., any effector described in Section II herein. In some embodiments, providing comprises contacting the plant with the recombinant polynucleotide.

In some aspects, the disclosure features a plant cell comprising a composition of the disclosure.

### i. Methods of delivery

A plant described herein can be exposed to any of the compositions described herein in any suitable manner that permits delivering or administering the composition to the plant. The modulating agent can be delivered either alone or in combination with other active or inactive substances and can be applied by, for example, spraying, leaf rubbing, microinjection, in a hydroponic system (e.g., to roots), pouring, dipping, in the form of concentrated liquids, gels, solutions, suspensions, sprays, powders, pellets, briquettes, bricks and the like, formulated to deliver an effective concentration of the recombinant polynucleotide. Amounts and locations for application of the compositions described herein are generally determined by the anatomy and physiology of the plant, the lifecycle stage at which the recombinant polynucleotide is to be delivered, the site where the application is to be made, and the physical and functional characteristics of the recombinant polynucleotide.

In some instances, the composition is sprayed directly onto a plant, e.g., by backpack spraying, aerial spraying, crop spraying/dusting, etc.. The plant receiving the recombinant polynucleotide can be at any stage of plant growth. For example, recombinant polynucleotides can be applied as a seed-coating or root treatment in early stages of plant growth or as a total plant treatment at later stages of the crop cycle. Further, the recombinant polynucleotide can be applied as a systemic agent (e.g., in the soil in which a plant grows, in the water that is used to water the plant, or in a hydroponic system in which the plant is grown or cultured) that is absorbed and distributed through the tissues (e.g., roots, leaves, and/or stems) of a plant. In some instances, plants are genetically transformed to express the recombinant polynucleotide. In some non-claimed embodiments, systemic or transgenic applications are used such that an insect, mollusk, nematode, or fungus feeding on the plant will obtain an effective dose of the recombinant polynucleotide and/or the effector.

Delayed or continuous release can be accomplished by coating the recombinant polynucleotide or a composition containing the recombinant polynucleotide with a dissolvable or bioerodable coating layer, such as gelatin, which coating dissolves or erodes in the environment of use, to then make the recombinant polynucleotide available, or by dispersing the agent in a dissolvable or erodable matrix. Such continuous release and/or dispensing means devices can be advantageously employed to consistently maintain an effective concentration of one or more of the recombinant polynucleotides described herein in a specific host habitat.

In some embodiments, providing the composition to the plant comprises delivering the composition to a leaf, root, stem, flower, seed, xylem, phloem, apoplast, symplast, meristem, fruit, embryo, microspore, pollen, pollen tube, ovary, ovule, or explant for transformation of the plant. In some embodiments, the plant is a monocot or a dicot. In some embodiments, the plant cell is a protoplast. In some embodiments, the fruit is pre-harvest fruit. In other embodiments, the fruit is a post-harvest fruit.

In some embodiments, the recombinant polynucleotide is delivered to the plant using a method described in U.S. Patent number 10597676, 10655136, 9121022, 10378012, or 8367895 or PCT publication WO2018140899 or WO2018085693.

### Delivery to the nucleus of a plant cell

In some disclosed but non-claimed aspects, the disclosure provides a method of delivering an RNA effector to the nucleus of a plant cell, comprising contacting a plant cell with a synthetic nuclear transporter comprising: (i) a single-stranded RNA (ssRNA) viroid sequence and (ii) a heterologous RNA sequence comprising or encoding an effector, wherein the ssRNA viroid sequence does not include a chloroplast localization sequence; wherein the synthetic nuclear transporter localizes to the nucleus of the plant cell, thereby delivering the effector to the nucleus.

In some disclosed but non-claimed aspects, the ssRNA viroid sequence has at least 80% sequence identity with a pospiviroid sequence.

In some disclosed but non claimed aspects, the ssRNA viroid sequence has at least 80% sequence identity to a sequence selected from the group consisting of SEQ ID NOs:51-54, SEQ ID NOs:65-66, SEQ ID NO:68, SEQ ID NO:75, SEQ ID NOs:77-79, SEQ ID NOs:84-96, SEQ ID NOs:98-107, SEQ ID NOs:123-124, SEQ ID NOs:126-132, SEQ ID NO:134, SEQ ID NOs:136-143, SEQ ID NOs:145-150, SEQ ID NOs:153-154, SEQ ID NO:159, SEQ ID NO:166, SEQ ID NO:168, SEQ ID NO:196, SEQ ID NO:242, SEQ ID NO:268, SEQ ID NO:274, SEQ ID NO:276, SEQ ID NO:289, SEQ ID NO:451, SEQ ID NOs:458-459, and SEQ ID NO:467. In some disclosed but non claimed aspects, the ssRNA viroid sequence has at least 90% sequence identity with SEQ ID NO:51.

In some aspects, the heterologous RNA sequence comprises coding RNA, non-coding RNA, or both coding and non-coding RNA.

In some aspects, the effector comprises coding RNA, non-coding RNA, or both coding and non-coding RNA.

In some aspects, the effector comprises non-coding RNA comprising at least one regulatory RNA or at least one interfering RNA that targets a transcript in a plant cell.

In some aspects, the disclosure provides a composition comprising a synthetic nuclear transporter, wherein the synthetic nuclear transporter comprises: (i) a single-stranded RNA (ssRNA) viroid sequence and (ii) a heterologous RNA sequence comprising or encoding an effector, wherein the ssRNA viroid sequence does not include a chloroplast localization sequence.

In some non-claimed aspects, the ssRNA viroid sequence has at least 80% sequence identity with a pospiviroid sequence.

In some non-claimed aspects, the ssRNA viroid sequence has at least 80% sequence identity to a sequence selected from the group consisting of SEQ ID NOs:51-54, SEQ ID NOs:65-66, SEQ ID NO:68, SEQ ID NO:75, SEQ ID NOs:77-79, SEQ ID NOs:84-96, SEQ ID NOs:98-107, SEQ ID NOs:123-124, SEQ ID NOs:126-132, SEQ ID NO:134, SEQ ID NOs:136-143, SEQ ID NOs:145-150, SEQ ID NOs:153-154, SEQ ID NO:159, SEQ ID NO:166, SEQ ID NO:168, SEQ ID NO:196, SEQ ID NO:242, SEQ ID NO:268, SEQ ID NO:274, SEQ ID NO:276, SEQ ID NO:289, SEQ ID NO:451, SEQ ID NOs:458-459, and SEQ ID NO:467.

In some non-claimed aspects, the ssRNA viroid sequence has at least 90% sequence identity with SEQ ID NO:51.

In some aspects, the heterologous RNA sequence comprises coding RNA, non-coding RNA, or both coding and non-coding RNA.

In some aspects, the effector comprises coding RNA, non-coding RNA, or both coding and non-coding RNA.

### iii. Replication and transmission by inheritance

The recombinant polynucleotide (e.g., RNA) replicates within the plant.

In some embodiments, the cell is transiently transformed with the recombinant polynucleotide. In other embodiments, the cell is stably transformed with the recombinant polynucleotide, e.g., the recombinant polynucleotide or a portion thereof (e.g., a portion comprising the effector) is integrated into the nuclear genome, chloroplast genome, or mitochondrial genome.

In some embodiments, the recombinant polynucleotide is inherited by a progeny of the plant (e.g., a seed of the plant, a seed fertilized by pollen of the plant, or an asexually propagated clone of the plant (e.g., a plantlet, cutting, runner, bulb, tuber, corm, sucker, or tissue culture of the plant)). In other embodiments, the recombinant polynucleotide is not inherited by a progeny of the plant, e.g., is not transmitted in pollen and/or seeds.

### iii. Plants, plant parts, and plant cells

Plants, plant parts, and plant cells are of any species of interest, including flowering plants (e.g., dicots and monocots); gymnosperms; seedless vascular plants (e.g., ferns); bryophytes (e.g., mosses); algae; and cyanobacteria. Plants of interest include row crop plants, fruit-producing plants and trees, vegetables, trees, and ornamental plants including ornamental flowers, shrubs, trees, groundcovers, and turf grasses. Examples of commercially important cultivated crops, trees, and plants include: alfalfa (*Medicago sativa*), almonds (*Prunus dulcis*), apples (*Malus x domestica*), apricots (*Prunus armeniaca*, *P. brigantine*, *P. mandshurica, P. mume, P. sibirica*), asparagus (*Asparagus officinalis*), bananas (*Musa* spp.), barley (*Hordeum vulgare*), beans (*Phaseolus* spp.), blueberries and cranberries (*Vaccinium* spp.), cacao (*Theobroma cacao*), canola and rapeseed or oilseed rape, (*Brassica napus*), Polish canola (*Brassica rapa*), and related cruciferous vegetables including broccoli, kale, cabbage, and turnips (*Brassica carinata, B. juncea, B. oleracea, B. napus, B. nigra,* and *B. rapa,* and hybrids of these), carnation (*Dianthus caryophyllus*), carrots (*Daucus carota sativus*), cassava (*Manihot esculentum*), cherry (*Prunus avium*), chickpea (*Cicer arietinum*), chicory (*Cichorium intybus*), chili peppers and other capsicum peppers (*Capsicum annuum, C. frutescens, C. chinense, C*. *pubescens*, *C. baccatum*), chrysanthemums (*Chrysanthemum* spp.), coconut (*Cocos nucifera*), coffee (*Coffea* spp. including *Coffea arabica* and *Coffea canephora*), cotton (*Gossypium hirsutum* L.), cowpea (*Vigna unguiculata* and other *Vigna* spp.), fava bean (*Vicia faba),* cucumber (*Cucumis sativus*), currants and gooseberries (Ribes spp.), date (*Phoenix dactylifera*), duckweeds (family Lemnoideae), eggplant or aubergine (*Solanum melongena*), eucalyptus (*Eucalyptus* spp.), flax (*Linum usitatissumum* L.), geraniums (*Pelargonium* spp.), grapefruit (*Citrus x paradisi*), grapes (*Vitus* spp.) including wine grapes (*Vitus vinifera* and hybrids thereof), guava (*Psidium guajava*), hops (*Humulus lupulus*), hemp and cannabis (*Cannabis sativa* and *Cannabis* spp.), irises (*Iris* spp.), lemon (*Citrus limon*), lettuce (*Lactuca sativa*), limes (*Citrus* spp.), maize (*Zea mays* L.), mango (*Mangifera indica*), mangosteen (*Garcinia mangostana*), melon (*Cucumis melo*), millets (*Setaria* spp., *Echinochloa* spp., *Eleusine* spp., *Panicum* spp., *Pennisetum* spp.), oats (*Avena sativa*), oil palm (*Ellis quineensis*), olive (*Olea europaea*), onion (*Allium cepa*) and other alliums (*Allium* spp.), orange (*Citrus sinensis*), papaya (*Carica papaya*), peaches and nectarines (*Prunus persica*), pear (*Pyrus* spp.), pea (*Pisa sativum*), peanut (*Arachis hypogaea*), peonies (*Paeonia* spp.), petunias (*Petunia* spp.), pineapple (*Ananas comosus*)*,* plantains (*Musa* spp.), plum (*Prunus domestica*), poinsettia (*Euphorbia pulcherrima*), poplar (*Populus* spp.), potato (*Solanum tuberosum*), pumpkins and squashes (*Cucurbita pepo, C. maximus, C. moschata*), rice (*Oryza sativa* L.), roses (*Rosa* spp.), rubber (*Hevea brasiliensis*), rye (*Secale cereale*), safflower (*Carthamus tinctorius* L), sesame seed (*Sesame indium*), sorghum (*Sorghum bicolor*), soybean (*Glycine max* L.), strawberries (*Fragaria* spp., *Fragaria x ananassa*), sugar beet (*Beta vulgaris*), sugarcanes (*Saccharum* spp.), sunflower (*Helianthus annuus*), sweet potato (*Ipomoea batatas*), tangerine (*Citrus tangerina*), tea (*Camellia sinensis*), tobacco (*Nicotiana tabacum* L.), tomato (*Solanum lycopersicum* or *Lycopersicon esculentum*), tulips (*Tulipa* spp.), walnuts (*Juglans* spp. L.), watermelon (*Citrulus lanatus*), wheat (*Triticum aestivum*), and yams (*Discorea* spp.).

The plant cells and derivative plants and seeds disclosed herein can be used for various purposes useful to the consumer or grower. In embodiments, the intact plant itself is desirable, e.g., plants grown as cover crops or as ornamentals. In other embodiments, processed products are made from the plant or its seeds, such as extracted proteins, oils, sugars, and starches, fermentation products, animal feed or human food, wood and wood products, pharmaceuticals, and various industrial products. Thus, further related aspects of the disclosure include a processed or commodity product made from a plant or seed or plant part that includes at least some cells that contain the recombinant polynucleotide or the effector. Commodity products include, but are not limited to, harvested leaves, roots, shoots, tubers, stems, fruits, seeds, or other parts of a plant, meals, oils (edible or inedible), fiber, extracts, fermentation or digestion products, crushed or whole grains or seeds of a plant, wood and wood pulp, or any food or non-food product.

In some aspects, the plant is a weed. As used herein, the term "weed" refers to a plant that grows where it is not wanted. Such plants are typically invasive and, at times, harmful, or have the risk of becoming so. In embodiments, weeds are treated with the present pest control (e.g., biopesticide or biorepellent) compositions to reduce or eliminate the presence, viability, or reproduction of the plant. For example, and without being limited thereto, the methods can be used to target weeds known to damage plants. For example, and without being limited thereto, the weeds can be any member of the following group of families: Gramineae, Umbelliferae, Papilionaceae, Cruciferae, Malvaceae, Eufhorbiaceae, Compositae, Chenopodiaceae, Fumariaceae, Charyophyllaceae, Primulaceae, Geraniaceae, Polygonaceae, Juncaceae, Cyperaceae, Aizoaceae, Asteraceae, Convolvulaceae, Cucurbitaceae, Euphorbiaceae, Polygonaceae, Portulaceae, Solanaceae, Rosaceae, Simaroubaceae, Lardizabalaceae, Liliaceae, Amaranthaceae, Vitaceae, Fabaceae, Primulaceae, Apocynaceae, Araliaceae, Caryophyllaceae, Asclepiadaceae, Celastraceae, Papaveraceae, Onagraceae, Ranunculaceae, Lamiaceae, Commelinaceae, Scrophulariaceae, Dipsacaceae, Boraginaceae, Equisetaceae, Geraniaceae, Rubiaceae, Cannabaceae, Hyperiacaceae, Balsaminaceae, Lobeliaceae, Caprifoliaceae, Nyctaginaceae, Oxalidaceae, Vitaceae, Urticaceae, Polypodiaceae, Anacardiaceae, Smilacaceae, Araceae, Campanulaceae, Typhaceae, Valerianaceae, Verbenaceae, Violaceae. For example, and without being limited thereto, the weeds can be any member of the group consisting of *Lolium rigidum, Amaramthus palmeri, Abutilon theopratsi, Sorghum halepense, Conyza canadensis, Setaria verticillata, Capsella pastoris,* and *Cyperus rotundas.* Additional weeds include, for example, *Mimosa pigra, Salvinia* spp., *Hyptis* spp., *Senna* spp., noogoora burr (*Xanthium spinosum*) and other burr weeds, *Jatropha gossypifolia, Parkinsonia aculeate, Chromolaena odorata, Cryptoslegia grandiflora,* and *Andropogon gayanus.* Weeds can include monocotyledonous plants (e.g., Agrostis, Alopecurus, Avena, Bromus, Cyperus, Digitaria, Echinochloa, Lolium, Monochoria, Rottboellia, Sagittaria, Scirpus, Setaria, Sida, or Sorghum) or dicotyledonous plants (Abutilon, Amaranthus, Chenopodium, Chrysanthemum, Conyza, Galium, Ipomoea, Nasturtium, Sinapis, Solanum, Stellaria, Veronica, Viola, or Xanthium).

In some embodiments, a composition comprising a recombinant polynucleotide comprising (i) a single-stranded RNA (ssRNA) plant viroid sequence that is a plant viroid genome or a derivative thereof, wherein the plant viroid is from the family Avsunviroidae and (ii) a heterologous RNA sequence that is not a viroid sequence and that comprises or encodes an effector, wherein (b) is inserted into (a), wherein the recombinant polynucleotide replicates in the plant is delivered to a plant that the viroid is known to infect, e.g., a plant in which the viroid has been observed. In other embodiments, a composition comprising a recombinant polynucleotide comprising (i) a single-stranded RNA (ssRNA) plant viroid sequence that is a plant viroid genome or a derivative thereof, wherein the plant viroid is from the family Avsunviroidae and (ii) a heterologous RNA sequence that is not a viroid sequence and that comprises or encodes an effector, wherein (b) is inserted into (a), wherein the recombinant polynucleotide replicates in the plant is delivered to a plant that the viroid has not been observed to infect. Descriptions of viroids infecting a range of host species (including horticultural and crop species) are provided, e.g., in Bagherian et al., Journal of Plant Physiology, 201: 42-53, 2016; Singh et al., VirusDis., 25(4): 415-424, 2014; and Constable et al., Viruses, 11(98): 2019.

In some embodiments, a composition comprising a recombinant polynucleotide comprising (i) a single-stranded RNA (ssRNA) plant viroid sequence of a viroid from the family Avsunviroidae and (ii) a heterologous RNA sequence that is not a plant viroid sequence and that comprises or encodes an effector, wherein (ii) is inserted into (i); and wherein the recombinant polynucleotide replicates in the plant is delivered to any plant, plant part, or plant cell type. A plant can be, e.g., an entire plant, e.g., an entire adult plant, juvenile plant, seedling, or embryo of any of the plant species described herein. Plant parts include, but are not limited to leaves (e.g., leaf blade, leaflet, phyllode, or petiole), seeds (including embryo, endosperm, or seed coat), roots (e.g., primary roots, secondary roots, radicles, root hairs, or root nodules), shoot vegetative organs/structures (e.g., leaves, stems, hypocotyls, rhizomes, or tubers), flowers and floral organs/structures (e.g., pollen, bracts, sepals, petals, stamens, carpels, anthers, or ovules), fruits (including mature ovaries and associated tissues, e.g., receptacle, hypanthium, or perianth), vegetables, pollen, seeds, spores, sap (e.g., phloem or xylem sap), or plant tissues (e.g., vascular tissue, ground tissue, parenchyma, sclerenchyma, collenchyma, or tumor tissue). Plant cell types include any cells of plants and plant parts described herein (e.g., epidermal cells, mesophyll cells, vasculature cells, parenchymal cells, meristematic cells, and root cells) and protoplasts thereof (e.g., leaf protoplasts or root protoplasts. In some aspects, the plant is a single-celled plant, e.g., a single-celled plastid-containing organism such as algae. In embodiments, the composition is delivered to only part of a plant, such as to meristematic tissue of a plant, or to rootstock onto which an untreated scion is grafted, or to a scion that is grafted onto untreated rootstock.

In some non-claimed embodiments, a composition comprising a recombinant polynucleotide comprising (i) a single-stranded RNA (ssRNA) viroid sequence and (ii) a heterologous RNA sequence comprising or encoding an effector is delivered to an unorganized cell culture, in which a plurality of the cultured cells are not organized into a tissue or organ of a multicellular plant, such as a leaf, root, shoot, or reproductive structure of a multicellular plant. Exemplary unorganized cell cultures include callus culture, cell suspension culture, and protoplast culture.

In some embodiments, the disclosure features a cell comprising a composition described herein. In some embodiments, the cell is a plant cell, e.g., a monocot cell or a dicot cell. In some embodiments, the plant cell is a protoplast. In some embodiments, the cell has been transiently transformed with the recombinant polynucleotide. In other aspects, the cell has been stably transformed with the recombinant polynucleotide.

### B. Methods of modifying plants

In some aspects, the disclosure provides a method of modulating a trait, phenotype, or genotype in a plant, the method comprising providing to the plant a composition described herein (e.g., a composition comprising or consisting of a recombinant polynucleotide (e.g., a vector) comprising (i) a single-stranded RNA (ssRNA) plant viroid sequence of a viroid from the family Avsunviroidae and (ii) a heterologous RNA sequence comprising or encoding an effector that is not a plant viroid sequence and that comprises or encodes an effector, wherein (ii) is inserted into (i); and wherein the recombinant polynucleotide replicates in the plant). The effector can be any moiety that can be integrated into a recombinant polynucleotide comprising an ssRNA plant viroid sequence of a viroid from the family Avsunviroidae (e.g., a viroid-derived vector) and that has a biological effect on (e.g., is capable of modulating a state of) a plant or a plant cell.

In some embodiments, modulating comprises expressing in the plant a protein or polypeptide, wherein the heterologous protein or polypeptide is encoded by the heterologous RNA sequence of the recombinant polynucleotide. The protein or polypeptide can be, e.g., a native protein or polypeptide of the plant to which the composition is delivered; a protein or polypeptide of another organism; or an artificial protein or polypeptide.

In some embodiments, modulating comprises reducing expression of a target gene of the plant. In embodiments, expression of the target gene is reduced by about 1%, 2%, 3%, %, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% relative to a reference level (e.g., a level found in a plant that does not receive a recombinant polynucleotide of the disclosure).

In some embodiments, modulating comprises increasing expression of a target gene of the plant. In embodiments, expression of the target gene is increased by about 1%, 2%, 3%, %, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or more than 100% relative to a reference level (e.g., a level found in a plant that does not receive a recombinant polynucleotide of the disclosure).

In some embodiments, modulating comprises regulating a target gene in the plant. In embodiments, the regulation is, e.g., regulation of transcription; regulation of RNA processing; regulation of translation; regulation of post-transcriptional modification; regulation of expression; regulation of post-translational modification; or regulation of degradation. In some embodiments, a status of the target gene (e.g., transcription, RNA processing, post-transcriptional modification, translation, post-translational modification, expression, or degradation) is decreased by about 1%, 2%, 3%, %, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% relative to a reference level (e.g., a level found in a plant, plant part, or plant cell that does not receive a recombinant polynucleotide of the disclosure). In other aspects, a status of the target gene is increased by about 1%, 2%, 3%, %, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or more than 100% relative to a reference level (e.g., a level found in a plant that does not receive a recombinant polynucleotide of the disclosure).

In some embodiments, modulating (e.g., modifying) comprises editing a target gene of the plant, e.g., editing gene encoded by the nuclear genome, plastid genome, or mitochondrial genome of the plant. In embodiments, the edited gene is inherited by a progeny of the plant (e.g., a seed of the plant, a seed fertilized by pollen of the plant, or an asexually propagated clone of the plant (e.g., a plantlet, cutting, runner, bulb, tuber, corm, sucker, or tissue culture of the plant)).

In some embodiments, the effector increases the fitness of the plant, e.g., increases the fitness of the plant by about 1%, 2%, 3%, %, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or more than 100% relative to a reference level (e.g., a level found in a plant, plant part, or plant cell that does not receive a recombinant polynucleotide of the disclosure).

In some embodiments, the effector decreases the fitness of the plant, e.g., increases the fitness of the plant by about 1%, 2%, 3%, %, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or more than 100% relative to a reference level (e.g., a level found in a plant, plant part, or plant cell that does not receive a recombinant polynucleotide of the disclosure).

Traits, phenotypes, and genotypes that can be modulated by a composition of the disclosure include, but are not limited to traits, phenotypes, and genotypes that increase or decrease plant fitness. In some embodiments of the compositions described herein, the increase in fitness is an increase in developmental rate, growth rate, size, yield (e.g., intrinsic yield), resistance to abiotic stressors, or resistance to biotic stressors relative to a reference level (e.g., a level found in a plant, plant part, or plant cell that does not receive a recombinant polynucleotide of the disclosure). In some embodiments, the increase in plant fitness is an increase in disease resistance, drought tolerance, heat tolerance, cold tolerance, salt tolerance, metal tolerance, herbicide tolerance, herbicide resistance, chemical tolerance, environmental stress resistance, water use efficiency, nitrogen utilization, resistance or tolerance to nitrogen stress (e.g., low or high nitrogen supply), nitrogen fixation, pest resistance, herbivore resistance, pathogen resistance, disease resistance, fungal disease resistance, virus resistance, nematode resistance, bacterial disease resistance, insect control, yield under water-limited conditions, vigor, photosynthetic capability, nutrition (e.g., human or animal nutrition), flavor, starch production, protein content, carbohydrate content, oil content, fatty acid content, lipid content, digestibility, biomass, shoot length, root length, root architecture, seed set, seed weight, seed quality (e.g., nutritional content), germination, fruit set, rate of fruit ripening, production of biopolymers, production of fibers, production of biofuels, production of pharmaceutical peptides, production of secretable peptides, enzyme production, improved processing traits, or amount of harvestable produce relative to a reference level (e.g., a level found in a plant, plant part, or plant cell that does not receive a recombinant polynucleotide of the disclosure). In some embodiments, the increase in fitness is earlier flowering. In some embodiments, the increase in plant fitness is an increase in the quality of products harvested from the plant. In some embodiments, the increase in plant fitness is an improvement in taste, appearance, or shelf-life of a product harvested from the plant relative to a reference level (e.g., a level found in a plant, plant part, or plant cell that does not receive a recombinant polynucleotide of the disclosure). In some embodiments, the increase in fitness is a decrease in production of an allergen that stimulates an immune response in an animal. In some embodiments, the trait, phenotype, or genotype that is modulated by a composition of the disclosure is of horticultural interest, e.g., relates to flower size, flower color, flower patterning, flower morphology, flower number, flower longevity, flower fragrance, leaf size, leaf color, leaf patterning, leaf morphology, plant height, or plan architecture.

Genotypes that can be modulated by a composition of the disclosure include genes of agronomic interest. As used herein, the term "gene of agronomic interest" refers to a transcribable polynucleotide molecule that, when expressed in a particular plant tissue, cell, or cell type, confers a desirable characteristic, e.g., a characteristic associated with plant morphology, physiology, growth, development, yield, product, nutritional profile, disease or pest resistance, and/or environmental or chemical tolerance. Genes of agronomic interest include, but are not limited to, those encoding a yield protein, a stress resistance protein, a developmental control protein, a tissue differentiation protein, a meristem protein, an environmentally responsive protein, a senescence protein, a hormone responsive protein, an abscission protein, a source protein, a sink protein, a flower control protein, a seed protein, an herbicide resistance protein, a disease resistance protein, a fatty acid biosynthetic enzyme, a tocopherol biosynthetic enzyme, an amino acid biosynthetic enzyme, a pesticidal protein, or any other agent such as an antisense or RNAi molecule targeting a particular gene for suppression. In embodiments, the product of a gene of agronomic interest acts within the plant in order to cause an effect upon the plant physiology or metabolism or acts as a pesticidal agent in the diet of a pest that feeds on the plant. Exemplary genes of interest include those described in U.S. Patent No. 10550401.

### EXAMPLES

The following are examples of the methods of the disclosure. It is understood that various other embodiments can be practiced, given the general description provided above.

**Table of Contents**

| Example | **Title** |
|---|---|
| Example 1 | *In vitro* transcription of linear RNAs |
| Example 2 | Production of circular RNA using splint ligation |
| Example 3 | Production of circular RNA using bacteria transfected with a tRNA ligase |
| Example 4 | Production of circular RNA using ligation of ribozyme-cleaved ends |
| Example 5 | RNA circularization efficiency |
| Example 6 | Circularized RNA is circular and not concatemeric |
| Example 7 | Reduced degradation susceptibility in circular RNAs |
| Example 8 | Isolation and purification of circular RNAs |
| Example 9 | Delivery of circular RNA to plant protoplasts |
| Example 10 | Delivery of circular RNA via leaf rubbing |
| Example 11 | Delivery of circular RNA via leaf infiltration |
| Example 12 | ELVd with a hairpin RNA targeting a tomato gene |
| Example 13 | ELVd with a guide RNA targeting a corn gene |
| Example 14 | PSTVd with a small RNA targeting a tomato gene |
| Example 15 | PSTVd TL-R or PSTVd R with a pre-miRNA targeting a tomato gene |
| Example 16 | Arabidopsis circRNA with a PSTVd replication motif |
| Example 17 | *In planta* viral trafficking motifs conjugated to a fluorescent aptamer as a circular fusion RNA |
| Example 18 | PSTVd with a Spinach RNA aptamer in *Arabidopsis* and corn |
| Example 19 | An RNA motif to traffic aptamers to a subcellular location |
| Example 20 | An RNA motif to traffic pre-miRNA to a specific cell type |
| Example 21 | An RNA motif to traffic an aptamer to a specific tissue type |
| Example 22 | Circular RNAs with PSTVd LT and EMCV or maize HSP101 IRES in monocots vs. dicots |
| Example 23 | PSTVd trafficking loop, replication loop, and Spinach RNA aptamer |
| Example 24 | An RNA vector containing two RNA motifs from two different viroid sources to deliver an aptamer to a specific cell type |
| Example 25 | *In planta* circularization of viral trafficking motifs conjugated to a fluorescent aptamer through transfection of a linear fusion RNA |
| Example 26 | PSTVd R+dsRNA to target RPL7 in CBP |
| Example 27 | A nuclear transporter based on PSTVd delivers RNA cargo to nucleus in *Nicotiana tabacum* protoplasts |

### Example 1. In vitro transcription of linear RNAs

This Example describes *in vitro* transcription of RNAs using the T7 RNA polymerase promoter. In embodiments, this method is used to produce linear RNAs, such as the linear RNAs described in this specification.

Linear RNAs useful as circular RNA precursors were synthesized as follows: linear, 5'-mono phosphorylated in vitro transcripts were generated using the HiScribeTM T7 Quick High Yield RNA Synthesis Kit (New England BioLabs^{®} Inc., REF: E2050S). In vitro transcription was performed according to the manufacturer's protocol. Around 40µg of linear RNA was generated in each reaction. After incubation, each reaction was treated with DNase to remove the DNA template. Linear RNA ribonucleotides were then column-purified using the Zymo RNA Clean & Concentrator-5 kit (Zymo Research: R1014). Linear transcribed ribonucleotides were quality tested by heating in vitro transcription products to 80°C for 7-10 minutes. Heated ribonucleotides were then run on an agarose gel to validate purity of transcribed RNA and RNA quality. The expected bands of the appropriate molecular weight were observed by gel electrophoresis.

A modified procedure was also carried out as follows: Linear RNAs useful as circular RNA precursors were synthesized as follows: linear, 5'-mono phosphorylated *in vitro* transcripts were generated using the Lucigen AmpliScribe T7-Flash kit (ASF3507). *In vitro* transcription was performed according to the manufacturer's protocol. Around 100µg of linear RNA was generated in each reaction. After incubation, each reaction was treated with DNase to remove the DNA template. Linear RNA ribonucleotides were then column-purified using the Monarch 500 RNA Clean-up Kit (T2050L). Linear transcribed ribonucleotides were quality tested by denaturing and/or native gel electrophoresis. Denaturing gel electrophoresis was performed by dissolving *in vitro* transcription products in gel loading buffer with a final concentration of greater than or equal to 50% formamide; heating at 95 °C for 3 minutes and cooling rapidly to 4 °C, followed by running on urea PAGE gel with TBE running buffer. Native gel electrophoresis was performed by heating *in vitro* transcription products in water at 95 °C for 3 minutes; cooling slowly to room temperature for at least 15 minutes; and running on agarose gel with a voltage of less than 100V until bands were resolved. The expected bands of the appropriate molecular weight were observed by gel electrophoresis.

### Example 2. Production of circular RNA using splint ligation

Non-naturally occurring circular RNAs can be engineered to include one or more desirable properties, and can be produced using recombinant DNA technology. This Example describes *in vitro* production of circular RNA from linear RNA using splint ligation. In embodiments, this method is used to circularize linear RNAs described herein or other linear RNAs useful in the methods and compositions described herein.

In this Example, a general protocol for generating the circular RNA is as follows: DNA templates for in vitro transcription are amplified from a plasmid comprising a sequence of interest. Amplified DNA templates are gel-purified with a DNA gel purification kit (Qiagen). 250-500ng of purified DNA template is subjected to in vitro transcription.

Linear, 5'-mono phosphorylated in vitro transcripts are generated using T7 RNA polymerase from each DNA template having corresponding sequences in the presence of 7.5mM guanosine monophosphate (GMP), 1.5mM guanosine triphosphate (GTP), 7.5mM uracil triphosphate (UTP), 7.5mM cytosine triphosphate (CTP), and 7.5mM adenosine triphosphate (ATP). Around 40µg of linear RNA is generated in each reaction. After incubation, each reaction is treated with DNase to remove the DNA template. The in vitro transcribed RNA is precipitated with ethanol in the presence of 2.5M ammonium acetate to remove unincorporated monomers.

Transcribed linear RNA is circularized using T4 RNA ligase 2 on a 20nt splint DNA oligomer as template. Splint DNA is designed to anneal between 5-25 nucleotides (nt) of each 5' or 3' end of the linear RNA, leaving 2 nt at each end of the RNA unpaired. After annealing with the splint DNA (3µM), 1µM linear RNA is incubated with 0.5U/µl T4 RNA ligase 2 at 37°C for 4 hours. A mixture without T4 RNA ligase 2 is used as a negative control.

The circularization of linear RNA is monitored by separating RNA on 6% denaturing PAGE. Because of their circular structure, circular RNAs migrate more slowly than linear RNAs on denaturing polyacrylamide gels. The addition of ligase to the RNA mixtures generates new bands that appear above the linear RNA bands that are present in the mixtures that lack ligase ((-) lanes). Slower-migrating bands appear in all RNA mixtures containing ligase, indicating that successful splint ligation (e.g., circularization) occurred for multiple constructs, but not for the negative control.

In one experiment, circular RNA was generated as follows: DNA templates for *in vitro* transcription were amplified from a plasmid with corresponding sequences with a T7 promoter-harboring forward primer and a 2-O-methylated nucleotide with a reverse primer. Amplified DNA templates were column-purified using DNA Clean and Concentrator 5 kit (Zymo Research). Linear, 5'-mono phosphorylated *in vitro* transcripts were generated from purified DNA template (250-500 ng) using T7 RNA polymerase in the presence of 7.5 mM guanosine monophosphate (GMP), 1.5 mM guanosine triphosphate (GTP), 7.5 mM uracil triphosphate (UTP), 7.5 mM cytosine triphosphate (CTP), and 7.5 mM adenosine triphosphate (ATP). Around 40 µg of linear RNA was generated in each reaction. After incubation, each reaction was treated with DNase to remove the DNA template. The *in vitro* transcribed RNA was precipitated with ethanol in the presence of 2.5 M ammonium acetate to remove unincorporated monomers.

Transcribed linear RNA was circularized using T4 RNA ligase 2 on a 20-nucleotide (nt) splint DNA oligomer as template. Splint DNA was designed to anneal to 10 nt of the 5' end of the linear RNA and to 10 nt of the 3' end of the linear RNA, leaving 2 nt at each end of the linear RNA unpaired. After annealing with the splint DNA (3 µM), 1 µM linear RNA was incubated with 0.5U/µl T4 RNA ligase 2 at 37 °C for 4 hours. A mixture without T4 RNA ligase 2 was used as a negative control. The circularization of linear RNA was monitored by separating RNA on 6% denaturing PAGE. The addition of ligase to the RNA mixtures generated new bands that appeared at higher apparent molecular weight (~10 kb) than the linear RNA bands (~1 kb) that were observed in the negative control lanes (mixtures without ligase). Slower-migrating bands (~10kb apparent molecular weight) appeared in all RNA mixtures containing ligase, indicating that successful splint ligation (i.e., circularization) occurred in all the the reactions including the ligase, but not in the negative control reactions lacking the ligase.

In another experiment, the circular RNA was generated as follows: DNA templates for *in vitro* transcription were amplified from a plasmid with corresponding sequences with a T7 promoter-harboring forward primer and reverse primer with 5' terminal nucleotide corresponding to the 3' terminal nucleotide of the desired RNA. Amplified DNA templates were column purified using DNA Clean and Concentrator 5 kit (Zymo Research). 250-500 ng of purified DNA template was subjected to in vitro transcription. Linear, 5'-tri-phosphorylated in vitro transcripts were generated using T7 RNA polymerase from each DNA template having corresponding sequences. Around 100ug of linear RNA was generated in each reaction.

After incubation, each reaction was treated with DNase to remove the DNA template. Linear RNA ribonucleotides were then column-purified using the Monarch 500 RNA Clean-up Kit (T2050L). Linear 5'-triphosphorylated RNAs were converted to linear 5'-mono-phosphorylated RNAs using pyrophosphohydrolase enzyme RppH (New England Biolabs, M0356S) according to the manufacturer's instructions. Linear 5' mono-phosphorylated RNAs were column purified using the Monarch 500 RNA Clean-up Kit (T2050L).

Transcribed linear RNA was circularized using T4 RNA ligase 2 (New England Biolabs) using a 30 nt splint DNA oligomer as template. The splint DNA was designed to anneal to 15 nt of the 5' end of the linear RNA and to 15 nt of the 3' end of the linear RNA, leaving no unpaired nt at either end of the linear RNA. After annealing with the splint DNA (3µM), 1µM linear RNA was incubated with 0.5U/µl T4 RNA ligase 2 at 37 °C for 4 hours. The circularization of linear RNA was monitored by separating RNA on 6% denaturing PAGE. The addition of ligase to the RNA mixtures generated new bands that appeared at higher apparent molecular weight (~10 kb) than the linear RNA bands (~1 kb) that were observed in the negative control lanes (mixtures without ligase). Slower-migrating bands (~10kb apparent molecular weight) appeared in all RNA mixtures containing ligase, indicating that successful splint ligation (i.e., circularization) occurred in all the the reactions including the ligase, but not in the negative control reactions lacking the ligase.

### Example 3. Production of circular RNA using bacteria transfected with a tRNA ligase

ELVd are plant pathogens consisting of a single-stranded circular RNA that replicates in host cells and is circularized by endogenous tRNA ligases. This Example describes production of circular RNA in a model bacterial system (*E*. *coli*) using co-expression of Eggplant Latent Viroid (ELVd) RNA as an exemplary

RNA and eggplant tRNA ligase as an exemplary tRNA ligase; any tRNA ligase can be used to circularize a viroid RNA. The fluorescent Spinach RNA aptamer (Huang et al., Nat Chem Biol, 10: 686-691, 2014) is used as a model effector. In some embodiments, this method is used to circularize other linear RNAs useful in the methods and compositions described herein.

As shown in the following Example, significant quantities of an aptamer-containing circular RNA were generated in *E. coli,* as described by Daros et al., Scientific Reports, 8: Article Number 1904, 2018. Briefly, plasmids were constructed using standard molecular cloning techniques, with a first plasmid containing an ELVd sequence with a Spinach RNA aptamer insertion and a second plasmid containing a sequence coding for eggplant tRNA ligase. The *E. coli* strains BL21 (DE3) (Novagen) and DH5-Alpha (New England BioLabs^{®} Inc.) were transformed or co-transformed with one or both plasmids, and recombinant clones were selected at 37°C on LB solid medium plates (10 g/L tryptone, 5 g/L yeast extract, 10 g/L NaCl and 1.5% agar) including the appropriate antibiotics (50 µg/mL ampicillin, 34 µg/mL chloramphenicol, or both). *E. coli* containing the plasmids of interest were grown in liquid cultures in Terrific Broth (TB) medium (12 g/L tryptone, 24 g/L yeast extract, 0.4% glycerol, 0.17 M KH₂PO₄ and 0.72 M K₂HPO₄), containing the appropriate antibiotics (see above), at 37 °C with shaking (225 revolutions per minute (rpm)). Cell densities were measured by absorbance at 600 nm with a spectrophotometer (Implen OD600 DiluPhotometer).

At the desired time points, 2 mL aliquots of the liquid cultures were taken and cells were sedimented by centrifuging at 13,000 rpm for 2 minutes. Cells were resuspended in 50 µL of TE buffer (10 mM Tris-HCl, pH 8.0 and 1 mM EDTA) by vortexing. One volume (50 µL) of a 1:1 (v/v) mix of phenol (saturated with water and equilibrated at pH 8.0 with Tris-HCl, pH 8.0) and chloroform were added, and the cells were broken by vigorous vortexing. The aqueous and organic phases were separated by centrifugation for 5 minutes at 13,000 rpm. The aqueous phases were recovered and re-extracted with one volume (50 µL) of chloroform. The aqueous phases containing total bacterial nucleic acids were finally recovered by pipetting.

Total RNA from *E. coli* was analyzed by denaturing PAGE. Twenty µL of RNA preparations were mixed with 1 volume (20 µL) of loading buffer (98% formamide, 10 mM Tris-HCl, pH 8.0, 1 mM EDTA, 0.0025% bromophenol blue and 0.0025% xylene cyanol), heated for 1.5 minutes at 95 °C, and snap cooled on ice. Electrophoresis was run for 2.5 hours at 200 V in 6% urea polyacrylamide gels, or 1 hour at 200 V in 10% urea polyacrylamide gels. Electrophoresis buffer was 1X TBE without urea. Gels were stained by shaking for 15 minutes in 200 mL of 1 µg/mL ethidium bromide. This allowed visualization of the separation of circular RNA from linear RNA. Slower-migrating bands appeared in all RNA mixtures, indicating successful circularization in the bacterial cells.

To assay the fluorescence of Spinach RNA aptamer, aliquots of the E. *coli* cultures are supplemented with 200 µM DFHBI and grown for one additional hour. Pelleted bacteria are photographed under a stereomicroscope (Leica MZ16 F, Leica Microsystems) with UV illumination and a GFP2 filter (Leica Microsystems). To assay the fluorescence of Spinach RNA aptamer in RNA extracts, the extracts were run on denaturing PAGE gels as described above. After running, gels were washed 3x in water for 5 minutes. Gels were then incubated in 10 mL aptamer buffer (10 mM MgCl₂, 50 mM Na-HEPES (pH 7.5), 100 mM KCI) containing 10 µM of the fluorophore (5Z)-5-[(3,5-Difluoro-4-hydroxyphenyl)methylene]-3,5-dihydro-2,3-dimethyl- 4H-Imidazol-4-one, (Z)-4-(3,5-Difluoro-4-hydroxybenzylidene)-1,2-dimethyl-1H-imidazol-5(4H)-one ("DFHBI", which fluoresces when bound to the Spinach aptamer, Sigma, SML1627-5MG) for 30 minutes with gentle agitation. Gels were briefly washed with water and then imaged using the Invitrogen iBright Imaging System with 470 nm excitation and 503 nm emission. Gels were then stained with ethidium bromide and imaged as described above. Composite images of DFHBI and ethidium bromide were generated. This gel electrophoresis and visualization revealed a band specifically stained by DFHBI, which migrated according to the known migration of circular ELVd polyribonucleotides. This band was only observed in RNA mixtures derived from cells containing heterologously expressed eggplant tRNA ligase, and confirmed the eggplant tRNA ligase-mediated circularization of the ELVd molecule containing the Spinach RNA aptamer.

### Example 4. Production of circular RNA using ligation of ribozyme-cleaved ends

This Example describes production of circular RNA using ligation of ribozyme-cleaved ends. The fluorescent Broccoli RNA aptamer is used as a model effector. In embodiments, this method is used to circularize other linear RNAs useful in the methods and compositions described herein.

As shown in the following Example, ribozyme-cleaved ends of linear RNAs can be joined to synthesize circular RNA, as described in Litke and Jaffrey, Nature Biotechnology, 37: 667-675, 2019. Recently described "Twister" ribozymes undergo self-cleavage to produce 5' hydroxyl and 2',3'-cylic phosphate ends. These ends are recognized for ligation by the *E. coli* RNA ligase RtcB. To trigger RNA circularization, RNA transcripts are expressed containing an RNA of interest flanked by ribozymes that undergo spontaneous autocatalytic cleavage. The resulting RNA contains 5' and 3' ends that are then ligated by the nearly ubiquitous endogenous RNA ligase RtcB, thereby producing circular RNAs.

DNA templates containing a Broccoli fluorogenic RNA aptamer sequence a 5' P3 Twister U2A ribozyme, and a 3' P1 Twister ribozyme are prepared, and RNA is synthesized as described in Example 1. RNA is gel-purified as described below in Example 8.

After gel purification of autocatalytically cleaved RNA, 300 pmol of the purified RNA are treated with T4 Polynucleotide Kinase (New England Biolabs^{®} Inc.) according to the manufacturer's protocol at 37°C for 30 minutes. The enzyme is then inactivated for 20 minutes at 65°C. The products are cleaned by phenol chloroform extraction using heavy phase-lock tubes (Quantabio 2302830). 10 pmol of the gel-purified RNA is ligated using RtcB Ligase (New England Biolabs^{®} Inc. M0458) for 1 hour at 37°C.

Total RNA (1.0-2.5 µg) is separated using precast 6% or 10% TBE-Urea Gels (Life Technologies EC68655), and run at 270 V in TBE buffer until completion. Gels are washed 3× 5 minutes with water and then stained for 30 minutes in 10 µM DFHBI in buffer prepared at room temperature containing 40 mM HEPES pH 7.4, 100 mM KCI, 1 mM MgCl₂. Broccoli RNA aptamer bands are then imaged using a ChemiDoc MP (Bio-Rad) with 470/30 nm excitation and 532/28 nm emission. Gels are washed additionally with water and stained with SYBR^{™} Gold (ThermoFisher Scientific, S11494) diluted in TBE buffer. RNA bands are then imaged using a ChemiDoc MP (Bio-Rad) with a preset channel (302 nm excitation and 590/110 nm emission). Gel band intensities are quantified in Image Lab 5.0 software (Bio-Rad).

In one experiment, DNA templates containing a Mangolll or Spinach fluorogenic RNA aptamer sequence, a 5' P3 Twister U2A ribozyme, and a 3' P1 Twister ribozyme were prepared, and RNA was synthesized as described in Example 1. RNA was gel-purified as described in Example 8.

After gel purification of autocatalytically cleaved RNA, 200 pmol of the gel-purified RNA was ligated using RtcB Ligase (New England Biolabs^{®} Inc. M0458) for 1 hour at 37°C. RNA mixtures were optionally subjected to exonuclease treatment as described in Example 7 to preferentially degrade linear RNAs. Total RNA (1.0-2.5 µg) were separated using precast 6% or 10% TBE-Urea Gels (Life Technologies EC68655) and run at 270 V in TBE buffer until completion. Gels were washed thrice for 5 minutes each time with water and then stained for 30 minutes in 10 µM TO1-Biotin (for Mango III) or 10 µM DFHBI (for Spinach) in buffer prepared at room temperature containing 40 mM HEPES pH 7.4, 100 mM KCI, 1 mM MgCl₂. Mango III or Spinach RNA aptamer bands were then imaged using a ChemiDoc MP (Bio-Rad) with 470/30 nm excitation and 532/28 nm emission. Gels were washed additionally with water and stained with SYBR^{™} Gold (ThermoFisher Scientific, S11494) diluted in TBE buffer. RNA bands were then imaged using a ChemiDoc MP (Bio-Rad) with a preset channel (302 nm excitation and 590/110 nm emission). Gel band intensities were quantified in Image Lab 5.0 software (Bio-Rad).

Gel electrophoresis and imaging revealed bands that stained brightly with TO1-Biotin and DFHBI, corresponding to Mango III RNA aptamer-containing RNAs and Spinach RNA aptamer-containing RNAs, respectively. RNA mixtures treated with RtcB ligase had differential migration compared with the no-ligase negative control, confirming the circular nature of the RNA molecules formed by the ligation reaction. When these ligase-treated RNA mixtures were subjected to exonuclease treatment, the resulting RNA mixtures still contained the putative circular bands upon electrophoresis, while the linear bands from the non-ligase-treated mixture were degraded upon exonuclease treatment and not observed upon electrophoresis. These observations confirmed the ligase-mediated circularization of aptamer-containing RNAs.

In a separate experiment, DNA templates containing a tobacco PDS gene sequence, a 5' P3 Twister U2A ribozyme, and a 3' P1 Twister ribozyme were prepared, and RNA was synthesized as described in Example 1. RNA was gel-purified as described in Example 8.

After gel purification of autocatalytically cleaved RNA, 200 pmol of the gel-purified RNA was ligated using RtcB Ligase (New England Biolabs^{®} Inc. M0458) for 1 hour at 37 °C. RNA mixtures were optionally subjected to exonuclease treatment as described in Example 7 to preferentially degrade linear RNAs. Total RNA (1.0-2.5 µg) were separated using precast 6% or 10% TBE-Urea Gels (Life Technologies EC68655) and run at 250 V in TBE buffer until completion. Gels were washed thrice for 5 minutes each time with water and then stained with ethidium bromide. RNAs were then imaged using iBright imager. Gel electrophoresis and imaging revealed different populations in each RNA mixture. RNA mixtures treated with RtcB ligase had differential migration compared with the no-ligase control, confirming the circular nature of the resulting molecules. When these ligase-treated RNA mixtures were subjected to exonuclease treatment, the resulting RNA mixtures still contained the putative circular bands upon electrophoresis, while the linear bands from the non-ligase-treated mixture were degraded upon exonuclease treatment and not observed upon electrophoresis. This confirmed the ligase-mediated circularization of RNAs with ribozyme-cleaved ends.

### Example 5. RNA circularization efficiency

This Example describes measurement of circularization efficiencies for the methods described in Examples 2-4. In some embodiments, this method is used to assess circularization of any RNA.

To measure circularization efficiency, linear RNA transcripts are generated as described in Example 1 and circularized using the methods described in Examples 2-4. The circular RNAs are resolved by 6% denaturing PAGE, and RNA bands on the gel corresponding to linear or circular RNA are excised for purification. Excised RNA gel bands are crushed and RNA is eluted with 800µl of 300mM NaCl overnight. Gel debris is removed by centrifuge filters, and RNA is precipitated with ethanol in the presence of 0.3M sodium acetate.

Alternatively, images of gels can be recorded and band intensities analyzed using ImageJ. Intensities of each band can be normalized to a standard curve of RNA with known concentration, such as a dilution series or molecular weight ladder. This can serve as a proxy for RNA amount for any given band. Circularization efficiency is calculated as follows: the amount of eluted circular RNA is divided by the total eluted RNA amount (circular + linear RNA).

In one experiment, a modified version of the method described above for the measurement of circularization efficiencies was employed. In embodiments, this method may be used to assess circularization of an RNA.

To measure circularization efficiency, linear RNA transcripts were generated as described in Example 1 and circularized using the methods described in Examples 2 - 4. Four circular RNAs (of 683, 709, 790, or 1026 nt, respectively) were resolved by 6% denaturing PAGE and stained for 5 minutes with 1µg/mL ethidium bromide. Images of gels were recorded and band intensities analyzed using ImageJ. Intensities of each band were recorded. Linear RNAs migrated at the expected molecular weight (~600 nt to ~1 kb), while circular molecules of the corresponding RNA migrated at higher apparent molecular weight (~8 kb to ~10 kb). Circularization efficiency was computed by dividing the intensity of the circular RNA band by the sum of the intensities of all bands in a given lane. Circularization efficiencies for the four RNAs were 78% (683 nt), 62% (709 nt), 75% (790 nt), and 65% (1026 nt), respectively.

### Example 6. Circularized RNA is circular and not concatemeric

This Example describes degradation of putative circular RNAs by RNAse H, which produces nucleic acid degradation products consistent with a circular and not a concatemeric RNA, thereby confirming that the RNAs are circular. In embodiments, this method is used to assess circularization of any RNA.

When incubated with a ligase, RNA can (i) not react, (ii) form an intramolecular bond, generating a circular (no free ends) RNA, or (iii) form an intermolecular bond, generating a concatemeric RNA. Treatment of each type of RNA with a complementary DNA oligomer and RNAse H, a nonspecific endonuclease that recognizes DNA/RNA duplexes, is expected to produce a unique number of degradation products of specific sizes depending on the RNA material which is tested.

To test circularization status of the RNA, 0.05pmol/µl of linear or circular RNA is incubated with 0.25U/µl of RNAse H, an endoribonuclease that digests DNA/RNA duplexes, and 0.3pmol/µl DNA oligomer against a region of the RNA at 37°C for 20 minutes. After incubation, the reaction mixture is analyzed by 6% denaturing PAGE.

For a linear RNA, it is expected that after binding of the DNA oligomer and subsequent cleavage by RNAse H, two cleavage products are obtained. A concatemer is expected to produce at least three cleavage products. A circular RNA is expected to produce a single cleavage product. This is visualized as the presence of one, two or three bands on the denaturing PAGE gel.

### Example 7. Reduced degradation susceptibility in circular RNAs

Circular RNA is more resistant to exonuclease degradation than linear RNA due to the lack of 5' and 3' ends. This Example describes reduced susceptibility of circular RNA to degradation by an exonuclease compared to linear RNA. RNAse R is used as a model exonuclease. In embodiments, this method is used to assess degradation susceptibility of any RNA.

Circular RNA is generated and circularized as described in Examples 2-4 for use in the assay. To test circularization, 20ng/µl of linear or circular RNA is incubated with 2U/µl of RNAse R, a 3' to 5' exoribonuclease that digests linear RNAs but does not digest lariat or circular RNA structures, at 37°C for 30 minutes. After incubation, the reaction mixture is analyzed by 6% denaturing PAGE.

The linear RNA bands present in the lanes lacking exonuclease are absent in the circular RNA lane, indicating that circular RNA shows higher resistance to exonuclease treatment as compared to a linear RNA control.

In one experiment, circular RNA was generated and circularized as described in Examples 1-4 for use in the assay. To test circularization, 260 ng ELVd RNA containing both circular and linear ELVd RNA was incubated with 10 U RNase R (Lucigen) (a 3' to 5' exoribonuclease that digests linear RNAs but does not digest lariat or circular RNA structures) and Terminator 5'-phosphate-dependent exoribonuclease (Lucigen). After incubation, the reaction mixture was column purified with 10 µg Monarch RNA Cleanup Kit (New England Biolabs) and analyzed by 6% denaturing PAGE. A low-range ssRNA ladder (New England Biolabs) was used as reference. The linear RNA bands at ~300 bases that were present in the samples that had not been treated with exonuclease had much higher intensity in comparison to the corresponding bands from samples that had been treated with exonuclease. In contrast, the circular RNA bands at ~500 bases that were present in the samples that had been treated with exonuclease had comparable intensity with that of the corresponding bands from samples not treated with exonuclease. This demonstrated that the circular RNAs had higher resistance to exoribonuclease treatment as compared to linear RNAs.

### Example 8. Isolation and purification of circular RNAs

This Example describes purification of circular RNAs. In some embodiments, this method is used to purify any circular RNA.

In certain embodiments, circular RNAs, as described in the previous Examples, are ligated, isolated from linear precursors, and purified before use in protoplasts or plants. This Example describes isolation of circular RNAs using UREA denaturing polyacrylamide gel separation.

Circular RNAs are synthesized as described in Examples 2-4. To purify the circular RNAs, ligation mixtures are resolved on 6% denaturing PAGE, and RNA bands corresponding to each of the circular RNAs are excised. Excised RNA gel fragments are crushed, and RNA is eluted with 800µl of 300mM NaCl overnight at 4°C. Gel debris is removed by filtration, and RNA is precipitated with ethanol in the presence of 0.3M sodium acetate. Eluted circular RNA is analyzed by 6% denaturing PAGE.

In one experiment, circular RNA was ligated and isolated from linear precursors using denaturing polyacrylamide (PAGE) gel separation in a protocol adapted from Nilsen, T. W. (2013). Gel purification of RNA. Cold Spring Harbor Protocols, 2013(2), pdb-prot072942. The resulting purified circular RNA is suitable for use in protoplasts or plants.

Circular RNAs were synthesized as described in Examples 2-4. To purify the circular RNAs, ligation mixtures were column purified using Monarch RNA cleanup kit (New England Biolabs) and resolved on 6% denaturing PAGE. RNA bands corresponding to each of the circular RNAs were excised and placed in a microcentrifuge tube with 400 µL of gel elution buffer (20 mM Tris-HCL, 0.25 M sodium acetate, 1 mM EDTA, 0.25% SDS), frozen on dry ice for 15 minutes, and incubated at room temperature overnight. (Alternatively, excised RNA gel fragments can be crushed using gel breaker tubes (IST Engineering Inc.), frozen on dry ice for 15 minutes, and incubated with gel elution buffer at room temperature for 6 hours or at 37 °C for 4 hours.) Gel debris was removed by microcentrifugation at maximum speed for 10 minutes at room temperature. RNA was then extracted from the supernatant with 1 volume of UltraPure phenol:chloroform:isoamyl alcohol (25:24:1, v/v) (Invitrogen), followed by 1 volume of chloroform. The RNA was precipitated with 2 volumes of ethanol and 1 µL glycogen (Thermo Scientific, v/v, 20 mg/mL) per mL of RNA/ethanol mixture, and eluted with RNase-free water. Eluted circular RNA was analyzed by 6% denaturing PAGE.

### Example 9. Delivery of circular RNA to plant protoplasts

This Example describes the production of plant protoplasts and delivery of circular RNA to protoplasts using polyethylene glycol (PEG).

As a model circular RNA, circular RNA containing an ELVd sequence with a Spinach RNA aptamer insertion is synthesized, isolated and purified as described in Examples 3 and 5. The method can be used to deliver other circular RNAs to protoplasts.

*Arabidopsis thaliana* and *Zea mays* are used as a model dicot and monocot, respectively. Protoplasts can be prepared from any plant, e.g., any dicot or monocot.

*Arabidopsis thaliana* and *Zea mays* plants are grown from seed for 3-4 weeks and 8-10 days, respectively. Protoplasts are prepared from them as described below.

### a. Monocot mesophyll protoplasts

The following mesophyll protoplast preparation protocol is generally suitable for use with monocot plants, e.g., maize (*Zea mays*) and rice (*Oryza sativa*):
An enzyme solution containing 0.6 molar mannitol, 10 millimolar MES pH 5.7, 1.5% cellulase R-10, and 0.3% macerozyme R-10 was prepared. The enzyme solution wa heated at 50-55 degrees Celsius for 10 minutes to inactivate proteases and facilitate R10 enzyme activity, and cooled to room temperature before adding 1 millimolar CaCl₂ , 5 millimolar beta-mercaptoethanol, and 0.1% bovine serum albumin. The enzyme solution was passed through a 0.45 micrometer filter. Washing solution containing 0.6 molar mannitol, 4 millimolar MES pH 5.7, and 20 millimolar KCI was prepared.

Second leaves of the plant were obtained, and the middle 6-8 centimeters are cut out. Ten leaf sections were stacked and cut into 0.5 millimeter-wide strips without bruising the leaves. The leaf strips were completely submerged in the enzyme solution in a petri dish, covered with aluminum foil, and incubated between 2-3 hours with gentle agitation. After digestion, the enzyme solution containing protoplasts was carefully transferred using a serological pipette through a 35 micrometer nylon mesh into a round-bottom tube; the petri dish was rinsed with 5 milliliters of washing solution and filtered through the mesh as well. The protoplast suspension was centrifuged at 1200 rpm, for 2 minutes in a swing-bucket centrifuge. The supernatant was aspirated without touching the pellet; the pellet was gently washed once with 20 milliliters washing buffer, and the supernatant was removed carefully. The pellet was resuspended by gently swirling in a small volume of washing solution, then resuspended in 10-20 milliliters of washing buffer. The tube was placed upright on ice for 30 minutes-4 hours (no longer). After resting on ice, the supernatant was removed by aspiration and the pellet resuspended with 2-5 milliliters of washing buffer. The concentration of protoplasts was measured using a hemocytometer and the concentration was adjusted to 2x10⁵ protoplasts/milliliter with washing buffer.

### b. Dicot mesophyll protoplasts

The following mesophyll protoplast preparation protocol (modified from one described by Niu and Sheen, Methods Mol. Biol., 876:195-206, 2012) is generally suitable for use with dicot plants such as *Arabidopsis thaliana* and brassicas such as kale (*Brassica oleracea*):
An enzyme solution containing 0.4 M mannitol, 20 millimolar KCI, 20 millimolar MES pH 5.7, 1.5% cellulase R-10, and 0.4% macerozyme R-10 was prepared and heated at 50-55°C for 10 minutes to inactivate proteases and facilitate R-10 enzyme activity. It was then cooled it to room temperature before adding 10 millimolar CaCl₂, 5 millimolar β-mercaptoethanol, and 0.1% bovine serum albumin, then passed through a 0.45 micrometer filter. A "W5" solution (154 millimolar NaCl, 125 millimolar CaCl₂, 5 millimolar KCI, and 2 millimolar MES at pH 5.7) and a "MMg solution" solution (0.4 molar mannitol, 15 millimolar MgC12 , and 4 millimolar MES at pH 5.7) were prepared.

The second or third pair of true leaves of the plant were obtained, and the middle section was cut. 4-8 leaf sections were stacked and cut into 0.5 millimeter wide strips without bruising the leaves. The leaf strips were submerged completely in the enzyme solution contained in a petri dish, covered with aluminum foil, and incubated for 2-3 hours with gentle agitation. After digestion, the enzyme solution containing protoplasts was carefully transferred using a serological pipette through a 35 micrometer nylon mesh into a round-bottom tube; the petri dish was rinsed with 5 milliliters of washing solution and filtered through the mesh as well. The protoplast suspension was centrifuged at 1200 rpm, 2 minutes in a swing-bucket centrifuge. The supernatant was aspirated without touching the pellet; the pellet was then gently washed once with 20 milliliters washing buffer, and the supernatant was removed carefully afterwards. The pellet was gently resuspended by swirling in a small volume of washing solution, then resuspended in 10-20 milliliters of washing buffer. The tube was placed upright on ice for 30 minutes-4 hours (no longer). After resting on ice, the supernatant was removed by aspiration and the pellet resuspended with 2-5 milliliters of MMg solution.

### c. Preparation of Arabidopsis and maize protoplasts

Plant protoplasts were prepared as described by Niu and Sheen, Plant Signalling Networks, 196-206, 2011. Briefly, *Arabidopsis thaliana* and *Zea mays* plants were grown from seed for 3-4 weeks, and well-expanded leaves were selected. The leaf tip was removed (3mm) and the middle part of the leaf was cut into 0.5-1mm strips. Leaf strips were transferred to the enzyme solution containing: 0.4 M mannitol, 20 mM KCI, 20 mM MES, pH 5.7, 1.5% cellulase R10 (Yakult Pharmaceutical Ind. Co., Ltd., Japan), 0.4% macerozyme R10 (Yakult Pharmaceutical Ind. Co., Ltd., Japan), 10 mM CaCl2, 1 mM b-mercaptoethanol, and 0.1% BSA. The petri dish was covered with aluminum foil and incubated for 2-3 hours with gentle agitation. The digestion time may vary depending on the material and experimental goals.

Protoplasts (monocot and dicot) were PEG transfected as described by Niu and Sheen, *Plant Signalling Networks,* 196-206, 2011. Briefly, protoplast cells were allowed to settle at the bottom of the tube and the W5 solution was pipetted out. The protoplast pellet was resuspended in MMg solution (0.4 M mannitol, 15 mM MgCl₂, 4 mM MES, pH 5.7) to a final concentration of 2 x 10⁵ /ml. 10 µl (10-20 ug) of circular RNA or linear control RNA, 100 µl of protoplasts in MMg solution, and 110 µl of PEG solution (40% (w/v) of PEG 4000 (Sigma-Aldrich), 0.2 M mannitol, and 0.1 M CaCl₂) were incubated at room temperature for 5-10 minutes. 440 µl of W5 solution was added and gently mixed by inverting to stop the transfection. The protoplasts were then pelleted by spinning at 110 x g for 1 minute, and the supernatant was removed. The protoplasts were gently resuspended with 500 µl of WI solution (0.5 M mannitol, 4 mM MES, pH 5.7, 20 mM KCI) in each well of a 12-well tissue culture plate and incubated for 24 and 48 hours.

### d. Confirmation of transfection

Presence of the transfected RNA in protoplasts can be confirmed using RNA extraction and quantitative RT-PCR. RNA extraction can be performed with the Maxwell^{®} RSC simplyRNA Blood Kit (Promega; AS1380) or RNAzol ^{®} RT (MRC). Quantitative RT-PCR can be employed on samples from different time points to measure level of circular RNA or linear control in protoplasts.

In instances in which the transfected RNA comprises or encodes a fluorescent moiety, presence of the target RNA in protoplasts can be confirmed using microscopy. To assay the fluorescence of RNA aptamer Spinach, protoplast aliquots are supplemented with 200 µM DFHBI and incubated for 1 additional hour. Pelleted protoplasts are photographed under a stereomicroscope (Leica MZ16 F) with UV illumination and a GFP2 filter (Leica). In the case of the RNA extracts, DFHBI is directly added to 20 µM of the RNA extract and photographed under the same conditions.

The presence of fluorescently labelled RNA can also be detected by microscopy. In one experiment, RNA including Cy3-fluorescently labeled UTPs was transfected into maize protoplasts. The transfected protoplasts were centrifuged at 110 x g for 1 minute and the supernatant was removed. The protoplasts were gently washed with WI solution, centrifuged at 110 x g for 1 minute, and the supernatant removed.

The protoplasts were then resuspended in 200 µL of WI; 20 µL of this protoplast suspension was imaged with an inverted fluorescent microscope (Olympus IXplore Standard), and the bright field and RFP filter images (at 10x or 40x magnification) were merged to provide a composite image. This allowed identification of a localized Cy3 signal in protoplast cells, confirming a positive transfection.

### Example 10. Delivery of circular RNA via leaf rubbing

This Example describes delivery of circular RNA to a plant via leaf rubbing. As a model circular RNA, circular RNA containing an ELVd sequence with a Spinach RNA aptamer insertion is synthesized, isolated and purified as described in Examples 3 and 5. In embodiments, this method is used to deliver other circular RNAs. *Arabidopsis thaliana* and *Zea mays* are used as a model dicot and monocot, respectively. In embodiments, leaf rubbing is used used to deliver circular RNA to any plant, e.g., any dicot or monocot.

*Arabidopsis thaliana* and *Zea mays* plants are grown from seed for 4 weeks. Circular RNA is diluted to a concentration of 10 µg/ml and delivered to leaves via rubbing as described by Hull, Current Protocols in Microbiology, 13(1): 16B.6.1-16B.6.4, 2009. Briefly, wearing a glove, the forefinger is wetted with the RNA solution and wiped gently onto one marked leaf. Alternatively, a glass spatula is used. In embodiments, the leaf rubbing protocol includes carborundum dusting. Ten minutes after rubbing, inoculated leaves are washed with water from a squeeze bottle. Plants are then placed in a growth chamber and incubated for 1, 2, 7 and 14 days.

To detect the total level of RNA, quantitative reverse transcriptase PCR (RT-qPCR) is performed on inoculated leaf, non-inoculated leaf, root and stem of plants from all groups. Spinach RNA aptamer levels are quantified using fluorescence microscopy as described in Example 3 and expressed as arbitrary units of fluorescence (a.u.f).

In one experiment, circular RNA, including a 21-nt sequence (as a mature miRNA or siRNA or alternatively as a miRNA precursor encoding the 21-nt mature miRNA) designed to silence the *Nicotiana benthamiana* phytoene desaturase (PDS) gene, was produced *in-vitro via* transformed *E.coli.* The total RNA from the transformed *E.coli* was extracted using a hot phenol RNA extraction method.

*Nicotiana benthamiana* were grown from seed for 4 weeks. The extracted RNA was diluted to a concentration of 25ng/µL of total RNA (estimated to be about 10% circular RNA), and 100µL of this RNA solution was delivered to each true leaf via rubbing as described by Hull, Current Protocols in Microbiology, 13(1): 16B.6.1-16B.6.4, 2009, with modification. Briefly, a forefinger of a gloved hand was wetted with the RNA solution and wiped gently onto a single marked leaf in the presence of powdered carborundum. One hour after rubbing, inoculated leaves were washed with millQ water from a squeeze bottle. Plants were then placed in a growth chamber and incubated under 16:8 light:dark cycle. Samples from each of four leaves per treatment condition were taken 8 days post-inoculation and RNA extracted from each leaf individually. Suppression of the target gene as an indicator of circular RNA delivery was assessed by measuring PDS levels with quantitative reverse transcriptase PCR (RT-qPCR) (two RT-qPCR reactions per RNA extraction). Samples from leaves treated with circular RNA molecules including either pre-miRNA or miRNA as cargos were compared to samples taken from a water-treated (negative) control. PDS levels in all samples were normalized to levels of the *Nicotiana tabacum* 60S ribosomal protein L23a-like reference gene (GENBank ID: 107805175). Results are provided in Table 7; decreased amounts of the PDS target gene were observed in samples from leaves treated with either of the circular RNAs, indicating that these circular RNAs including a pre-miRNA or a mature miRNA suppressed the PDS target gene.

**Table 7. Results**

| **Sample** | **Mean Percent Suppression** | **Standard Deviation** | **Relative Comparison** |
|---|---|---|---|
| Circular pre-miRNA | 14.4% | 49.4% | Circular pre-miRNA to Water Negative |
| Circular miRNA | 57.2% | 13.4% | Circular pre-miRNA to Water Negative |
| Water (negative) Control | -1.6% | 19.2% | Water Negative to Water Negative |

### Example 11. Delivery of circular RNA via leaf infiltration

This Example describes delivery of circular RNA to a plant via leaf infiltration.

As a model circular RNA, circular RNA containing ELVd sequence with a Spinach RNA aptamer insertion is synthesized, isolated, and purified as described in Examples 3 and 5. In embodiments, this method is used to deliver other circular RNAs. *Arabidopsis thaliana* and *Zea mays* are used as a model dicot and monocot, respectively. Leaf infiltration can be used to deliver circular RNA to any plant, e.g., any dicot. *Arabidopsis thaliana* and *Zea mays* plants are grown from seed for 4 weeks. Circular RNA is diluted to a concentration of 10 µg/ml and delivered to leaves via infiltration as described by Leuzinger et al., Journal of Visualized Experiments, 77: 50521, 2013. Briefly, 100 µl of the prepared RNA is loaded into a syringe without a needle and a small nick is made with the needle in the epidermis on the back side of a marked leaf.

Then, taking a firm hold of the front side of the leaf and applying gentle counter pressure to the nick with the thumb of one hand, the RNA solution is injected into the nick with the needle-less syringe. The injection is continued into the nick until the darker green circle indicating infiltration stops expanding. Another nick is made, and the injection repeated until the entire leaf is infiltrated and the whole leaf turns darker green. Plants are then placed in a growth chamber and incubated for 1, 2, 7 and 14 days.

To detect the total level of RNA, RT-qPCR is performed on inoculated leaf, non-inoculated leaf, root and stem of plants from all groups. Spinach RNA aptamer levels are quantified using fluorescence microscopy as described in Example 3 and expressed as a.u.f.

In one experiment, circular RNA containing ELVd wild-type sequence was synthesized, isolated, and purified as described in Examples 3 and 5, and infiltrated into *Nicotiana benthamiana. Nicotiana benthamiana* plants were grown from seed for 4 weeks. Circular RNA was diluted to a concentration of 3 mg/mL and delivered to leaves via infiltration as described by Leuzinger et al., Journal of Visualized Experiments, 77: 50521, 2013. Briefly, 100 µL of the prepared RNA was loaded into a syringe without a needle and a small nick was made with the needle in the epidermis on the back side of a marked leaf. The front side of the leaf was firmly held and a gentle pressure counter to the pressure of the nick was applied to infiltrate the RNA solution into the nick with the needle-less syringe. Infiltration was continued into the nick until the darker green circle indicating infiltration stopped expanding. Another nick was made, and the injection was repeated until the entire leaf was infiltrated and the whole leaf turned darker green. Plants were then placed in a growth chamber and incubated for 48 hours.

An RT-qPCR assay specific to circular ELVd molecules was used to measure total circular RNA from leaf-disc samples taken from circular ELVd-infiltrated, compared to samples taken from water-infiltrated (negative control) leaves, and normalized to levels of the *Nicotiana tabacum* 60S ribosomal protein L23alike reference gene (GENBank ID: 107805175). Furthermore, the measured amounts of circular ELVd were normalized to total RNA, and estimated circular ELVd copy number was determined with a standard curve containing known copy numbers of circular ELVd. Results are provided in Table 8.

**Table 8. Results**

| Hours after infiltration | Average Circular ELVd Fold Change (ELVd-infiltrated vs. water-infiltrated) | Standard Deviation | Average Copy Number Circular ELVd | Standard Deviation |
|---|---|---|---|---|
| 0 | 8.4 x 10^4 | 1.2 x 10^4 | 3.0 x 10^8 | 2.2 x 10^7 |
| 24 | 1.5 x 10^3 | 2.6 x 10^2 | 3.5 x 10^7 | 7.9 x 10^6 |

### Example 12. ELVd with a hairpin RNA targeting a tomato gene

This example describes the modification of a viroid to produce and deliver an RNA molecule that includes an effector to a plant and change its phenotype.

In this example, the RNA vector includes the following:
- ELVd (Eggplant Latent Viroid) (SEQ ID NO: 1)
- Effector: hpRNA-SIPDS: hairpin RNA (SEQ ID NO: 2) targeting a tomato (*Solanum lycopersicum;* SI) endogenous gene, PDS (phytoene desaturase)
   The hpRNA targeting SIPDS (SEQ ID NO: 2) is derived from virus-induced gene silencing (VIGS) PDS RNAi design (Liu et al., The Plant Journal, 31(6): 777-786, 2002), with a 9nt loop region replacing the original *Flaveria trinervia* Pyruvate orthophosphate DiKinase 2 (PDK) intron and 40nt targeting PDS exons. The hairpin (hp) RNA is inserted in U245-U246 of ELVd. The RNA vector (SEQ ID NO: 3) is synthesized in a bacterial viroid system as described in Example 3.

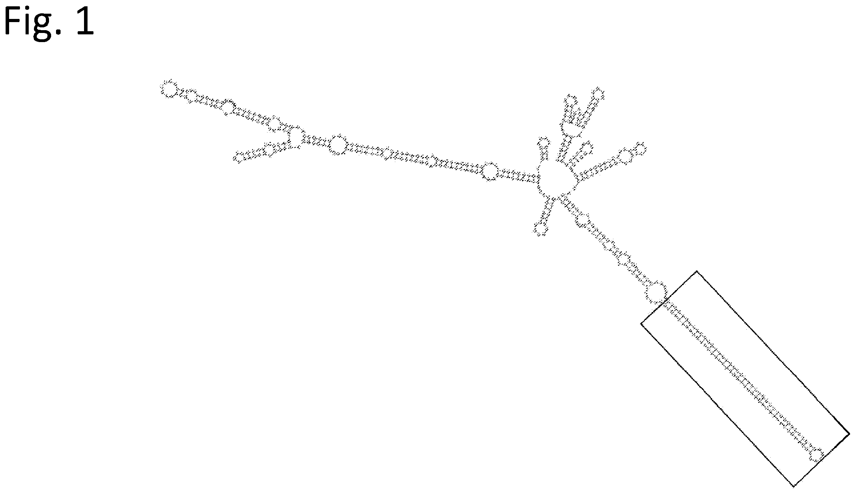

The resulting RNA vector, ELVd-hpRNA, (see Fig. 1; SEQ ID NO: 3), which targets tomato PDS (SIPDS, Gene ID: 544073), is transfected into tomato mesophyll protoplast cells, and its impact on PDS gene expression is detected with RT-qPCR. After the confirmation of PDS gene expression reduction by ELVd-hpRNA in protoplasts, the RNA vector is mechanically inoculated on tomato leaves by direct rubbing. Knockdown of PDS gene expression in tomato causes a photobleaching phenotype (Liu et al., The Plant Journal, 31(6): 777-786, 2002). The photobleaching phenotype is monitored for two weeks after inoculation, and the RNA vector and PDS gene expression are detected by RT-qPCR, as described herein.

### Characterization

The synthesized RNA vector, and ELVd alone as a control, are quantified using an Agilent 2100 Bioanalyzer system and analyzed using RNase H and RNase R assays, as described in Examples 6 and 7.

### Delivery and bioassay

### 1. Protoplast assay

Micro-Tom tomato (Totally Tomatoes) protoplast isolation is performed as described in Example 9 with the following modifications. The enzyme digestion step runs overnight (14 hours) at 26°C with gentle shaking at 25 rpm. After overnight digestion, protoplast cells are collected and purified with sucrose-gradient centrifugation. The RNA vector, and ELVd as a control, are delivered as described in Example 9 to Micro-Tom tomato protoplasts with polyethylene glycol (PEG). The cells are harvested at 6 hours, 12 hours, and 24 hours after transfection.

RNA extraction is performed using the Maxwell^{®} RSC simplyRNA Blood Kit (Promega; AS1380). Quantitative RT-PCR is employed on samples from different time points to measure transcript levels of the endogenous PDS gene and the RNA vector.

### 2. In planta assay

The RNA vector is mechanically inoculated into Micro-Tom tomatoes by leaf rubbing, as described in Example 10. Photobleaching phenotype is monitored for two weeks after inoculation. The inoculated leaves and distant leaves are imaged and processed using ImageJ to quantify photobleaching. RNA extraction is performed using the Maxwell^{®} RSC Plant RNA Kit (AS1500). Quantitative RT-PCR is employed on samples from different time points (12 hours, 1 day, 2 days, 4 days, 7 days, and 2 weeks following inoculation) to measure transcript levels of the endogenous PDS gene and the RNA vector, including siRNA produced downstream of hpRNA processing.

### Example 13. ELVd with a guide RNA targeting a corn gene

This example describes the modification of a viroid to produce and deliver an RNA molecule that includes an effector to a plant cell and edit the genome of the plant cell.

In this example, the RNA vector includes the following:
- ELVd (Eggplant Latent Viroid) (SEQ ID NO: 1)
- Effector: gRNA-gl2: guide RNA (SEQ ID NO: 4) targeting a corn (*Zea mays;* Zm) endogenous gene, gl2 (glossy2)

The guide RNA targeting the corn gene glossy2 (Zmgl2) (SEQ ID NO: 6) is designed based on the LbCas12a gRNA1 provided in Lee et. al. (Plant Biotechnology Journal, 17(2): 362-372, 2019) with a AsCas12a direct repeat (DR, SEQ ID NO: 7) on both 5' and 3' ends. The guide RNA is inserted in U245-U246 of ELVd. The RNA vector (SEQ ID NO: 5) is synthesized in a bacterial viroid system, as described in Example 3.

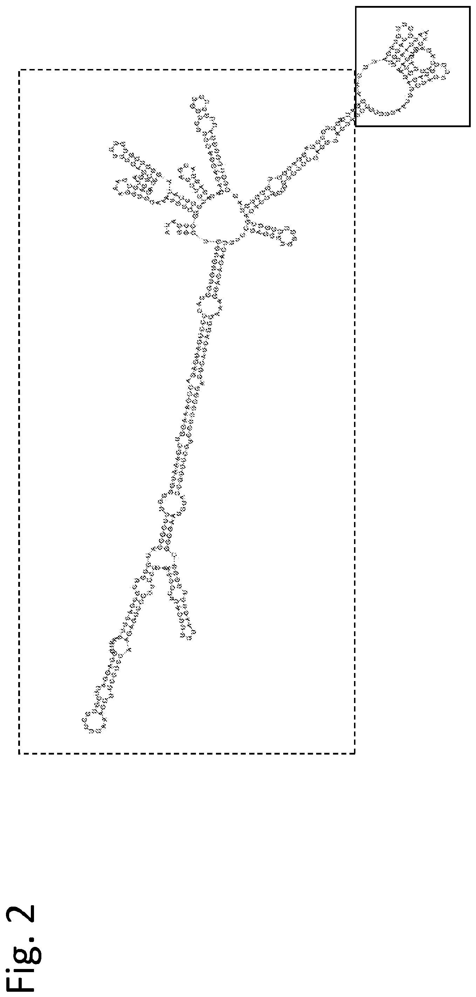

The resulting RNA vector, ELVd-gRNA, (see Fig. 2; SEQ ID NO: 5), which targets the corn *gl2* gene *(ZmgI2,* Gene ID: 103645956), is co-transfected with *Acidaminococcus* sp. Cas12a (AsCas12a; IDT^{™}, Cat# 10001272) into corn B73 mesophyll protoplast cells, and the editing efficiency is evaluated using Sanger sequencing and analyzed with the online tool ICE (Inference of CRISPR Edits) provided by Synthego.

### Characterization

The synthesized RNA vector, and ELVd alone as a control, are quantified using an Agilent 2100 Bioanalyzer system and analyzed using RNase H and RNase R assays, as described in Examples 6 and 7.

### Delivery and bioassay

### 1. In vitro AsCas12a nuclease cutting assay

The synthesized RNA vector is incubated with AsCas12a in IDTE buffer (IDT^{™}, Cat#11-05-01-05) for 30 minutes at room temperature or 37°C and then analyzed by gel electrophoresis. The product after incubation is also analyzed by incubating with a PCR amplicon (1kb) containing a gRNA-gl2 targeting site. The incubation is carried out at room temperature or 37°C and then analyzed by gel electrophoresis.

### 2. Protoplast assay

Maize B73 protoplast isolation is performed as described in Example 9. As a control, gRNA-gl2 is purchased from Integrated DNA Technologies (IDT^{™}) as the standard AsCas12a crRNA. The gl2 crRNA is complexed with AsCas12a protein to form RNP following the manufacturer's protocol. RNP and the RNA vector are delivered as described in Example 9 to maize B73 protoplasts with polyethylene glycol (PEG). The cells are harvested at 24 hours after transfection.

Genomic DNA extraction is performed using the Maxwell^{®} RSC Plant DNA Kit (Cat# AS1490). A 1kb PCR amplicon containing the guide RNA targeting region is amplified, followed by Sanger sequencing. The editing efficiency is calculated and analyzed with the online tool ICE (Inference of CRISPR Edits provided by Synthego.

In one experiment, an RNA vector was constructed to include a viroid sequence modified to include an effector, in this case a CRISPR guide RNA (gRNA) for editing a gene in a plant. More specifically, the vector included an Eggplant Latent Viroid (ELVd) modified to include the effector gRNA-LcPro3, a guide RNA targeting a corn (*Zea mays;* Zm) endogenous LC gene (see www[dot]maizegdb[dot]org/gene_center/gene/GRMZM5G822829). The guide RNA gRNA-LcPro3 used to target the corn gene, ZmLc (SEQ ID NO: 912) was designed based on the Cpf1 LcPro3 (SEQ ID NO: 913) disclosed in US Patent Application Publication US2019/0352655A1, with a AsCas12a direct repeat ("DR", SEQ ID NO: 914) on both the 5' and 3' ends. This guide RNA sequence was inserted at U245-U246 of the native ELVd sequence. The resulting RNA vector, ELVd-gRNA (SEQ ID NO: 915) was synthesized using the *in vitro* transcription system described in Example 1. Circularization was carried out by incubating with T4 PNK (T4 Polynucleotide kinase, New England Biolabs, catalogue number M0201S) for 1.5 hours, followed by ligation with T4 ligase 1 (New England Biolabs, catalogue number) for 3 hours. The observed efficiency of ligation was 33.3%, calculated based on the corresponding band intensity in polyacrylamide gel electrophoresis. The circular ELVd-gRNA was enriched to about 63% following RNase R treatment (Lucigen, catalogue number RNR07250) and quantified using polyacrylamide gel electrophoresis and analyzed using RNase R assays, as described in Examples 7. The synthesized RNA vector ELVd-gRNA (SEQ ID NO: 915) was subjected to an *in vitro* AsCas12a nuclease cutting assay to verify the insertion of the guide RNA. Briefly, the vector was incubated with AsCas12a in IDTE buffer (IDT^{™}, catalogue number 11-05-01-05) for 30 minutes at 37 °C and then analyzed by gel electrophoresis. A small band representing the guide RNA part (DR+LcPro3) was released from the full length ELVd-gRNA vector after AsCas12a incubation.

This vector, ELVd-gRNA (SEQ ID NO: 915) was tested for its ability to edit the target Lc gene in corn B73 mesophyll protoplast cells. Maize B73 protoplast isolation was performed as described in Example 9. As a control, gRNA-LcPro3 was purchased from Integrated DNA Technologies (IDT) as the standard AsCas12a crRNA. The LcPro3 crRNA was complexed with *Acidaminococcus* sp. Cas12a (AsCas12a; IDT, catalogue number 10001272) protein to form a ribonucleoprotein (RNP) following the manufacturer's protocol. The linear and circular ELVd-gRNA were either preassembled with AsCas12a to form RNPs or co-transfected with AsCas12a. These samples were delivered as described in Example 9 to maize B73 protoplasts with polyethylene glycol (PEG). The cells were harvested at 24 hours after transfection. Genomic DNA extraction was performed using Qiagen DNeasy Plant Mini Kit (Qiagen, catalogue number 69104). A 1107 bp PCR amplicon (SEQ ID NO: 916) containing the guide RNA targeting region was amplified with primers ZmLc-F (SEQ ID NO: 917) and ZmLc-R (SEQ ID NO: 918), followed by Sanger sequencing to determine the presence of edits. Editing efficiency was analyzed with the publicly available online tool TIDE (shinyapps[dot]datacurators[dot]nl/tide/). Results are provided in Table 9. The data demonstrate that viroids modified to include CRISPR guide RNAs are effective in editing a target gene.

**Table 9. Results**

| Replicate | IDT LcPro3 crRNA* | IDT LcPro3 crRNA** | Linear ELVd-gRNA* | Linear ELVd-gRNA** | Circular ELVd-gRNA* | Circular ELVd-gRNA** |
|---|---|---|---|---|---|---|
| 1 | 6.2 | 5.6 | 1.6 | 2.2 | 1.7 | 6.6 |
| 2 | 6.3 | 4.9 | 2.3 | 0.6 | 0.3 | 0.2 |
| 3 | 7.2 | 10.2 | 2.5 | 3 | 0.5 | 0.4 |

| | | | | | | |
|---|---|---|---|---|---|---|
| * transfected as a pre-assembled RNP ** co-transfected with AsCas12a | | | | | | |

### Example 14. PSTVd with a small RNA targeting a tomato gene

This example describes the modification of a viroid to deliver an RNA molecule that includes an effector to a plant, thereby changing the phenotype of the plant.

In this example, the RNA vector includes the following:
- PSTVd-RG1 (GenBank Acc. No. U23058): potato spindle tuber viroid strain RG1 (SEQ ID NO: 8)
- Effector: sRNA-SIPDS: small RNA (SEQ ID NO: 9) targeting a tomato endogenous gene, PDS (phytoene desaturase)

The small RNA targeting SIPDS is derived from VIGS PDS RNAi design (Liu et al., The Plant Journal, 31(6): 777-786, 2002), with 21nt targeting PDS. The region 191-211nt in the sense strand (+) of PSTVd-RG1 is removed and replaced with the effector sRNA-SIPDS sequence. The RNA vector (SEQ ID NO: 10) is synthesized using in vitro transcription, as described in Example 2.

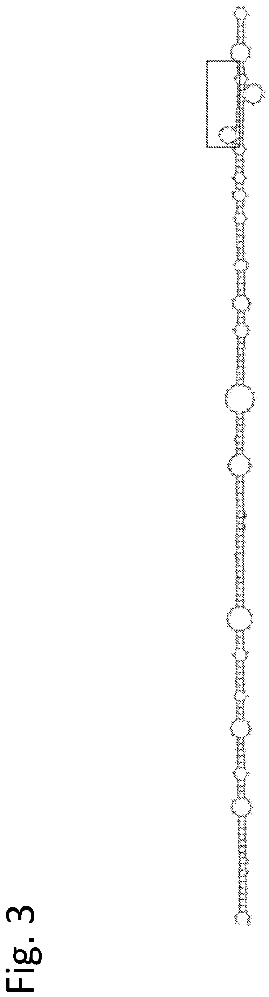

The resulting RNA vector, PSTVd-sRNA, (see Fig. 3; SEQ ID NO: 10), which targets the tomato gene PDS (SIPDS, Gene ID: 544073), is transfected into tomato mesophyll protoplast cells and its impact on PDS gene expression is detected with qRT-PCR. After the confirmation of PDS gene reduction by PSTVd-sRNA in protoplasts, the RNA vector is mechanically inoculated on tomato leaves by direct rubbing. Knockdown of PDS gene expression in tomato causes a photobleaching phenotype (Liu et al., The Plant Journal, 31(6): 777-786, 2002). The photobleaching phenotype is monitored for two weeks after inoculation, and the RNA vector and PDS gene expression are detected by qRT-PCR, as described herein.

### Characterization

The synthesized RNA vector, and PSTVd-RG1 alone as a control, are quantified using an Agilent 2100 Bioanalyzer system and analyzed using RNase H and RNase R assays, as described in Examples 6 and 7.

### Delivery and bioassay

### 1. Protoplast assay

Micro-Tom tomato protoplast isolation is performed as described in Example 12. The RNA vector and PSTVd-RG1 as control are delivered as described in Example 9 to Micro-Tom tomato protoplasts with polyethylene glycol (PEG). The cells are harvested at 6 hours, 12 hours, and 24 hours after transfection. RNA extraction is performed using the Maxwell^{®} RSC simplyRNA Blood Kit (AS1380). Quantitative RT-PCR is employed on samples from different time points to measure transcript levels of the endogenous PDS gene and the RNA vector.

### 2. In planta assay

The RNA vector is mechanically inoculated to Micro-Tom tomato by leaf rubbing. The photobleaching phenotype is monitored for two weeks after inoculation. The infected leaves and distant leaves are imaged and processed using ImageJ to quantify. RNA extraction is performed using the Maxwell^{®} RSC Plant RNA Kit (AS1500). Quantitative RT-PCR is employed on samples from different time points (12 hours, 1 day, 2 days, 4 days, 7 days, and 2 weeks following inoculation) to measure transcript levels of the endogenous PDS gene and the RNA vector.

In one experiment, a viroid was modified to deliver an RNA molecule including an effector to a plant, resulting in a change in the plant's phenotype. More specifically, this example illustrates a viroid vector based on potato spindle tuber viroid strain RG1 (SEQ ID NO: 8, PSTVd-RG1,GenBank Acc. No. U23058) and modified to include as the effector a small RNA, "sRNA-SIPDS" (SEQ ID NO: 9) that targets a tomato endogenous gene, PDS (phytoene desaturase). The sRNA-SIPDS sequence was derived from a viral-induced gene silencing ("VIGS") PDS RNAi design described Liu et al. (2002) Plant J., 31:777-786, with 21 nucleotides that target PDS; suppression of PDS expression in tomato plants causes a photobleached phenotype. The region at positions 191-211 nt in the sense (+) strand of wild-type PSTVd-RG1 (SEQ ID NO:8) was deleted and replaced with the effector sRNA-SIPDS sequence (SEQ ID NO:9). The resulting RNA vector (SEQ ID NO:919) was synthesized using *in vitro* transcription, as described in Example 2. Two additional RNA vectors, "PSTVd-siRNA 1"(SEQ ID NO: 920) and "PSTVd-siRNA 2" (SEQ ID NO: 921), were constructed similarly to include the 21-nucleotide targeting the tomato PDS gene. The RNA vectors were individually inoculated into tomato leaves by rubbing (see Example 10) and the plants monitored for photobleaching for 7 weeks after inoculation.

The RNA vectors, PSTVd-sRNA, (SEQ ID NO: 919), "PSTVd-siRNA 1"(SEQ ID NO:920) and "PSTVd-siRNA 2" (SEQ ID NO: 921), which target the tomato gene PDS (SIPDS, Gene ID: 544073), and the wild-type PSTVd-RG1 as a control, were quantified using an Agilent 2100 Bioanalyzer system and analyzed using RNase H and RNase R assays, as described in Examples 6 and 7. The PSTVd-sRNA vector was mechanically inoculated on Rutgers tomato leaves by direct rubbing. Knockdown of PDS gene expression in tomato causes a photobleaching phenotype (Liu et al., The Plant Journal, 31(6): 777-786, 2002). The photobleaching phenotype was monitored for 7 weeks after inoculation, and the RNA vector and PDS gene xpression were detected by qRT-PCR, as described herein. Leaf samples from the inoculated plants were taken, and RNA extracted using the MagMax^{™} mirVANA^{™} Total RNA Isolation Kit (A27828). Quantitative RT-PCR was used to measure transcript levels of the endogenous PDS gene and of the RNA vector, normalized to the reference gene GAPDH (GenBank ID: U93208.1). The percent suppression of the target gene PDS in PSTVd-sRNA-treated plants, relative to the PSTVd-RG1-treated control plants, is shown in Table 10.

**Table 10. Results.**

| Sample | Percent Suppression: Mean (SD) |
|---|---|
| PSTVd-sRNA (SEQ ID NO: 919) | 39.5% (27.4%) |
| PSTVd-siRNA 1 (SEQ ID NO: 920) | 34.9% (25.7%) |
| PSTVd-siRNA 2 (SEQ ID NO: 921) | 22.2% (49.7%) |

### Example 15. PSTVd TL-R or PSTVd R with a pre-miRNA targeting a tomato gene

This example describes the utilization of the replication motif from a viroid to replicate an RNA molecule that includes an effector in a plant cell or plant protoplast.

In this example, the RNA vector includes the following:
- Replication domain of PSTVd (potato spindle tuber viroid), consisting of either:
   ∘ PSTVd TL-R (Table 2, SEQ ID NO: 11): the left-terminal region of PSTVd, including a binding site for the DNA-dependent RNA polymerase II (Pol II) and the transcription factor IIIA containing seven zinc finger domains (TFIIIA-7ZF); or
   ∘ PSTVd TL-CCR (SEQ ID NO: 12): the left-terminal and central conserved region of PSTVd
- Effector: amiR-PDS: a pre-miRNA (SEQ ID NO: 13) targeting a plant endogenous gene, PDS (phytoene desaturase)

Pre-miRNA targeting tomato (*Solanum lycopersicum;* SI) PDS (SIPDS) (amiR-SIPDS, SEQ ID NO: 13) is designed with Web MicroRNA Designer (WMD3) on the Arabidopsis precursor MIR319a. The amiR-SIPDS is fused to PSTVd TL-R (SEQ ID NO: 11) or PSTVd TL-CCR (SEQ ID NO: 12). The RNA vectors (SEQ ID NO: 14 and SEQ ID NO: 15) are synthesized with in vitro transcription, as described in Example 2. Two forms (linear and circular) of each RNA vector are synthesized and tested in protoplast cell assays.

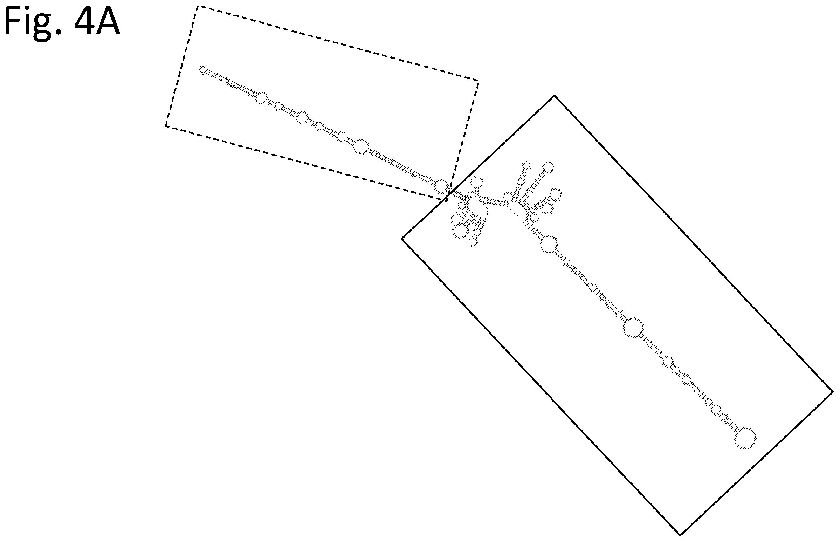

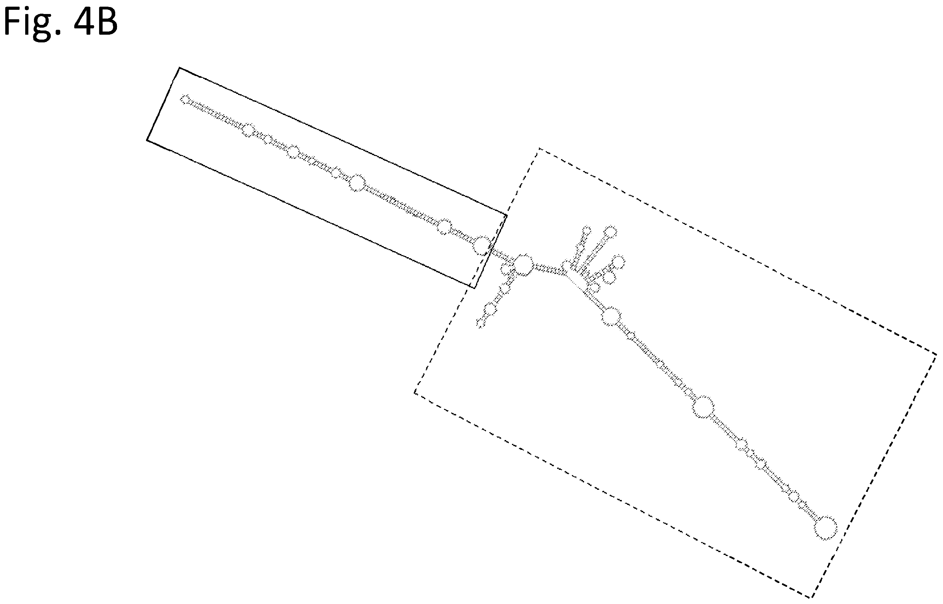

The resulting RNA vectors, TL-R-amiR-PDS (Fig. 4A; SEQ ID NO: 14) and R-amiR-PDS (Fig. 4B; SEQ ID NO: 15), which target tomato PDS (SIPDS, Gene ID: 544073), are transfected into tomato mesophyll protoplast cells. Replication of the RNA vectors and their impact on PDS gene expression are detected using qRT-PCR.

### Characterization

The synthesized linear and circular fusion RNA vectors are quantified using an Agilent 2100 Bioanalyzer system and analyzed using RNase H and RNase R assays, as described in Examples 6 and 7.

### Delivery and bioassay

The RNA vectors are delivered to Micro-Tom tomato protoplasts with polyethylene glycol
(PEG), and their replication is detected by qRT-PCR measurement of RNA vectors and of transcripts of the endogenous PDS gene.

Micro-Tom tomato protoplast isolation is performed as described in Example 12. The RNA vector and PSTVd-RG1 as control are delivered as described in Example 9 to Micro-Tom tomato protoplasts with polyethylene glycol (PEG). The cells are harvested at 6 hours, 12 hours, and 24 hours after transfection. RNA extraction is performed using the Maxwell^{®} RSC simplyRNA Blood Kit (AS1380). Quantitative RT-PCR is employed on samples from different time points to measure transcript levels of the endogenous PDS gene and the circular and linear fusion RNAs.

### Example 16. Arabidopsis circRNA with a PSTVd replication motif

This example describes the use of an RNA replication motif to amplify the effects of an endogenous regulatory RNA in a plant.

In this example, the RNA vector includes the following:
- PSTVd Left Terminal domain (TL; SEQ ID NO: 33)
- Effector: circGORK: circular RNA (SEQ ID NO: 16) derived from the intron segment flanking exons 2 and 3 of the *Arabidopsis* GATED OUTWARDLY-RECTIFYING K+ CHANNEL (GORK) gene. GORK regulates stomatal opening and drought resistance.

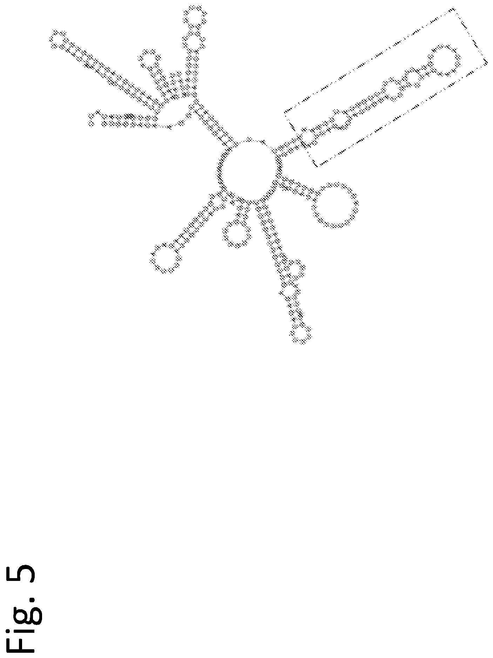

The PSTVd TL region is as shown in Table 2. The circGORK is designed according to the endogenous circular RNA detected and described by Zhang et al., The Plant Journal, 98(4): 697-713, 2019. The RNA vector (SEQ ID NO: 17) is synthesized using the in vitro transcription system described in Example 2. The resulting RNA vector, PSTVd/TL-circGORK, (see Fig. 5; SEQ ID NO: 17), which targets drought resistance pathways, is transfected into *Arabidopsis* plants by mechanical leaf rubbing, as described herein, and its ability to replicate in leaves is quantified using qRT-PCR. Once presence and replication of RNA is confirmed, plants are subjected to a drought assay to measure the ability of PSTVd/TLR-circGORK to induce a drought resistance phenotype as described by Zhang et al., The Plant Journal, 98(4): 697-713, 2019 for circGORK.

### Characterization

The synthesized RNA vector (SEQ ID NO: 17) and PSTVd alone as a control are quantified using an Agilent 2100 Bioanalyzer system and analyzed using RNase H and RNase R assays, as described in Examples 6 and 7.

### Delivery and bioassay

The RNA vector is mechanically inoculated to Arabidopsis by leaf rubbing. Control and inoculated plants are subjected to a drought stress as described by Zhang et al., The Plant Journal, 98(4): 697-713, 2019. Briefly, the soil moisture of each treatment is monitored by measuring the relative soil water content, followed by rationing of water to maintain a designated soil moisture. The pots are weighed and watered twice daily. After the most serious stress reached the preset levels, the plants are maintained for an extra 1 day, then the whole plants are harvested for fresh weight measurement and leaves are harvested for RNA extraction. Fresh weight of drought-resistant plants is higher than that of control plants.

RNA extraction is performed using the Maxwell^{®} RSC simplyRNA Blood Kit (AS1380). Quantitative RT-PCR is employed on samples from different time points to measure transcript levels of the RNA vector.

### Example 17. In planta viral trafficking motifs conjugated to a fluorescent aptamer as a circular fusion RNA

This example demonstrates the *in planta* trafficking of a circular fusion RNA conjugated to a fluorescent aptamer using splint ligation. The circular fusion RNAs contain a PSTVd loop 27 (Table 2) viral motif sequence (5'-UUUUCA-3'; SEQ ID NO: 18) previously described to be essential for viral trafficking within the host plant (Wu et al., PLoS Pathogens, 15(10): e1008147, 2019). The fusion RNA is synthesized as described in Example 2. This trafficking motif is synthesized in tandem with either an intact or split Broccoli RNA aptamer sequence as an exemplary cargo.

### Design

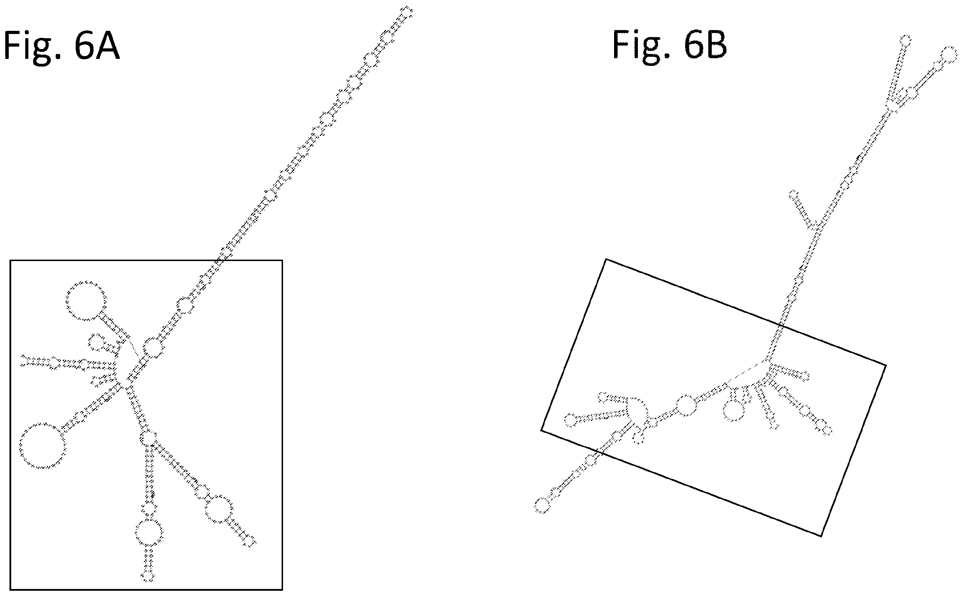

A circular fusion RNA 1 (CircRNA1) construct (Fig. 6A; SEQ ID NO: 20) is generated containing the following elements:
- PSTVd right terminal domain containing transmission motifs (loop 26 and loop 27) (SEQ ID NO: 18)
- Intact Broccoli RNA aptamer sequence (SEQ ID NO: 19)

A circular fusion RNA 2 (CircRNA2) construct (Fig. 6B; SEQ ID NO: 23) is generated containing the following elements:
- PSTVd right terminal domain containing transmission motifs (loop 26 and loop 27) (SEQ ID NO: 18)
- Linker region (SEQ ID NO: 21)
- Split Broccoli aptamer sequence (SEQ ID NO: 22)

Linear transcripts are synthesized using a T7 *in vitro* transcription reaction, and circular fusion RNAs are generated using T4 RNA ligase 2 on a 20nt splint DNA oligomer template (SEQ ID NO: 24), as described in Example 2.

### Characterization

The ratio of linear to circular fusion RNAs is detected after incubation with RNase R and migration of samples on a 6% PAGE gel. Transcript topology can also be visualized by circularization-dependent aptamer coordination of the fluorescent molecule DFHBI-1T.

### Delivery and Bioassay

To test whether the viral motif embodied in the circRNA is capable of trafficking throughout the tomato plant, the synthesized, circularized CircRNA1 or CircRNA2 is rubbed onto a leaf at the base of an *Arabidopsis thaliana* plant. Linear transcripts of CircRNA1 and CircRNA2 are provided as negative controls. Leaves distal to the site of inoculation are analyzed for Broccoli aptamer transcripts by qRT-PCR. Successful trafficking of the circular RNA throughout the plant is confirmed by distal plant structures containing circRNA transcripts, as measured by qRT-PCR. Transcripts in distal leaves in circular RNA are not expected to be observed in plants treated with constructs that do not have the trafficking motif sequence. Additionally, efficiently circularized and trafficked RNA constructs are visualized by green fluorescence in distal leaf structures when incubated with 10µM DFHBI-1T fluorogen (Tocris, 5610) for 30 minutes to one hour. Linear constructs are expected to have lower or absent fluorescence when compared with CircularRNAs. Linear CircRNA1 containing the intact Broccoli aptamer is expected to have lower fluorescence than a circularized CircRNA1. Linear CicRNA2 containing the split Broccoli aptamer is expected to have no fluorescence and will only fluoresce upon circularization and formation of the full aptamer sequence.

Detection of Broccoli aptamer transcripts, as well as the emission of green fluorescence at 472nm, indicates both efficient circularization and trafficking of the circular fusion RNA.

### Example 18. PSTVd with a Spinach RNA aptamer in Arabidopsis and corn

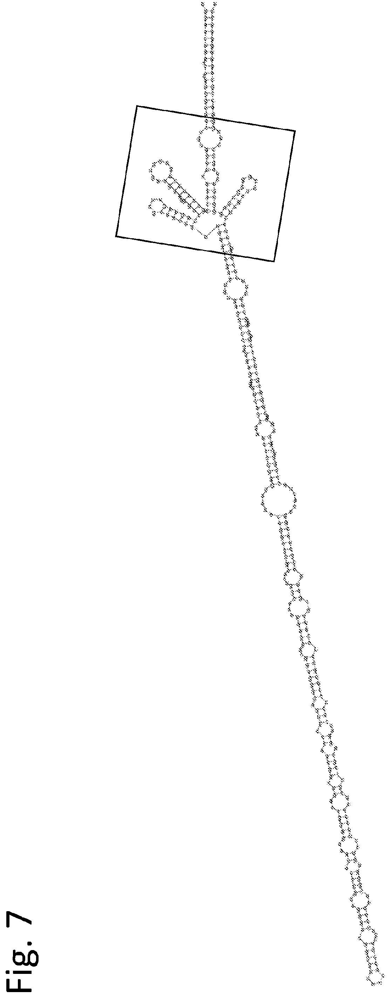

This example describes PSTVd infection that is host-specific. To illustrate host specific infection of PSTVd, the pathogenicity domain (SEQ ID NO: 25) of PSTVd is deleted and replaced with a Spinach RNA aptamer (SEQ ID NO: 26; Fig. 7). The RNA construct further contains a linker (1-100nt) containing one or more of randomly generated sequence; contiguous or split fluorescent aptamers (e.g., Baby_spinach, Mango3, or Broccoli); and sequence derived from non-coding plant RNAs retrieved from publicly available RNAseq databases. *In vitro* transcription of RNAs is performed using a T7 *in vitro* transcription reaction.

### Design

In this example, the RNA vector includes the following:
- PSTVd lacking the pathogenicity domain (See Table 2)
- Spinach aptamer (SEQ ID NO: 26)

### Characterization

RNAs are treated with DNase to remove the DNA template. Linear RNAs are then column-purified using the Zymo RNA Clean & Concentrator-5 kit (Zymo Research: R1014). Linear transcribed RNAs are quality tested by heating *in vitro* transcription products to 80°C for 7-10 minutes. Heated RNAs are then run on a 6% denaturing PAGE gel to validate purity of transcribed RNA and RNA quality.

### Delivery and Bioassay

The linear PSTVd-Spinach fusion RNA (SEQ ID NO: 27) is rubbed onto the leaves of *Arabidopsis* and corn plants to test for infection of the host (*Arabidopsis*) and non-host species (corn). Leaves distal and proximal to the site of inoculation are removed and incubated with 10µM DFHBI-1T fluorogen (Tocris, 5610) for 30 minutes to one hour. Successful circularization and infection of the host plant results in an emission of green fluorescence at 472 nm. Spinach RNA aptamer transcripts are also quantified using qRT-PCR of proximal and distal leaves after inoculation. Absence of PSTVd-Spinach transcripts in distal corn leaves will indicate that PSTVd infection is specific to previously described host plants.

### Example 19. An RNA motif to traffic aptamers to a subcellular location

This example describes the use of an RNA targeting motif to traffic a Spinach RNA aptamer to a subcellular location, the chloroplast.

In this example, the RNA vector includes the following:
- ELVd (Eggplant Latent Viroid) (SEQ ID NO: 1) Effector: Spinach RNA aptamer (SEQ ID NO: 26) ELVd is designed using the sequence of the complete eggplant latent viroid genome (GenBank Acc. No. AJ536613.1; SEQ ID NO: 28) and the repetition of the plus-strand hammerhead ribozyme domain for the longer-than-unit ELVd (SEQ ID NO: 1); see, e.g., Branch et al.(1981) Proc. Natl. Acad. Sci. USA, 78:6381 - 6381, DOI: 10.1073/pnas.78.10.6381; Tabler et al.(1992) Virology, 190:746 - 753, DOI: 10.1016/0042-6822(92)90912-9; Cordero et al. (2018) Frontiers Microbiol., 9:635, DOI: 10.3389/fmicb.2018.00635; Flores et al. (2012) Frontiers Microbiol.,3:217, DOI: doi.org/10.3389/fmicb.2012.00217. The Spinach RNA aptamer is designed using the sequence described in Example 3. The RNA vector (SEQ ID NO: 29) is synthesized using a bacterial transcription system as described in Example 3.

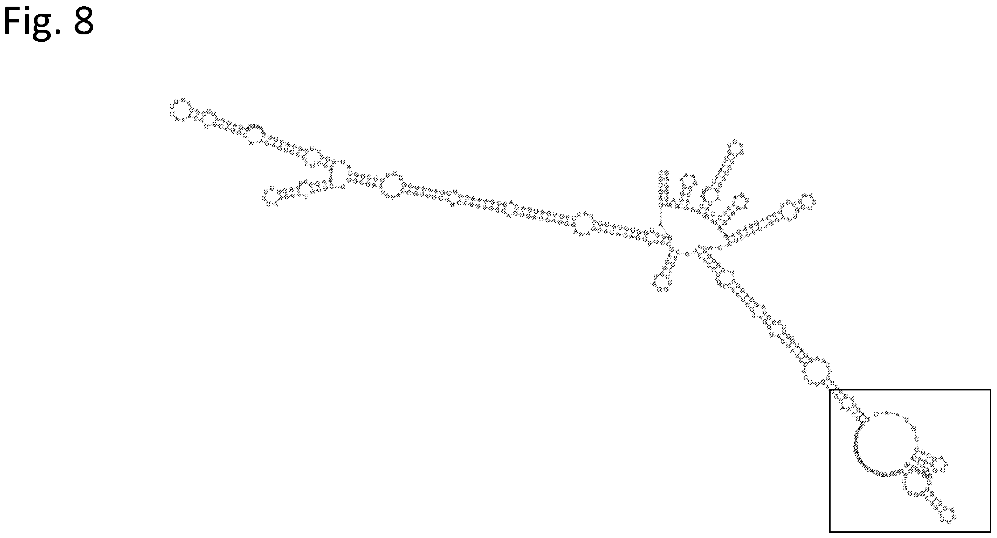

The resulting RNA vector, ELVd-Spinach, (see Fig. 8B; SEQ ID NO: 29) is transfected into tomato mesophyll protoplast cells and its trafficking to chloroplasts is detected with qRT-PCR and fluorescence imaging. After the confirmation of trafficking, the RNA vector is mechanically inoculated on tomato leaves by direct rubbing. Fluorescence and RNA presence are quantified in leaves by imaging and qRT-PCR.

### Characterization

The synthesized RNA vector (SEQ ID NO: 29) and linear RNA controls are quantified using an Agilent 2100 Bioanalyzer system and analyzed using RNase H and RNase R assays, as described in Examples 6 and 7.

### Delivery and bioassay

### 1. Protoplast assay

Micro-Tom tomato protoplast isolation is performed as described in Example 9 with modifications listed here. Micro-Tom tomato protoplast isolation is performed as described in Example 12. The RNA vector and linear RNA as control are delivered as described in Example 9 to Micro-Tom tomato protoplasts with polyethylene glycol (PEG). The cells are harvested at 6 hours, 12 hours, and 24 hours after transfection. Quantitative RT-PCR is employed on samples from each time point to measure transcript level of the RNA vector.

### 2. In planta assay

The RNA vector is mechanically inoculated into Micro-Tom tomatoes by leaf rubbing, as described in Example 10. Quantitative RT-PCR is employed on samples from different time points (12 hours, 1 day, 2 days, 4 days, 7 days, and 2 weeks following inoculation) to measure transcript levels of the RNA vector. Spinach RNA aptamer fluorescence is detected using the protocol described in Example 3 using leaf tissue.

### Example 20. An RNA motif to traffic pre-miRNA to a specific cell type

This example describes the use of an RNA targeting motif to traffic and deliver an RNA molecule that includes an effector to a specific plant cell type and change its phenotype.

In this example, the RNA vector includes the following:
- PSTVd right terminal domain containing transmission motifs (loop 26 and loop 27) (PSTVd TR; SEQ ID NO: 18).
- Effector: tomato PDS (phytoene desaturase) pre-miRNA (amiR-PDS) (SEQ ID NO: 13).
   Loop 27 (L27) (177 to 182 nts) (SEQ ID NO: 18) of PSTVd is selected based on the sequence identified by Wu et al., PloS Pathogens, 15(10): e1008147, 2019. This RNA motif enables trafficking of the vector from epidermal to palisade spongy mesophyll cells. The RNA vector is synthesized using in vitro transcription and splint ligation, as described in Example 2.

The resulting RNA vector (SEQ ID NO: 32), PSTVd/TR-amiRPDS (Fig. 9), which targets tomato PDS (SIPDS, Gene ID: 544073), is transfected into tomato mesophyll protoplast cells, and its impact on PDS gene expression is detected with qRT-PCR. After the confirmation of PDS gene reduction by PSTVd/TR-amiRPDS in protoplasts, the RNA vector is mechanically inoculated on tomato leaves by direct rubbing. Knockdown of PDS gene expression in tomato causes a photobleaching phenotype (Liu et al., The Plant Journal, 31(6): 777-786, 2002). The photobleaching phenotype is monitored for two weeks after inoculation, and the RNA vector and PDS gene expression are detected by qRT-PCR, as described herein.

### Characterization

The synthesized RNA vector (SEQ ID NO: 32) and linear RNA controls are quantified using an Agilent 2100 Bioanalyzer system and analyzed using RNase H and RNase R assays, as described in Examples 6 and 7.

### Delivery and bioassay

### 1. In planta assay

The RNA vector is mechanically inoculated into Micro-Tom tomatoes by leaf rubbing, as described in Example 10. Photobleaching phenotype is monitored for two weeks after inoculation. The inoculated leaves and distant leaves are imaged and processed using ImageJ to quantify photobleaching. RNA extraction is performed using the Maxwell^{®} RSC Plant RNA Kit (AS1500). Quantitative RT-PCR is employed on samples from different time points (12 hours, 1 day, 2 days, 4 days, 7 days, and 2 weeks following inoculation) to measure transcript levels of the endogenous PDS gene and the RNA vector, including miRNA produced downstream of pre-RNA processing.

### Example 21: An RNA motif to traffic an aptamer to a specific tissue type

This example describes the use of an RNA targeting motif to traffic and deliver an RNA molecule that includes an aptamer to a specific plant tissue type.

In this example, the RNA vector includes the following:
- PSTVd Left Terminal domain (TL; SEQ ID NO: 33)
- Effector: Spinach RNA aptamer SEQ ID NO: 26

Loop 6 (L6) (U43/C318) (SEQ ID NO: 33) of PSTVd is selected based on the motif identified by Zhong et al., The EMBO Journal, 26(16): 3836-3846, 2007. This RNA motif enables trafficking of the vector into vascular tissue for transport into non-inoculated parts of the plant. The RNA vector is synthesized using in vitro transcription and splint ligation, as described in Example 2.

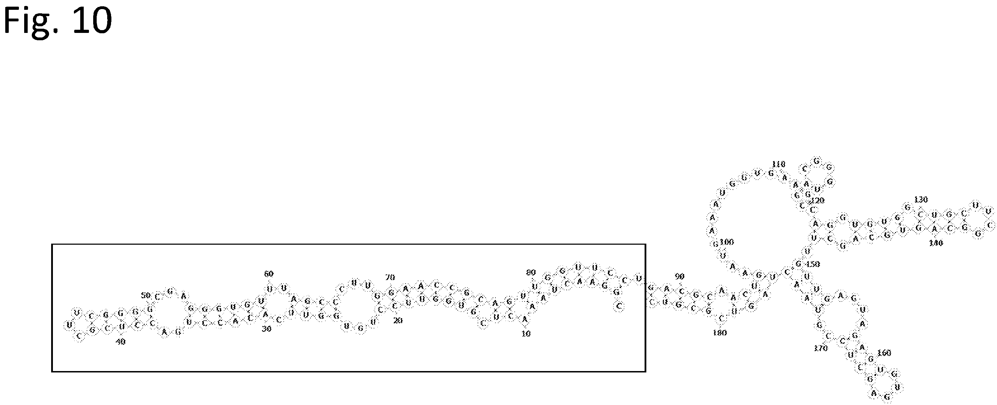

The resulting RNA vector, PSTVd/TL-Spinach, (SEQ ID NO: 34; Fig. 10) is mechanically inoculated on tomato leaves by direct rubbing. Fluorescence and RNA presence are quantified in distal tissues by imaging and qRT-PCR.

### Characterization

The synthesized RNA vector (SEQ ID NO: 34) and linear RNA controls are quantified using an Agilent 2100 Bioanalyzer system and analyzed using RNase H and RNase R assays, as described in Examples 6 and 7.

### Delivery and bioassay

### 1. In planta assay

The RNA vector is mechanically inoculated to Micro-Tom tomato by leaf rubbing, as described in Example 10. This delivers the vector to epidermal cells, and trafficking to vascular tissue is enabled by Loop 6. Quantitative RT-PCR is employed on inoculated and non-inoculated leaf, stem and root tissue samples from different time points (12 hours, 1 day, 2 days, 4 days, 7 days, and 2 weeks following inoculation) to measure transcript levels of the RNA vector. Spinach RNA aptamer fluorescence is detected using the protocol described in Example 3 using all tissues.

### Example 22. Circular RNAs with PSTVd LT and EMCV or maize HSP101 IRES in monocots vs. dicots

This example demonstrates *in vitro* synthesis of circular fusion RNA and the subsequent expression of circRNA *in planta.*

### Design

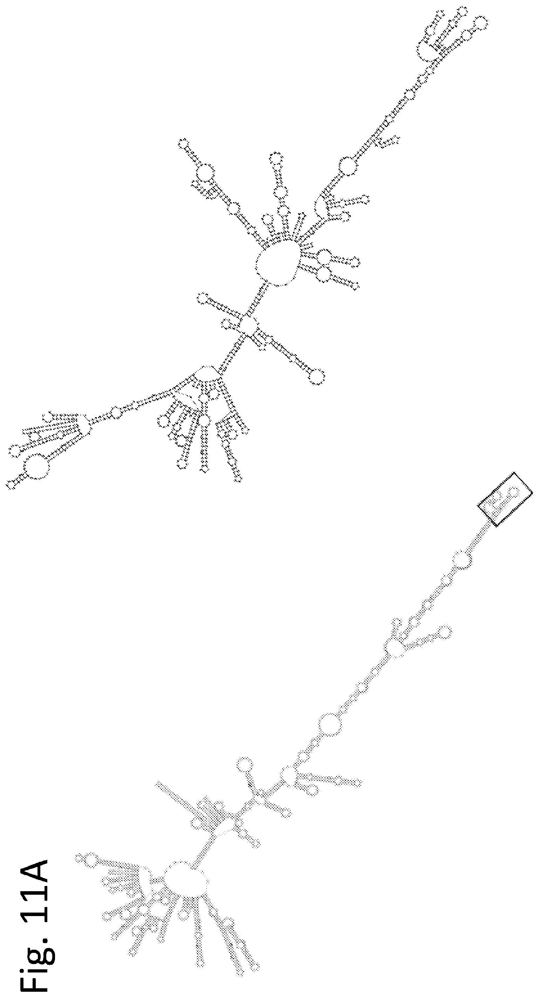

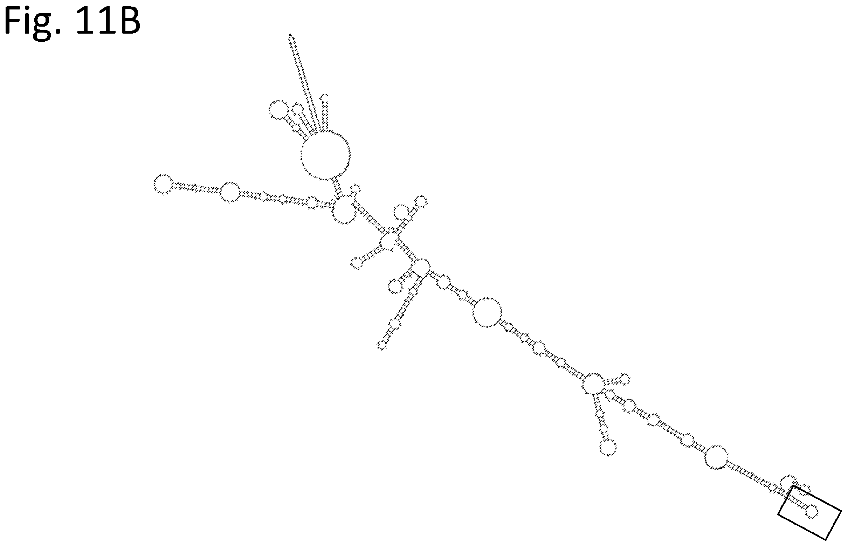

In this example, the RNA vector includes the following:
A first vector (Fig. 11A)
   - PSTVd left terminal region (PSTVD LT) (SEQ ID NO: 18)
   - Encephalomyocarditis virus internal ribosome entry site (EMCV IRES) (SEQ ID NO: 37)
   - PSP72 cloning vector (SEQ ID NO: 49)
   - Nanoluciferase (SEQ ID NO: 39)
A second vector (Fig. 11B)
   - PSTVd left terminal region (PSTVD LT) (SEQ ID NO: 18)
   - Maize HSP101 internal ribosome entry site (MAIZE HSP101 IRES) (SEQ ID NO: 38)
   - PSP72 cloning vector (SEQ ID NO: 49)
   - Nanoluciferase (SEQ ID NO: 39)

A circular RNA construct (Figs. 11A and 11B, SEQ ID NO: 35 or SEQ ID NO: 40) is generated and contains the left terminal region of the PSTVd viroid sequence (SEQ ID NO: 36) found in Table 1 linked to an encephalomyocarditis virus (EMCV) internal ribosomal entry site (IRES) (SEQ ID NO: 37) or a maize heat shock protein of 101 KDa (HSP101) IRES (SEQ ID NO: 38) found on the IRESite online database for IRES sequences. IRES elements are placed upstream of the protein coding luciferase enzyme (SEQ ID NO: 39) and is subcloned into a pSP72 expression vector (Promega, REF: P2221). *In vitro* transcription of RNAs is performed using the T7 RNA polymerase promoter. Circular RNA precursors are synthesized using the the HiScribe^{™} T7 Quick High Yield RNA Synthesis Kit (New England BioLabs^{®} Inc., REF: E2050S). DNase is incubated with the *in vitro* transcription products to remove template DNA. CircularRNA is enriched as previously described in Example 16 by starting with 20µg of RNA diluted in water. RNAs are then heated to 65°C for 3 minutes, followed by incubating RNAs with RNase R at 37°C for 15 minutes. Splint ligation is performed as described in Example 2.

### Characterization

Circular RNA is column purified using MEGAClear^{™} Transcription Clean-Up Kit (ThermoFisher, AM1908) as previously described (Wesselhoeft et al., Nature Communications, 9: Article no. 2629, 2018) and in Example 8.

### Delivery and Bioassay

Plant protoplasts are isolated as described in Example 9 and are incubated with linear or circular RNAs for 24 hours. Circular RNAs containing the EMCV IRES-luciferase or maize HSP101 IRES-luciferase are transfected into *Arabidopsis* and maize protoplasts respectively.

To measure the total level of transcribed RNAs of luciferase or fluorescent proteins, quantitative reverse transcription PCR (qRT-PCR) is performed. The total RNA as well as RNA treated with RNase R to enrich for circularRNA is converted to cDNA using cDNA Superscript^{™} III First Strand Synthesis System with random hexamers according to the manufacturer's instructions (ThermoFisher Scientific, 18080051). Additionally, the transcription efficiency of the IRES sequence in the circular RNA construct is measured using RNA scope *in situ* hybridization to quantify the total number of RNA transcripts as well as the identity and spatial localization of RNA transcripts within plant structures (ACD Bio CAT NO: 323120). Additionally, luciferase expression is quantified using the NanoLuc^{®} kit (Promega) using a Spectramax^{®} i3x Multi-Mode Plate Reader (Molecular Devices).

Additionally, 10⁵ plant protoplasts containing circular RNAs are homogenized and collected in Lysis Buffer (Promega, A8261). Protein lysates are collected and antibodies against GFP or luciferase are used to demonstrate protein expression relative to plant protoplasts that do not contain circular RNAs. Proteins are separated on a SDS-PAGE gel (BioRad). A commercially available standard (BioRad) is used as a size marker. After samples are electrotransferred to a polyvinylidene fluoride (PVDF) membrane (BioRad, 1704156), transfers are performed using a Trans-Blot^{®} Turbo^{™} Transfer System (BioRad, 1704150) according to the manufacturer's protocol and visualized on a chemiluminescent kit (Rockland, KCA001). It is expected that strong luciferase expression in the linear RNA negative control will be absent due to degradation of the linear transcript by endogenous exonucleases *in vitro,* compared with strong expression of luciferase produced from circular RNAs.

### Example 23. PSTVd trafficking loop, replication loop, and Spinach RNA aptamer

This example describes the generation of a circular fusion RNA capable of replicating and trafficking through a host plant. In this example, the PSTVd left terminal domain comprising loops 1-6 (SEQ ID: 41), fused to Spinach RNA aptamer (SEQ ID: 26), the PSTVD right terminal region containing the trafficking loop 27 (SEQ ID: 18), and the PSTVd left terminal domain containing the RNA pol II and TFIIIA-7ZF domains required for replication (SEQ ID NO: 42) (Jiang et al., Journal of Virology, 92(20): e1004-18, 2018) are fused to the Spinach RNA aptamer (SEQ ID NO: 26) and PSTVd trafficking loop 27 (SEQ ID NO: 42) respectively.

### Design

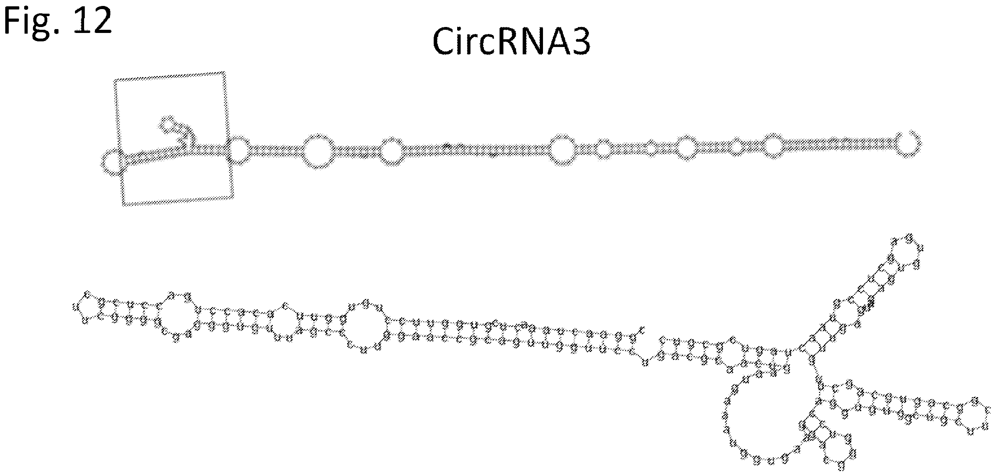

A circular fusion RNA 3 (CircRNA3; Fig. 12; SEQ ID NO: 43) construct is generated containing the following elements:
- PSTVd viral trafficking motif sequence (SEQ ID NO:42 )
- Spinach RNA aptamer (SEQ ID NO: 26)
- PSTVd replication motif sequence (SEQ ID NO: 41)

### Characterization

RNAs are treated with DNase to remove the DNA template. Linear RNAs are then column purified using the Zymo RNA Clean & Concentrator-5 kit (Zymo Research: R1014). To confirm the purity and quality of transcribed RNAs, an aliquot of RNA is heated to 80°C for 10 minutes and run on a 6% denaturing PAGE gel as described in Example 16.

### Delivery and Bioassay

To test whether the viral motif embodied in the circular RNA is capable of replicating and trafficking throughout the tomato plant, the synthesized, circularized fusion RNA is rubbed onto a leaf at the base of an *Arabidopsis thaliana* plant (as described in Example 10). Leaves and stems that are distal to the site of inoculation are analyzed for Spinach aptamer transcripts by qRT-PCR. Linear constructs are expected to have lower or absent fluorescence when compared with circular RNAs. Linear CircRNA3 containing the intact Spinach aptamer is expected to have lower fluorescence than a circularized CircRNA 3. Additionally, efficiently circularized and trafficked and replicated RNA constructs can be visualized by green fluorescence in distal leaf and stem structures when incubated with the 10 µM DFHBI-1T fluorogen (Tocris, 5610) for 30 minutes to one hour. Detection of Spinach aptamer transcripts, as well as the emission of green fluorescence at 472nm, indicate both efficient circularization and trafficking of the circular fusion RNA.

### Example 24. An RNA vector containing two RNA motifs from two different viroid sources to deliver an aptamer to a specific cell type

This example describes the use of a first RNA motif to replicate and amplify the vector, and a second RNA targeting motif to traffic and deliver an RNA molecule that includes an aptamer to a specific plant cell type.

In this example, the RNA vector includes the following:
- PSTVd Left Terminal domain (TL; SEQ ID NO: 33)
- Terminal left region (TLR) (1 to 72 nts) of tomato planta macho viroid (TPMVd) isolate Mex8 (SEQ ID NO: 45) (GenBank Acc. No. GQ131573.1)
- Effector: Spinach RNA aptamer (SEQ ID NO: 26)

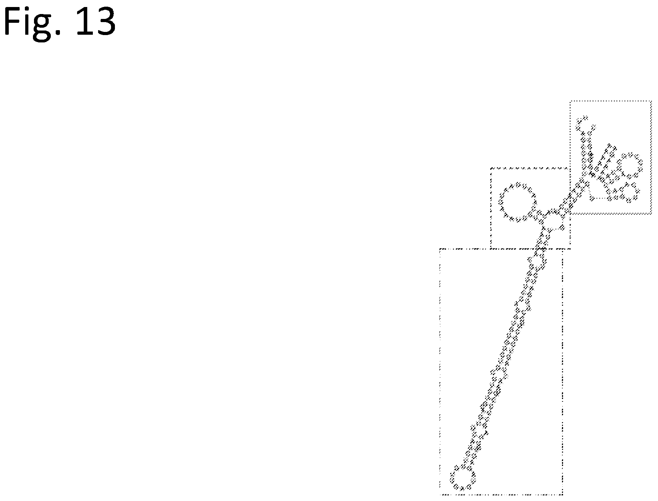

The resulting RNA vector, PSTVd/TL-Spinach-TPMVd/TLR, (see Fig. 13; SEQ ID NO: 46) is mechanically inoculated on tomato leaves by direct rubbing. Fluorescence and RNA presence are quantified in distal tissues by imaging and qRT-PCR.

### Design

The TL is selected according to the region identified by Wang et al., Plant Cell, 28: 1094-1107, 2016. This RNA motif enables replication of the vector in host cells. The TLR (1 to 72 nts) of TPMVd isolate Mex8 (SEQ ID NO: 45) (GenBank Acc. No. GQ131573.1) is designed according to the region identified by Yanagisawa et al., Virology, 526: 22-31, 2019. This second RNA motif enables trafficking of the RNA vector to pollen. The RNA vector is synthesized using in vitro transcription and splint ligation, as described in Example 2.

### Characterization

The synthesized RNA vector and linear RNA controls are quantified using an Agilent 2100 Bioanalyzer system and analyzed using RNase H and RNase R assays, as described in Examples 6 and 7.

### Delivery and in planta bioassay

The RNA vector is mechanically inoculated to Micro-Tom tomato by leaf rubbing, as described in Example 10. Quantitative RT-PCR is employed on inoculated leaf and pollen grain samples from different time points (12 hours, 1 day, 2 days, 4 days, 7 days, and 2 weeks following inoculation) to measure transcript levels of the RNA vector. Spinach RNA aptamer fluorescence is detected using the protocol described in Example 3 using leaf and pollen tissues.

### Example 25. In planta circularization of viral trafficking motifs conjugated to a fluorescent aptamer through transfection of a linear fusion RNA

This example demonstrates the *in planta* introduction of a linear fusion RNA and detection of subsequent endogenous circularization by detection of a fluorescent aptamer. The circular fusion RNAs contain a PSTVd loop 27 viral motif sequence (5'-UUUUCA-3') (SEQ ID NO: 18) previously described to be essential for viral trafficking within the host plant, synthesized as described in Example 16 (Wu et al., PloS Pathogens, 15(10): e1008147, 2019). This trafficking motif is synthesized in tandem with an intact Broccoli RNA aptamer as described in Example 16 (SEQ ID NO: 19).

### Design

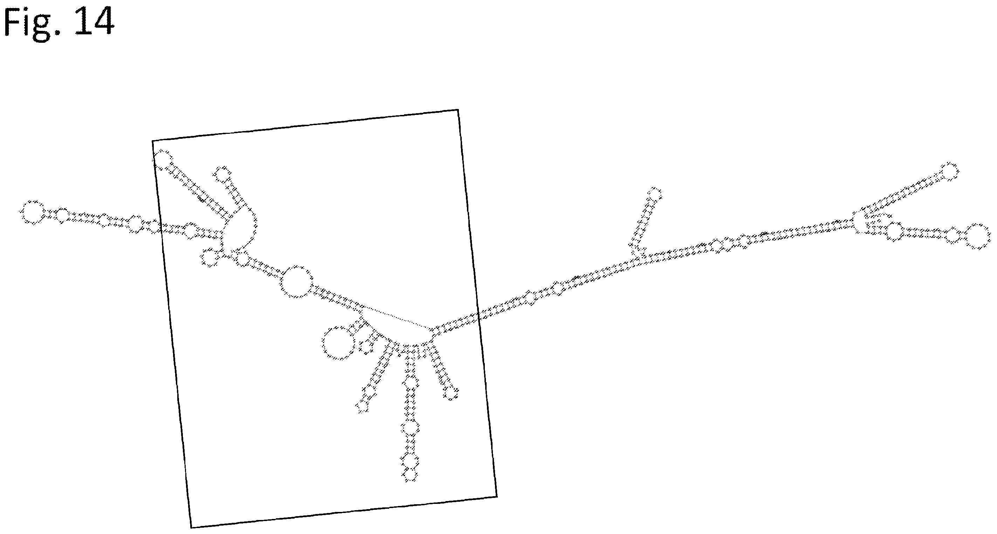

A circular fusion RNA 4 (CircRNA4; Fig. 14; SEQ ID NO: 47) construct is generated containing the following elements:
- PSTVd viral trafficking motif sequence (SEQ ID NO: 18)
- Intact Broccoli RNA aptamer sequence (SEQ ID NO: 19)

### Characterization

RNAs are treated with DNase to remove the DNA template. Linear RNAs are then column purified using the Zymo RNA Clean & Concentrator-5 kit (Zymo Research: R1014). To confirm the purity and quality of transcribed RNAs, an aliquot of RNA is heated to 80°C for 10 minutes and run on a 6% denaturing PAGE gel as described in Example 16.

### Delivery and Bioassay

To test whether a linearized circular fusion RNA is capable of endogenous circularization after inoculation and trafficking throughout the tomato or *Arabidopsis thaliana* plant, linearized CircRNA1 (as previously described in Example 16) is rubbed onto a leaf at the base of the tomato or *Arabidopsis thaliana* plant. Proximal and distal leaves are then removed and incubated with 10µM DFHBI-1T fluorogen (Tocris, 5610) for 30 minutes to one hour. Successful circularization and trafficking of the linear fusion RNA throughout the plant is evidenced by circular RNA transcripts in more distal plant structures. qRT-PCR is used to quantify RNA transcripts of the fusion RNAs. Linear constructs are expected to have lower or absent fluorescence when compared with circular RNAs. Linear CircRNA4 containing the intact Broccoli aptamer is expected to have lower fluorescence than a circularized circRNA4. Additionally, efficiently circularized and trafficked RNA constructs can be visualized by green fluorescence in distal leaves by the emission of green fluorescence at 472nm after incubation with the DFHBI-1T fluorogen. Fluorescence indicates both efficient circularization and trafficking of the circular fusion RNA.

### Example 26: PSTVd R+dsRNA to target RPL7 in CBP

This example describes the utilization of the replication motif from a viroid to replicate an RNA molecule that includes an effector in a plant cell and then deliver to insects by ingestion. The effector targets insect endogenous gene and cause mortality or stunting in larvae of *Leptinotarsa* or other species In this example, the RNA vector includes the following:
- replication domain of PSTVd (potato spindle tuber viroid), consisting of PSTVd TL-CCR (SEQ ID NO: 12): the left-terminal and central conserved region of PSTVd
- effector: hpRNA-RPL7, hairpin RNA (SEQ ID NO: 898) targeting an endogenous gene, Ribosomal Protein L7, from *Leptinotarsa decemlineata* (Colorado potato beetle, CPB)

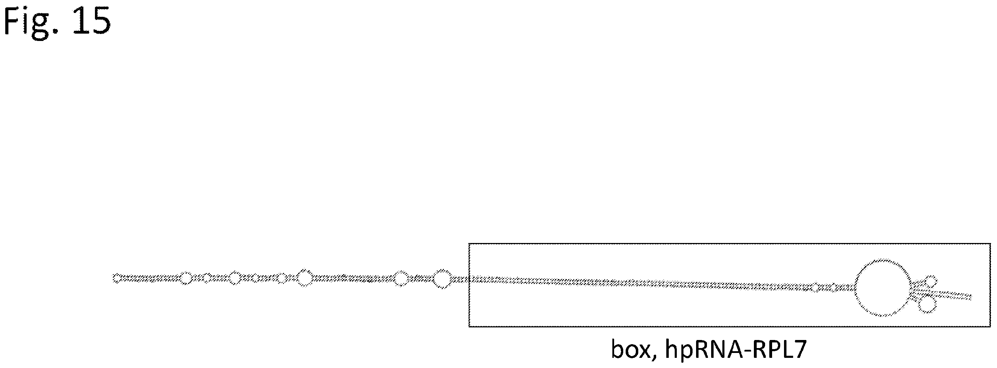

The resulting RNA vector (SEQ ID NO: 899), R-hpRNA-RPL7 (Fig. 15) targeting CPB RPL7 gene (NCBI Gene ID: 111514553) is tested using leaf disc assay to test mortality or stunting of CPB larvae.

### Design

The hpRNA targeting RPL7 (SEQ ID NO: 898) is derived from a hairpin dsRNA encoded by the DNA construct from SEQ ID NO:1105 of US9777288B2 with 90nt targeting RPL7 gene flanking the loop region (149nt). The hpRNA is fused to PSTVd TL-CCR. The RNA vector (SEQ ID NO: 899) is synthesized with in vitro transcription as described in Example 2. Two forms (linear and circular) of the RNA vector are synthesized and tested with leaf disc assay.

### Characterization

The synthesized linear and circular fusion RNAs are quantified using an Agilent 2100 Bioanalyzer system and analyzed using RNase H and RNase R assays, as described in Examples 6 and 7.

### Delivery and bioassay

The RNA vector is added in a 0.1% Silwet^{®} L77 solution in nuclease free water and then applied to potato leaves. The control leaves are treated with the formulation 0.1% Silwet^{®} L77 solution. Treated leaves are collected at different time points, including 12 hours, 24 hours, 2 days, 4 days and 7 days. The replication of the RNA vector is measured by qPCR from leaf samples collected at different time points. Treated leaves are also cut into leaf discs and placed individually into wells of 128-well plate containing 0.5ml/well of a solidified 2% agar. A single CPB neonate is placed in each well and incubated overnight to allow it to consume the leaf disc. The next day, CPB larvae are transferred to a feeding arena made from a covered, aerated 453.6 grams (16-ounce) translucent plastic container lined at its base with filter paper and containing potato foliage with stems inserted in a water-filled tube for freshness. The insects are incubated in the feeding arena in an environmental chamber (27 degrees Celsius; 60% relative humidity; 16 hours light/8 hours dark) with potato foliage replenished as needed. Larval viability is monitored daily and recorded as alive or dead for 16 days.

### Example 27: A nuclear transporter for delivering an RNA cargo or effector to the nucleus of a cell

This example describes a method of delivering an RNA cargo that is carried by a synthetic viroid to a cell organelle. More specifically, this example illustrates the use of a synthetic or recombinant "nuclear transporter" polynucleotide to transport a heterologous effector or RNA cargo to the nucleus in a plant cell. In embodiments, such a synthetic nuclear transporter comprises (i) a single-stranded RNA (ssRNA) viroid sequence and (ii) a heterologous RNA sequence comprising or encoding an effector, wherein the ssRNA viroid sequence does not include a chloroplast localization sequence. In embodiments, the ssRNA viroid sequence is, or is derived from, the sequence of a viroid that replicates in a plant cell nucleus, such as a pospivirus, e.g., any of the pospiviroids identified by name and sequence identifier in Table 1 above.

In one experiment, the sequence of a longer-than-unit PSTVd (SEQ ID NO: 922) was employed as the scaffold for designing a synthetic nuclear transporter including a heterologous effector or RNA cargo to be transported into the nucleus of a plant cell. In related embodiments, other pospiviroid sequences are used as the scaffold for a synthetic nuclear transporter. *In vitro* synthesized nuclear transporters (SEQ ID NOs: 923 and 924) based on the longer-than-unit PSTVd scaffold modified to include a 21-nucleotide *Solanum lycopersicum* phytoene desaturase sequence (SEQ ID NO:9) as a heterologous effector or RNA cargo was produced using methods previously described (Examples 1 - 4 and 8) and Cy3-labeled using HyperScribe T7 High Yield Cy3 RNA Labeling Kit (APExBIO) following the manufacturer's protocol. *Nicotiana tabacum* BY2 protoplasts were isolated and transfected as described in Example 9. Ten µg of a Cy3-labeled synthetic nuclear transporter carrying the PDS sequence as an RNA cargo were transfected into 200 µL of 1x10⁶ /mL BY2 protoplasts. After transfection, protoplasts were kept in the dark and incubated at room temperature for five hours. The protoplasts were then stained for 30 minuntes with Hoechst 33342 dye (ThermoFisher, final concentration of 20 µg/mL), followed by imaging with an Olympus IX83 fluorescence microscope. Hoechst 33342 was visualized with a DAPI filter for nuclear localization. Cy3 was visualized with an RFP filter to identify areas in the cell to which the synthetic nuclear transporter carrying the cargo RNA had localized. The Cy3 signals were co-localized with the Hoechst 33342 signals, indicating that the synthetic nuclear transporter and its RNA cargo had localized to nucleus. These results demonstrate that a synthetic nuclear transporter based on a viroid scaffold sequence and including a heterologous effector or RNA cargo can transport the heterologous effector or RNA cargo to the nucleus of a plant cell.

In other embodiments, synthetic nuclear transporters, such as synthetic viroids based on PSTVd similar to the one described above, are used to transport at least one heterologous effector or RNA cargo to a predetermined subcellular location or organelle (in this case, the nucleus) in a plant cell. Such synthetic nuclear transporters are useful for delivering diverse heterologous effectors or RNA cargoes, such as, but not limited to, one or more small RNAs (e.g., siRNAs, trans-acting siRNAs, miRNAs, crRNAs, guide RNAs, or precursors of any of these), tRNAs or tRNA-like motifs, RNA aptamers, or combinations of any of these or other RNA cargoes to the nucleus of a plant; see also, e.g., Examples 14, 18, and 26, which further illustrate incorporation of a heterologous RNA sequence into a viroid-derived scaffold. In embodiments, at least one synthetic nuclear effector that includes one or more heterologous effectors or RNA cargoes is co-delivered with a polypeptide, e.g., a nuclease or a ligase. In an embodiment, a synthetic nuclear transporter carrying a heterologous effector or RNA cargo that includes two guide RNAs in tandem is co-delivered with a Cas nuclease to a plant cell. In another embodiment, a synthetic nuclear transporter carrying a heterologous effector or RNA cargo including an siRNA or siRNA precursor (e.g., a hairpin) or an miRNA or miRNA precursor (e.g., an engineered miRNA precursor) is delivered to a plant, e.g., topically applied to the surface of a plant or injected into a plant's vascular system for systemic delivery or delivery to other parts of the plant; specific embodiments include those where the siRNA or siRNA precursor or an miRNA or miRNA precursor targets a gene of a plant pest or pathogen. In an example, a synthetic nuclear transporter based on a longer-than-unit PSTVd scaffold (SEQ ID NO: 922) is designed to include as the heterologous effector or RNA cargo at least one hairpin RNA (SEQ ID NO: 898) targeting the endogenous gene, Ribosomal Protein L7, from *Leptinotarsa decemlineata* (Colorado potato beetle, CPB); in other embodiments, other siRNA or siRNA precursors or miRNA or miRNA precursors that target an essential gene of a plant pest or pathogen are similarly used.

In embodiments, the synthetic nuclear transporter comprises (i) a single-stranded RNA (ssRNA) viroid sequence and (ii) a heterologous RNA sequence comprising or encoding an effector, wherein the ssRNA viroid sequence does not include a chloroplast localization sequence. In embodiments, the ssRNA viroid is a pospiviroid, e.g., any of the pospiviroids identified by name and sequence identifier in Table 1 above. In embodiments, the ssRNA viroid has a sequence having at least 80% sequence, at least 85%, at least 90%, at least 95%, at least 98%, at least 99%, or 100% sequnce identity to a sequence selected from the group consisting of SEQ ID NOs:51-54, SEQ ID NOs:65-66, SEQ ID NO:68, SEQ ID NO:75, SEQ ID NOs:77-79, SEQ ID NOs:84-96, SEQ ID NOs:98-107, SEQ ID NOs:123-124, SEQ ID NOs:126-132, SEQ ID NO:134, SEQ ID NOs:136-143, SEQ ID NOs:145-150, SEQ ID NOs:153-154, SEQ ID NO:159, SEQ ID NO:166, SEQ ID NO:168, SEQ ID NO:196, SEQ ID NO:242, SEQ ID NO:268, SEQ ID NO:274, SEQ ID NO:276, SEQ ID NO:289, SEQ ID NO:451, SEQ ID NOs:458-459, and SEQ ID NO:467. In embodiments, the ssRNA viroid has a sequence having at least 80% sequence, at least 85%, at least 90%, at least 95%, at least 98%, at least 99%, or 100% sequnce identity to SEQ ID NO:51.

### Appendix 1. Plant viroid NCBI references

MK303580.1, MK303579.1, MH025994.1, MF770204.1, MF770203.1, MF770202.1, MF770201.1, MF770200.1, MF770199.1, MF770197.1, MF882925.1, MF882924.1, KY522741.1, KY522740.1, KY522739.1, KY522738.1, KY522737.1, KY522736.1, KY522735.1, KY522734.1, KY522733.1, KY522732.1, KY522731.1, KY522730.1, KY522729.1, KY522728.1, MH476293.1, MH476292.1, MH476291.1, MH771135.1, LC433633.1, LC433632.1, LC433631.1, LC433630.1, LC433629.1, LC433628.1, LC430901.1, LQ907271.1, MH758760.1, MF770198.1, MF770196.1, LC388862.1, LC388861.1, LC388860.1, LC388859.1, LC388858.1, LC388857.1, LC388856.1, LC388855.1, LC388854.1, LC388853.1, LC388852.1, NC_027432.1, NC_011590.1, NC_003637.1, NC_003613.1, NC_003538.1, NC_002030.1, NC_002015.1, NC_000885.1, NC_001464.1, NC_001558.1, NC_001553.1, MG450358.1, MG450357.1, MG132059.1, MG132058.1, MG132057.1, MG132056.1, MG132055.1, S79831.1, KM214229.1, KM214228.1, KM214227.1, KM214226.1, KM214225.1, KM214224.1, KM214223.1, KM214222.1, KM214221.1, KM214220.1, KM214219.1, KM214218.1, KM214217.1, KM214216.1, KM214215.1, KM214214.1, KM214213.1, KM214212.1, KM214211.1, KM214210.1, KM214209.1, KM214208.1, KM214207.1, KC143305.1, KC143304.1, KC143303.1, KC143302.1, KC143301.1, KC143300.1, KC143299.1, KC143298.1, KC143297.1, KC143296.1, KC143295.1, KC143294.1, KC143293.1, KC143292.1, KC143291.1, KC143290.1, KC143289.1, JF742641.1, JF742640.1, JF742639.1, JF742638.1, JF742637.1, JF742636.1, JF742635.1, JF742634.1, JF742633.1, JF742632.1, DQ923061.1, DQ923060.1, DQ923059.1, DQ923058.1, DQ076250.1, DQ061193.1, DQ061192.1, DQ022677.1, KY936885.1, KY936884.1, KY936883.1, KY936882.1, KY936881.1, KY936880.1, KY936879.1, KY936878.1, KY936877.1, KY936876.1, KY936875.1, KY936874.1, KY936873.1, MG470649.1, KY110722.1, KY110721.1, MF803029.1, KY646193.1, KY328288.1, KY328287.1, KY810784.1, KY810771.1, MF140254.1, MF140253.1, MF140252.1, MF140251.1, MF140250.1, MF140249.1, MF140248.1, MF140247.1, MF140246.1, MF359723.1, MF359722.1, MF359721.1, MF359720.1, MF359719.1, MF359718.1, MF359717.1, MF359716.1, MF359715.1, MF359714.1, MF359713.1, MF359712.1, MF359711.1, MF359710.1, MF359709.1, MF359708.1, MF359707.1, MF359706.1, MF359705.1, MF359704.1, MF359703.1, MF359702.1, MF359701.1, MF359700.1, MF359699.1, MF359698.1, MF359697.1, MF359696.1, MF359695.1, MF359694.1, MF359693.1, MF359692.1, KY654688.1, KY235369.1, KY473932.1, KY473931.1, KX579067.1, KX819237.1, KX819236.1, KX819235.1, KX819234.1, KX819233.1, KX819232.1, KX819231.1, KX159284.1, KX159283.1, KX159282.1, KX159281.1, KX156933.1, KX156932.1, KX156931.1, KX156930.1, LC192498.1, LC192497.1, LC192496.1, LC192495.1, LC192494.1, LC192493.1, LC192492.1, LC192491.1, LC192490.1, LC192489.1, LC192488.1, LC192487.1, LC192486.1, LC192485.1, LC192484.1, LC192483.1, LC192482.1, LC192481.1, LC192480.1, LC192479.1, LC192478.1, LC192477.1, LC192476.1, LC192475.1, LC192474.1, LC192473.1, LC192472.1, LC192471.1, LC192470.1, LC192469.1, LC192468.1, LC192467.1, LC192466.1, LC192465.1, LC192464.1, LC192463.1, LC192462.1, LC192461.1, LC192460.1, LC192459.1, LC192458.1, LC192457.1, LC192456.1, LC192455.1, KX096576.1, KX096575.1, KX096574.1, KX096573.1, KX096572.1, KX096571.1, KX096570.1, KX096569.1, KX096568.1, KX096567.1, KX096566.1, KX096565.1, KX096564.1, KX096563.1, KX096562.1, KX096561.1, KX096560.1, KX096559.1, KX096558.1, KX096557.1, KX096556.1, KX096555.1, KX096554.1, KX096553.1, KX096552.1, KX096551.1, KX096550.1, KX096549.1, KX096548.1, KX096547.1, KX096546.1, KX096545.1, KX096544.1, KX096543.1, KX096542.1, KX096541.1, KX096540.1, KX096539.1, KX096538.1, KX096537.1, KX096536.1, KX096535.1, KX096534.1, KX096533.1, KX096532.1, KX096531.1, KX096530.1, KX096529.1, KX096528.1, KX096527.1, KX096526.1, KX096525.1, KX096524.1, KX096523.1, KX096522.1, KX096521.1, KX096520.1, KX096519.1, KX096518.1, KX096517.1, KX096516.1, KX096515.1, KX096514.1, KX096513.1, KX096512.1, KX096511.1, KX096510.1, KX096509.1, KX096508.1, KX096507.1, KX096506.1, KX096505.1, KX096504.1, KX096503.1, KX096502.1, KX096501.1, KX096500.1, KX096499.1, KX096498.1, KX096497.1, KX096496.1, KX096495.1, KX096494.1, KX096493.1, KX096492.1, KX096491.1, KX096490.1, KX096489.1, KX096488.1, KX096487.1, KX096486.1, KX096485.1, KX096484.1, KX096483.1, KX096482.1, KX096481.1, KX096480.1, KX096479.1, KX096478.1, KX096477.1, KX096476.1, KX096475.1, KX096474.1, KX096473.1, KX096472.1, KX096471.1, KX096470.1, KX096469.1, KX096468.1, KX096467.1, KX096466.1, KX096465.1, KX096464.1, KX096463.1, KX096462.1, KX096461.1, KX096460.1, KX096459.1, KX096458.1, KX096457.1, KX096456.1, KX096455.1, KX096454.1, KX096453.1, KX096452.1, KX096451.1, KX096450.1, KX096449.1, KX096448.1, KX096447.1, KX096446.1, KX096445.1, KX096444.1, KX096443.1, KX096442.1, KX096441.1, KX096440.1, KX096439.1, KX096438.1, KX096437.1, KX096436.1, KX096435.1, KX096434.1, KX096433.1, KX096432.1, KX096431.1, KX096430.1, KX096429.1, KX096428.1, KX096427.1, KX096426.1, KX096425.1, KX096424.1, KX096423.1, KX096422.1, KX096421.1, KX096420.1, KX096419.1, KX096418.1, KX096417.1, KX096416.1, KX096415.1, KX096414.1, KX096413.1, KX096412.1, KX096411.1, KX096410.1, KX096409.1, KX096408.1, KX096407.1, KX096406.1, KX096405.1, KX096404.1, KX096403.1, KX096402.1, KX096401.1, KX096400.1, KX096399.1, KX096398.1, KX096397.1, KX096396.1, KX096395.1, KX096394.1, KX096393.1, KX096392.1, KX096391.1, KX096390.1, KX096389.1, KX096388.1, KX096387.1, KX096386.1, KX096385.1, KX096384.1, KX096383.1, KX096382.1, KX096381.1, KX096380.1, KX096379.1, KX096378.1, KX096377.1, KX096376.1, KX096375.1, KX096374.1, KX096373.1, KX096372.1, KX096371.1, KX096370.1, KX096369.1, KX096368.1, KX096367.1, KX096366.1, KX096365.1, KX096364.1, KX096363.1, KX096362.1, KX096361.1, KX096360.1, KX096359.1, KX096358.1, KX096357.1, KX096356.1, KX096355.1, KX096354.1, KX096353.1, KX096352.1, KX096351.1, KX096350.1, KX096349.1, KX096348.1, KX096347.1, KX370618.1, KM204319.1, KM204318.1, KM204317.1, KX618912.1, KX084712.1, KX084711.1, KX084710.1, KX084709.1, KX084708.1, KU714937.1, KU714936.1, KU714935.1, KU714934.1, KP454054.1, KP454053.1, KP454052.1, KP454051.1, KP454050.1, KP454049.1, KP454048.1, KP454047.1, KP454046.1, KP454045.1, KP454044.1, KP454043.1, KP454042.1, KP454041.1, KP454040.1, KP454039.1, KP454038.1, KP454037.1, KJ857498.1, KJ857497.1, KJ857496.1, JX259396.1, JX259395.1, JX259394.1, JX259393.1, JX259392.1, KT923159.1, LC093172.1, LC093171.1, LC093170.1, LC093169.1, KU323793.1, KU323792.1, KU323791.1, KU323790.1, KT987925.1, KU535585.1, KU535584.1, KU535583.1, KU535582.1, KU535581.1, KU535580.1, KU535579.1, KP863712.1, KP863711.1, KP863710.1, KP759284.1, KP759283.1, KT725630.1, LC123589.1, HG739074.1, LC062712.1, LC062713.1, KR020041.1, KR020040.1, KR020039.1, KR020038.1, KR020037.1, KR611376.1, KR611375.1, KR611374.1, KR611373.1, KR611372.1, KR611371.1, KR611370.1, KR611369.1, KR611368.1, KR611367.1, KR611366.1, KR611365.1, KR611364.1, KR611363.1, KR611362.1, KR611361.1, KR611360.1, KR611359.1, KR611358.1, KR611357.1, KR611356.1, KR611355.1, KR611354.1, KR611353.1, KR611352.1, KR611351.1, KR611350.1, KR611349.1, KR611348.1, KR611347.1, KR611346.1, KR611345.1, KR611344.1, KR611343.1, KR611342.1, KR611341.1, KR611340.1, KR611339.1, KR611338.1, KR611337.1, KR611336.1, KR611335.1, KR611334.1, LC090814.1, LC090813.1, AB279771.1, AB279770.1, AB279769.1, AB279768.1, LC088323.1, LC088322.1, KT005827.1, KT005826.1, KT005825.1, KT005824.1, KT005823.1, KT005822.1, KT005821.1, KT005820.1, KT005819.1, KT005818.1, KT005817.1, KT005816.1, KT005815.1, KT005814.1, KT005813.1, KT005812.1, KT005811.1, KT005810.1, KT005809.1, KT005808.1, KT005807.1, KT005806.1, KT005805.1, KT005804.1, KT005803.1, KR677387.1, HF954920.1, HF954919.1, HF954918.1, HF954917.1, HF954916.1, KF683201.1, KF683199.1, KF683198.1, KF484878.1, KF265340.1, KP262537.1, KP262536.1, KP262535.1, KP262534.1, KJ956803.1, KJ956802.1, KJ956801.1, KJ956800.1, KJ956799.1, KJ956798.1, KJ810544.1, KJ810543.1, KC290928.1, KC290927.1, JX909290.1, LN681196.1, KJ635807.1, KM588065.1, DI377399.1, HG739082.1, HG739081.1, HG739080.1, HG739079.1, HG739078.1, HG739077.1, HG739076.1, HG739075.1, HG739073.1, HG739072.1, HG739071.1, HG739070.1, KJ817885.1, HG916812.1, KF418774.1, KF418773.1, KF418772.1, KF418771.1, KF418770.1, KF418769.1, KF418768.1, KF418767.1, KF437621.1, KJ810547.1, KJ810546.1, KJ810545.1, KJ538556.1, KJ538555.1, KJ538554.1, JQ904597.1, KF683200.1, KF973205.1, HG916813.1, FR851463.1, KF049417.1, KC282384.1, KC282383.1, KC282382.1, KC282381.1, KF419168.1, KC707563.1, EU879926.1, EU879925.1, EU879924.1, EU879923.1, EU879922.1, EU879921.1, EU879920.1, EU879919.1, EU879918.1, EU879917.1, EU879916.1, EU879915.1, EU879914.1, EU879913.1, KF484879.1, KF493732.1, KC762954.1, KC762953.1, KC762952.1, KF021223.1, KC810010.1, KC81 0009.1, KC81 0008.1, KC810007.1, KC810006.1, KC810005.1, AB746838.1, KC707564.1, JQ362419.1, JQ809268.1, JQ809267.1, JQ809266.1, JQ809265.1, JQ809264.1, JQ809263.1, JQ809262.1, JQ809261.1, JQ809260.1, JQ809259.1, JQ809258.1, JQ809257.1, JQ809256.1, JQ809255.1, JQ809254.1, JQ809253.1, JQ809252.1, JX945671.1, KC345440.1, JX280944.1, JX885867.1, JX885866.1, JX885865.1, JX885864.1, JX867556.1, JX237458.1, JX237457.1, JQ975098.1, EU872276.1, HQ284030.1, HQ284029.1, HQ284028.1, HQ284027.1, HQ284026.1, HQ284025.1, HQ284024.1, HQ284023.1, HQ284022.1, HQ284021.1, HQ284020.1, HQ284019.1, HQ284018.1, HQ284017.1, HQ284016.1, HQ284015.1, HQ284014.1, HQ284013.1, HQ284012.1, HQ284011.1, JQ974379.1, JQ974378.1, JQ904911.1, JQ889854.1, JQ889853.1, JQ889852.1, JQ889851.1, JQ889850.1, JQ889849.1, JQ889848.1, JQ889847.1, JQ889846.1, JQ889845.1, JQ889844.1, JQ889843.1, JQ889842.1, JQ889841.1, JQ889840.1, JQ889839.1, JQ806338.1, JQ685742.1, JQ685741.1, JQ685740.1, JQ685739.1, JQ685738.1, JQ685737.1, JQ685736.1, JQ685735.1, JQ685734.1, JQ889690.1, JQ889689.1, JN872140.1, JF927790.1, JF414238.1, AB679214.1, AB679213.1, AB679212.1, AB679211.1, AB679210.1, AB679209.1, AB679208.1, AB679207.1, AB679206.1, AB679205.1, AB679204.1, AB679203.1, AB679202.1, AB679201.1, AB679200.1, AB679199.1, AB679198.1, AB679197.1, AB679196.1, AB679195.1, AB679194.1, AB679193.1, JN872144.1, JN872143.1, JN872142.1, JN872141.1, JQ083647.1, JQ083646.1, JQ083645.1, JN543957.1, JN559764.1, JN559763.1, AB689034.1, AB689033.1, AB689032.1, AB689031.1, HE575349.1, HQ667141.1, HQ667140.1, HQ667139.1, HQ667138.1, GQ229575.1, JA451622.1, JA451621.1, GU295988.1, JF938538.1, AB623143.1, HQ891019.1, HQ891018.1, HQ639701.1, HQ639700.1, HQ639699.1, HQ639698.1, HQ639697.1, FN994891.1, FW588639.1, JF446939.1, JF446938.1, JF446937.1, JF446936.1, JF446935.1, JF446934.1, JF446933.1, JF446932.1, JF446931.1, JF446930.1, JF446929.1, JF446928.1, JF446927.1, JF446926.1, JF446925.1, JF446924.1, JF446923.1, JF446922.1, JF446921.1, JF446920.1, JF446919.1, JF446918.1, JF446917.1, JF446916.1, JF446915.1, JF446914.1, JF446913.1, JF446912.1, JF446911.1, JF446910.1, JF446909.1, JF446908.1, JF446907.1, JF446906.1, JF446905.1, JF446904.1, JF446903.1, JF446902.1, JF446901.1, JF446900.1, JF446899.1, JF446898.1, JF446897.1, JF446896.1, JF446895.1, JF446894.1, JF446893.1, JF446892.1, JF446891.1, JF446890.1, JF446889.1, JF446888.1, JF446887.1, JF446886.1, JF446885.1, HQ731652.1, FN669540.2, FN669539.2, FN669538.2, FN669537.2, FN669536.2, FN669535.2, FN669534.2, FN669533.2, FN669532.2, FN669531.2, FN669530.2, GQ853469.1, GQ853468.1, GQ853467.1, GQ853466.1, GQ853465.1, GQ853464.1, GQ853463.1, GQ853462.1, GQ853461.1, GQ853460.1, GQ853459.1, GQ853458.1, GQ853457.1, GU300810.1, HQ456944.1, FJ409044.1, HQ423166.1, HQ454937.1, HQ454936.1, HQ454935.1, HQ454934.1, HQ454933.1, HQ454932.1, HQ454931.1, HQ454930.1, HQ454929.1, HQ454928.1, HQ454927.1, HQ454926.1, HQ454925.1, HQ454924.1, HQ454923.1, HQ454922.1, HQ454921.1, HQ454920.1, HQ454919.1, HQ454918.1, HQ454917.1, HQ454916.1, HQ454915.1, HQ454914.1, GU825981.1, GU825980.1, FJ751934.1, FJ751933.1, FJ751932.1, FJ751931.1, FJ751930.1, FJ751929.1, FJ751928.1, FJ751927.1, FJ751926.1, FJ751925.1, FJ662762.1, EU382204.1, EU382203.1, EU382202.1, EU273604.1, EU257478.1, EF582393.1, EF582392.1, EF186991.1, EF186990.1, EF186989.1, EF126048.1, EF126047.1, DQ831486.1, DQ831485.1, HQ452417.1, HQ452416.1, HQ452415.1, HQ452414.1, HQ452413.1, HQ452412.1, HQ452411.1, HQ452410.1, HQ452409.1, HQ452408.1, HQ452407.1, HQ452406.1, HQ452405.1, HQ452404.1, HQ452403.1, HQ452402.1, HQ452401.1, HQ452400.1, HQ452399.1, HM753555.1, EF626530.1, EF488068.1, EF488067.1, EF488066.1, EF488065.1, EF488064.1, EF488063.1, EF488062.1, EF488061.1, EF488060.1, EF488059.1, EF488058.1, EF488057.1, EF488056.1, EF488055.1, EF488054.1, EF488053.1, EF488052.1, EF488051.1, EF488050.1, EF488049.1, AY937194.1, AY937193.1, AY937192.1, AY937191.1, AY937190.1, AY937189.1, AY937188.1, AY937187.1, AY937186.1, AY937185.1, AY937184.1, AY937183.1, AY937182.1, AY937181.1, AY937180.1, AY937179.1, HH970096.1, HH970094.1, HH970091.1, HM560977.1, GU481092.1, GU481091.1, GU481090.1, EF494697.1, EF494696.1, EF494695.1, EF494694.1, EF494693.1, EF494692.1, EF494691.1, EF494690.1, EF494689.1, EF494688.1, EF494687.1, EF494686.1, EF494685.1, EF494684.1, EF494683.1, EF494682.1, EF494681.1, EF494680.1, EF494679.1, EF494678.1, EF494677.1, EF051631.1, EF044305.1, EF044304.1, EF044303.1, EF044302.1, AM777161.1, AM774357.1, AM774356.1, EU094208.1, EU094207.1, FJ773255.1, FJ773254.1, FJ773253.1, EU862231.1, EU862230.1, GU911351.1, GU911350.1, EU729744.2, FJ797614.1, GU592444.1, DQ859013.1, FM995507.1, FM995506.1, FJ715463.1, FJ715462.1, HM043820.1, HM043819.1, HM043818.1, HM043817.1, HM043816.1, HM043815.1, HM043814.1, HM043813.1, HM043812.1, HM043811.1, HM043810.1, GQ131573.1, GQ131572.1, FJ822878.1, FJ822877.1, FJ824844.1, M93686.1, AF162131.1, AY372399.2, GQ169709.1, EF459701.1, EF459700.1, EF459699.1, EF459698.1, EF459697.1, FJ394072.1, FJ394071.1, FJ394070.1, AY222078.1, AY222077.1, AY222076.1, AY222075.1, AY222074.1, AY222073.1, AY222072.1, AF459007.1, AF459006.1, AF459005.1, AF459004.1, AF459003.1, AF459002.1, AF459001.1, AF459000.1, AF458999.1, AF458998.1, AF458997.1, AF458996.1, AF458995.1, AF458994.1, AF458993.1, AF458992.1, AF458991.1, AF458990.1, AF458989.1, AF458988.1, AF458987.1, AF458986.1, GQ396665.1, GQ396664.1, FN673554.1, FN673553.1, S67442.1, S67446.1, S67441.1, S67440.1, S67438.1, S67437.1, S52178.1, GQ915310.1, GQ260199.1, GQ260198.1, GQ260197.1, GQ260196.1, FN646407.1, GQ246194.1, GQ246192.1, GQ246191.1, GQ174502.1, GQ174501.1, AY492083.2, AY492084.1, AY492082.1, AY492081.1, AY492080.1, AY492079.1, AY492078.1, AY492077.1, AY492076.1, AY492075.1, U23060.1, U23059.1, U23058.1, M88678.1, EF580923.1, AY532801.1, AY373446.1, AY372400.1, AY372398.1, AY372397.1, AY372396.1, AY372395.1, AY372394.1, AY372393.1, AY372392.1, AY372391.1, AY372390.1, AY367350.1, AY365230.1, EU877746.1, EU877745.1, EU877744.1, EU877743.1, EU877742.1, EU625577.1, FJ904297.1, FJ904296.1, FJ904295.1, FJ904294.1, FJ904293.1, FJ904292.1, FJ872825.1, FJ872824.1, FJ872823.1, EU447280.1, M38345.1, FJ773261.1, FJ773260.1, FJ773259.1, FJ773258.1, FJ773257.1, FJ773256.1, FJ626866.1, FJ626865.1, FJ626864.1, FJ626863.1, FM998552.1, FM998551.1, FM998550.1, FM998549.1, FM998548.1, FM998547.1, FM998546.1, FM998545.1, FM998544.1, FM998543.1, FM998542.1, AB255880.1, AB255879.1, AB329668.1, X15663.1, EU926739.1, FJ031232.1, EU564185.1, EU564184.1, EU564183.1, EU564175.1, EU564174.1, EU564173.1, EU564172.1, EU564171.1, EU564170.1, EU564169.1, EF192396.2, EF192395.1, EF192394.1, EF192393.1, EU517117.1, EU512994.1, AM920649.1, AM774355.1, EU180221.1, DQ471996.1, DQ471995.1, DQ471994.1, DQ444474.1, DQ444473.1, EF551346.1, AM698095.1, AM698094.1, AM698093.1, DQ406591.1, DQ308561.1, DQ308560.1, DQ308559.1, DQ308558.1, DQ308557.1, DQ308556.1, DQ308555.1, DQ318794.1, DQ318793.1, DQ318792.1, DQ318791.1, DQ318790.1, X76845.1, X76846.1, X76844.1, X76848.1, X76847.1, EF015581.1, DQ846886.1, DQ846885.1, DQ846884.1, DQ846883.1, E00278.1, DQ431996.1, DQ431995.1, DQ431994.1, DQ431993.1, DQ431992.1, DQ431991.1, DQ400342.1, DD220190.1, DD220188.1, DD220185.1, DQ144506.1, DQ315388.1, X53716.1, AJ564803.1, AJ564802.1, AJ564801.1, AJ564800.1, AJ564799.1, AJ564798.1, AJ564797.1, AJ564796.1, AJ564795.1, X52040.1, X52039.1, X52038.1, X52037.1, X52036.1, DQ094298.1, DQ094297.1, DQ094296.1, DQ094295.1, DQ094294.1, DQ094293.1, AJ969017.1, AY962324.1, V01465.1, V01107.1, AY513268.1, AY671957.1, AY671956.1, AY671955.1, AY671954.1, AY671953.1, AY671952.1, X58388.1, AY673974.1, AJ634596.1, AY532804.1, AY532803.1, AY532802.1, AY523584.1, AY523583.1, AY523582.1, AY514447.1, AY514446.1, AY514444.1, AY518940.1, AY518939.1, AY517496.1, AY517495.1, AY517494.1, AY493560.1, AY493559.1, AY456136.1, AJ585258.1, AJ583449.1, AF540963.1, AF540962.1, AF540961.1, AF540960.1, AY360446.1, AY229990.1, X16409.1, X16408.1, X16407.1, D88895.1, AB006737.1, AY152841.1, AY152840.1, AJ515261.1, AF536193.1, AJ490825.1, AB054599.1, AB054598.1, AB054597.1, AB054596.1, AB054595.1, AB054594.1, AB054593.1, AB054592.1, Y00328.1, AY062121.1, AF483473.1, AF483472.1, AF483471.1, AF483470.1, M93685.1, E50939.1, AF458776.1, AF458775.1, AF458772.1, AF458771.1, AF454395.1, AF394453.1, AF394452.1, AF434678.1, AF428064.1, AF428063.1, AF428062.1, AF428061.1, AF428060.1, AF428059.1, AF428058.1, AF298178.1, AF298177.1, AF369530.1, AB055974.1, Y09382.1, Y09383.1, Y08852.1, Y09381.1, Y09577.1, Y09576.1, Y09575.1, Y09574.1, Y09889.1, Y09888.1, Y09887.1, Y09886.1, Y09890.1, Y09891.1, AF148717.1, AJ007489.1, L78463.1, L78462.1, L78461.1, L78460.1, L78459.1, L78458.1, L78457.1, L78456.1, L78454.1, U82445.1, AJ001853.1, AJ001852.1, AJ001851.1, AJ001850.1, AJ001849.1, AJ000046.1, X06390.1, X95292.1, X95293.1, X95734.1, X97387.1, Z34272.1, X17268.1, Z68201.1, U51895.1, U21126.1, M30870.1, M30869.1, M30871.1, M30868.1, K00817.1, K00818.1, M88677.1, M88681.1, M14814.1, M36163.1, M25199.1, M16826.1, M19506.1, M34917.1, K00965.1, K00964.1, J02053.1.

## Claims

1. A method of delivering an effector to a plant, comprising providing to the plant a composition comprising a recombinant polynucleotide comprising: (i) a single-stranded RNA (ssRNA) plant viroid sequence of a viroid from the family Avsunviroidae and (ii) a heterologous RNA sequence that is not a plant viroid sequence and that comprises or encodes an effector, wherein (ii) is inserted into (i); and wherein the recombinant polynucleotide replicates in the plant, whereby the effector comprised by or encoded by the heterologous RNA sequence is delivered to the plant.

2. The method of claim 1, wherein the composition is delivered to only part of a plant, to only meristematic tissue of a plant, to rootstock onto which an untreated scion is grafted, or to a scion that is grafted onto untreated rootstock.

3. The method of claim 1 or 2, wherein the composition provides an increase in disease resistance, drought tolerance, heat tolerance, cold tolerance, salt tolerance, metal tolerance, herbicide tolerance, herbicide resistance, chemical tolerance, environmental stress resistance, water use efficiency, nitrogen utilization, resistance or tolerance to nitrogen stress, nitrogen fixation, pest resistance, herbivore resistance, pathogen resistance, disease resistance, fungal disease resistance, virus resistance, nematode resistance, bacterial disease resistance, insect control, yield under water-limited conditions, vigor, photosynthetic capability, nutrition, flavor, starch production, protein content, carbohydrate content, oil content, fatty acid content, lipid content, digestibility, biomass, shoot length, root length, root architecture, seed set, seed weight, seed quality, germination, fruit set, rate of fruit ripening, production of biopolymers, production of fibers, production of biofuels, production of pharmaceutical peptides, production of secretable peptides, enzyme production, improved processing traits, or amount of harvestable produce relative to a reference level.

4. The method of claim 1 or 2, wherein the ssRNA plant viroid sequence is a genome of a plant viroid from the family Avsunviroidae or a derivative thereof.

5. The method of claim 1 or 2, wherein the plant viroid is eggplant latent viroid (ELVd), or
wherein the ssRNA plant viroid sequence has at least 90% sequence identity to a sequence selected from the group consisting of SEQ ID NOs: 50, 55, 56, 57, 58, 59, 60, 61, 62, 63, 64, 67, 69, 70, 71, 72, 73, 156, 157, 158, 160, 161, 162, 163, 164, 165, 167, 171, 172, 173, 174, 176, 177, 178, 179, 180, 181, 182, 183, 184, 185, 186, 187, 188, 189, 190, 191, 192, 193, 194, 195, 197, 198, 199, 200, 201, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 237, 238, 239, 240, 241, 245, 246, 247, 254, 272, 273, 275, 391, 402, 434, and 460.

6. The method of any one of claims 1-5, wherein the effector:
(a) comprises or is encoded by an ssRNA sequence;
(b) is an siRNA, ta-siRNA, miRNA, pre-miRNA, or a guide RNA;
(c) comprises coding RNA, non-coding RNA, or both coding and non-coding RNA;
(d) comprises non-coding RNA comprising at least one regulatory RNA or at least one interfering RNA that regulates a target gene or its transcript in a target cell; or
(e) modifies a trait, phenotype, or genotype in the target cell, optionally wherein the modifying comprises reducing expression of the target gene; increasing expression of the target gene; editing the target gene; and/or regulating the target gene.

7. The method of any one of claims 1-6, wherein the heterologous RNA sequence comprises coding RNA, non-coding RNA, or both coding and non-coding RNA.

8. The method of claim 1 or 2, wherein the ssRNA plant viroid sequence effects one or more results selected from the group consisting of entry into a tissue or cell of the plant; transmission through a tissue or cell or subcellular component of the plant; replication in a tissue or cell of the plant; targeting to a tissue or cell of the plant; and binding to a factor in a tissue or cell of the plant.

9. The method of any one of claims 1-8, wherein the recombinant polynucleotide lacks free ends and/or is circular.

10. The method of any one of claims 1-9, wherein the composition is topically delivered to the plant, and wherein the topical delivery is spraying, leaf rubbing, soaking, coating, injecting, seed coating, or delivery through root uptake, or wherein the composition is delivered by injection into the plant's vascular system.

11. A composition comprising a recombinant polynucleotide comprising: (a) a single-stranded RNA (ssRNA) plant viroid sequence that is a plant viroid genome or a derivative thereof , wherein the plant viroid is from the family Avsunviroidae, and (b) a heterologous RNA sequence that is not a viroid sequence and that comprises or encodes an effector, wherein (b) is inserted into (a), wherein the recombinant polynucleotide is capable of replicating in a plant, and wherein the effector comprises a double-stranded RNA stem comprising 50 - 100 base pairs.

12. The composition of claim 11, wherein the plant viroid genome is a genome of an eggplant latent viroid (ELVd).

13. The composition of claim 11, wherein the ssRNA plant viroid sequence has at least 90% sequence identity to a sequence selected from the group consisting of SEQ ID NOs: 50, 55, 56, 57, 58, 59, 60, 61, 62, 63, 64, 67, 69, 70, 71, 72, 73, 156, 157, 158, 160, 161, 162, 163, 164, 165, 167, 171, 172, 173, 174, 176, 177, 178, 179, 180, 181, 182, 183, 184, 185, 186, 187, 188, 189, 190, 191, 192, 193, 194, 195, 197, 198, 199, 200, 201, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 237, 238, 239, 240, 241, 245, 246, 247, 254, 272, 273, 275, 391, 402, 434, and 460.

14. The composition of any one of claims 11-13, wherein the composition is formulated for delivery to a plant or to the environment in which the plant grows.

15. A plant cell comprising the composition of any one of claims 11-14, optionally wherein the cell is a dicot plant cell.

16. The cell of claim 15, wherein the cell has been transiently transformed with the recombinant polynucleotide, or wherein the cell has been stably transformed with the recombinant polynucleotide.

17. The method of any one of claims 1 - 10, wherein the plant is capable of passing the recombinant polynucleotide on to a progeny plant

## Patentansprüche

1. Verfahren zum Verabreichen eines Effektors an eine Pflanze, umfassend das Bereitstellen einer Zusammensetzung an die Pflanze, wobei die Zusammensetzung ein rekombinantes Polynukleotid umfasst, welches Folgendes umfasst: (i) eine einzelsträngige RNA (ssRNA)-Pflanzenviroidsequenz eines Viroids aus der Familie der Avsunviroidae und (ii) eine heterologe RNA-Sequenz, die keine Pflanzenviroidsequenz ist und die einen Effektor umfasst oder für diesen kodiert,
wobei (ii) in (i) inseriert ist; und
wobei sich das rekombinante Polynukleotid in der Pflanze repliziert, wodurch der von der heterologen RNA-Sequenz umfasste oder kodierte Effektor an die Pflanze verabreicht wird.

2. Verfahren nach Anspruch 1, wobei die Zusammensetzung nur an einen Teil einer Pflanze, nur an meristematisches Gewebe einer Pflanze, an einen Wurzelstock, auf den ein unbehandelter Edelreis gepfropft ist, oder an einen Edelreis, der auf einen unbehandelten Wurzelstock gepfropft ist, verabreicht wird.

3. Verfahren nach Anspruch 1 oder 2, wobei die Zusammensetzung eine Steigerung der Krankheitsresistenz, Trockenheitstoleranz, Hitzetoleranz, Kältetoleranz, Salztoleranz, Metalltoleranz, Herbizidtoleranz, Herbizidresistenz, Chemikalientoleranz, Resistenz gegenüber Umweltstress, Wassernutzungseffizienz, Stickstoffverwertung, Resistenz oder Toleranz gegenüber Stickstoffstress, Stickstofffixierung, Schädlingsresistenz, Resistenz gegen Pflanzenfresser, Resistenz gegen Krankheitserreger, Krankheitsresistenz, Resistenz gegen Pilzkrankheiten, Virusresistenz, Resistenz gegen Nematoden, Resistenz gegen bakterielle Krankheiten, Insektenbekämpfung, des Ertrags unter wasserbegrenzten Bedingungen, der Wuchskraft, Photosynthesefähigkeit, Nährstoffversorgung, des Geschmacks, der Stärkeproduktion, des Proteingehaltes, Kohlenhydratgehalts, Ölgehalts, Fettsäuregehalts, Lipidgehalts, der Verdaulichkeit, Biomasse, Sprosslänge, Wurzellänge, Wurzelarchitektur, des Samenansatzes, Samengewichts, der Samenqualität, Keimfähigkeit, des Fruchtansatzes, der Reifegeschwindigkeit der Früchte, Produktion von Biopolymeren, Produktion von Fasern, Produktion von Biokraftstoffen, Produktion pharmazeutischer Peptide, Produktion von sekretierbarer Peptide, Enzymproduktion, verbesserte Verarbeitungseigenschaften oder der Menge an erntefähigem Ertrag im Verhältnis zu einem Referenzwert bereitstellt.

4. Verfahren nach Anspruch 1 oder 2, wobei die ssRNA-Pflanzenviroidsequenz ein Genom eines Pflanzenviroids aus der Familie der Avsunviroidae oder ein Derivat davon ist.

5. Verfahren nach Anspruch 1 oder 2, wobei das Pflanzenviroid das latente Auberginen-Latent-Viroid (Eggplant latent viroid, ELVd) ist, oder
wobei die ssRNA-Pflanzenviroidsequenz eine Sequenzidentität von mindestens 90 % zu einer Sequenz aufweist, die ausgewählt ist aus der Gruppe bestehend aus SEQ ID NOs: 50, 55, 56, 57, 58, 59, 60, 61, 62, 63, 64, 67, 69, 70, 71, 72, 73, 156, 157, 158, 160, 161, 162, 163, 164, 165, 167, 171, 172, 173, 174, 176, 177, 178, 179, 180, 181, 182, 183, 184, 185, 186, 187, 188, 189, 190, 191, 192, 193, 194, 195, 197, 198, 199, 200, 201, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 237, 238, 239, 240, 241, 245, 246, 247, 254, 272, 273, 275, 391, 402, 434 und 460.

6. Verfahren nach einem der Ansprüche 1 bis 5, wobei der Effektor:
(a) eine ssRNA-Sequenz umfasst oder durch eine solche kodiert wird;
(b) eine siRNA, ta-siRNA, miRNA, pre-miRNA oder eine guide-RNA ist;
(c) kodierende RNA, nicht-kodierende RNA oder sowohl kodierende als auch nicht-kodierende RNA umfasst;
(d) nicht-kodierende RNA umfasst, die mindestens eine regulatorische RNA oder mindestens eine interferierende RNA umfasst, die ein Zielgen oder dessen Transkript in einer Zielzelle reguliert; oder
(e) ein Merkmal, einen Phänotyp oder einen Genotyp in der Zielzelle modifiziert, wobei das Modifizieren optional das Reduzieren der Expression des Zielgens, das Erhöhen der Expression des Zielgens, das Editieren des Zielgens und/oder das Regulieren des Zielgens umfasst.

7. Verfahren nach einem der Ansprüche 1 bis 6, wobei die heterologe RNA-Sequenz kodierende RNA, nicht-kodierende RNA oder sowohl kodierende als auch nicht-kodierende RNA umfasst.

8. Verfahren nach Anspruch 1 oder 2, wobei die ssRNA- Pflanzenviroidsequenz ein oder mehrere Ergebnisse bewirkt, ausgewählt aus der Gruppe bestehend aus dem Eindringen in ein Gewebe oder eine Zelle der Pflanze; der Übertragung durch ein Gewebe, eine Zelle oder eine subzelluläre Komponente der Pflanze; der Replikation in einem Gewebe oder einer Zelle der Pflanze; der Zielorientierung auf ein Gewebe oder eine Zelle der Pflanze; und der Bindung an einen Faktor in einem Gewebe oder einer Zelle der Pflanze.

9. Verfahren nach einem der Ansprüche 1 bis 8, wobei das rekombinante Polynukleotid keine freien Enden aufweist und/oder zirkulär ist.

10. Verfahren nach einem der Ansprüche 1 bis 9, wobei die Zusammensetzung topisch an die Pflanze verabreicht wird und wobei die topische Verabreichung durch Besprühen, Einreiben der Blätter, Einweichen, Beschichten, Injizieren, Beschichten von Saatgut oder Verabreichung über die Wurzelaufnahme erfolgt, oder wobei die Zusammensetzung durch Injektion in das Gefäßsystem der Pflanze verabreicht wird.

11. Eine Zusammensetzung, umfassend ein rekombinantes Polynukleotid, welches Folgendes umfasst: (a) eine einzelsträngige RNA (ssRNA)-Pflanzenviroidsequenz, die ein Pflanzenviroid-Genom oder ein Derivat davon ist, wobei das Pflanzenviroid aus der Familie der Avsunviroidae stammt, und (b) eine heterologe RNA-Sequenz, die keine Viroidsequenz ist und die einen Effektor umfasst oder für diesen kodiert, wobei (b) in (a) inseriert ist, wobei das rekombinante Polynukleotid in der Lage ist, sich in einer Pflanze zu replizieren, und wobei der Effektor einen doppelsträngigen RNA-Stamm umfasst, der 50-100 Basenpaare umfasst.

12. Zusammensetzung nach Anspruch 11, wobei das Pflanzenviroid-Genom ein Genom eines Auberginen-Latent-Viroid (Eggplant latent viroid, ELVd) ist.

13. Zusammensetzung nach Anspruch 11, wobei die ssRNA-Pflanzenviroidsequenz eine Sequenzidentität von mindestens 90 % zu einer Sequenz aufweist, die ausgewählt ist aus der Gruppe bestehend aus SEQ ID NOs: 50, 55, 56, 57, 58, 59, 60, 61, 62, 63, 64, 67, 69, 70, 71, 72, 73, 156, 157, 158, 160, 161, 162, 163, 164, 165, 167, 171, 172, 173, 174, 176, 177, 178, 179, 180, 181, 182, 183, 184, 185, 186, 187, 188, 189, 190, 191, 192, 193, 194, 195, 197, 198, 199, 200, 201, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 237, 238, 239, 240, 241, 245, 246, 247, 254, 272, 273, 275, 391, 402, 434 und 460.

14. Die Zusammensetzung nach einem der Ansprüche 11 bis 13, wobei die Zusammensetzung zur Verabreichung an eine Pflanze oder an die Umgebung, in der die Pflanze wächst, formuliert ist.

15. Eine Pflanzenzelle, umfassend die Zusammensetzung gemäß einem der Ansprüche 11 bis 14, optional wobei die Zelle eine dikotyle Pflanzenzelle ist.

16. Die Zelle nach Anspruch 15, wobei die Zelle transient mit dem rekombinanten Polynukleotid transformiert wurde oder wobei die Zelle stabil mit dem rekombinanten Polynukleotid transformiert wurde.

17. Verfahren nach einem der Ansprüche 1 bis 10, wobei die Pflanze in der Lage ist, das rekombinante Polynukleotid an eine Nachkommenzelle weiterzugeben.

## Revendications

1. Procédé de délivrance d'un effecteur à une plante, comprenant la fourniture à la plante d'une composition comprenant un polynucléotide recombinant comprenant : (i) une séquence de viroïde de plante à ARN simple brin (ssRNA) d'un viroïde appartenant à la famille des Avsunviroidae et (ii) une séquence d'ARN hétérologue qui n'est pas une séquence de viroïde de plante et qui comprend ou code un effecteur, sachant que (ii) est insérée dans (i) ; et sachant que le polynucléotide recombinant se réplique dans la plante, moyennant quoi l'effecteur compris dans ou codé par la séquence d'ARN hétérologue est délivré à la plante.

2. Le procédé de la revendication 1, sachant que la composition est délivrée seulement à une partie d'une plante, seulement un tissu méristématique d'une plante, à un porte-greffe sur lequel un greffon non traité est greffé, ou à un greffon qui est greffé sur un porte-greffe non traité.

3. Le procédé de la revendication 1 ou 2, sachant que la composition fournit une augmentation de la résistance aux maladies, de la tolérance à la sécheresse, de la tolérance à la chaleur, de la tolérance au froid, de la tolérance au sel, de la tolérance aux métaux, de la tolérance aux herbicides, de la résistance aux herbicides, de la tolérance aux produits chimiques, de la résistance au stress environnemental, de l'efficacité d'utilisation de l'eau, de l'utilisation de l'azote, de la résistance ou de la tolérance au stress azoté, de la fixation de l'azote, de la résistance aux ravageurs, de la résistance aux herbivores, de la résistance aux agents pathogènes, de la résistance aux maladies, de la résistance aux maladies fongiques, de la résistance aux virus, de la résistance aux nématodes, de la résistance aux maladies bactériennes, de la lutte contre les insectes, du rendement dans des conditions de disponibilité limitée en eau, de la vigueur, de la capacité photosynthétique, de la nutrition, de la saveur, de la production d'amidon, de la teneur en protéines, de la teneur en glucides, de la teneur en huile, de la teneur en matières grasses, de la teneur en lipides, de la digestibilité, de la biomasse, de la longueur des pousses, de la longueur des racines, de l'architecture racinaire, de la nouaison des graines, du poids des graines, de la qualité des graines, de la germination, de la nouaison des fruits, de la vitesse de maturation des fruits, de la production de biopolymères, de la production de fibres, de la production de biocarburants, de la production de peptides pharmaceutiques, de la production de peptides sécrétables, de la production d'enzymes, de caractéristiques de transformation améliorées, ou de la quantité de produits récoltables par rapport à un niveau de référence.

4. Le procédé de la revendication 1 ou 2, sachant que la séquence de viroïde de plante ssRNA est un génome d'un viroïde de plante appartenant à la famille des Avsunviroidae ou un dérivé de celui-ci.

5. Le procédé de la revendication 1 ou 2, sachant que le viroïde de plante est le viroïde latent de l'aubergine (ELVd), ou
sachant que la séquence de viroïde de plante ssRNA présente au moins 90 % d'identité de séquence avec une séquence sélectionnée dans le groupe constitué de SEQ ID N^{os} 50, 55, 56, 57, 58, 59, 60, 61, 62, 63, 64, 67, 69, 70, 71, 72, 73, 156, 157, 158, 160, 161, 162, 163, 164, 165, 167, 171, 172, 173, 174, 176, 177, 178, 179, 180, 181, 182, 183, 184, 185, 186, 187, 188, 189, 190, 191, 192, 193, 194, 195, 197, 198, 199, 200, 201, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 237, 238, 239, 240, 241, 245, 246, 247, 254, 272, 273, 275, 391, 402, 434, et 460.

6. Le procédé de l'une quelconque des revendications 1 à 5, sachant que l'effecteur :
(a) comprend ou est codé par une séquence ssRNA ;
(b) est un siRNA, ta-siRNA, miRNA, pré-miRNA, ou un ARN guide ;
(c) comprend un ARN codant, un ARN non codant, ou à la fois un ARN codant et non codant ;
(d) comprend un ARN non codant comprenant au moins un ARN régulateur ou au moins un ARN interférent qui régule un gène cible ou son transcrit dans une cellule cible ; ou
(e) modifie une caractéristique, un phénotype, ou un génotype dans la cellule cible, facultativement sachant que la modification comprend la réduction de l'expression du gène cible ; l'augmentation de l'expression du gène cible ; l'édition du gène cible ; et/ou la régulation du gène cible.

7. Le procédé de l'une quelconque des revendications 1 à 6, sachant que la séquence d'ARN hétérologue comprend un ARN codant, un ARN non codant, ou à la fois un ARN codant et non codant.

8. Le procédé de la revendication 1 ou 2, sachant que la séquence de viroïde de plante ssRNA entraîne un ou plusieurs résultats sélectionnés dans le groupe constitué de l'entrée dans un tissu ou une cellule de la plante ; la transmission à travers un tissu ou une cellule ou un composant subcellulaire de la plante ; la réplication dans un tissu ou une cellule de la plante ; le ciblage d'un tissu ou d'une cellule de la plante ; et la liaison à un facteur dans un tissu ou une cellule de la plante.

9. Le procédé de l'une quelconque des revendications 1 à 8, sachant que le polynucléotide recombinant est dépourvu d'extrémités libres et/ou est circulaire.

10. Le procédé de l'une quelconque des revendications 1 à 9, sachant que la composition est délivrée par voie topique à la plante, et sachant que la délivrance topique est une pulvérisation, une friction de feuilles, un trempage, un enrobage, une injection, un enrobage de graines, ou une délivrance par absorption racinaire, ou sachant que la composition est délivrée par injection dans le système vasculaire de la plante.

11. Composition comprenant un polynucléotide recombinant comprenant : (a) une séquence de viroïde de plante à ARN simple brin (ssRNA) qui est un génome de viroïde de plante ou un dérivé de celui-ci, sachant que le viroïde de plante appartient à la famille des Avsunviroidae, et (b) une séquence d'ARN hétérologue qui n'est pas une séquence de viroïde et qui comprend ou code un effecteur, sachant que (b) est insérée dans (a), sachant que le polynucléotide recombinant est capable de se répliquer dans une plante, et sachant que l'effecteur comprend une tige d'ARN double brin comprenant 50 à 100 paires de bases.

12. La composition de la revendication 11, sachant que le génome de viroïde de plante est un génome d'un viroïde latent de l'aubergine (ELVd).

13. La composition de la revendication 11, sachant que la séquence de viroïde de plante ssRNA présente au moins 90 % d'identité de séquence avec une séquence sélectionnée dans le groupe constitué de SEQ ID N^{os} 50, 55, 56, 57, 58, 59, 60, 61, 62, 63, 64, 67, 69, 70, 71, 72, 73, 156, 157, 158, 160, 161, 162, 163, 164, 165, 167, 171, 172, 173, 174, 176, 177, 178, 179, 180, 181, 182, 183, 184, 185, 186, 187, 188, 189, 190, 191, 192, 193, 194, 195, 197, 198, 199, 200, 201, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 237, 238, 239, 240, 241, 245, 246, 247, 254, 272, 273, 275, 391, 402, 434, et 460.

14. La composition de l'une quelconque des revendications 11 à 13, sachant que la composition est formulée pour être délivrée à une plante ou à l'environnement dans lequel pousse la plante.

15. Cellule de plante comprenant la composition de l'une quelconque des revendications 11 à 14, facultativement sachant que la cellule est une cellule de plante dicotylédone.

16. La cellule de la revendication 15, sachant que la cellule a été transformée transitoirement avec le polynucléotide recombinant, ou sachant que la cellule a été transformée de manière stable avec le polynucléotide recombinant.

17. Le procédé de l'une quelconque des revendications 1 à 10, sachant que la plante est capable de transmettre le polynucléotide recombinant à une plante descendante.
